# EUROPEAN PATENT APPLICATION

(11) **EP 4 537 842 A1**
(43) Date of publication of application: **16.04.2025**
(21) Application number: 23819849.3
(22) Date of filing: 06.06.2023
(51) Int. Cl.: A61K 39/395, A61P 21/00, C07K 16/18, C12N 15/13, A61P 25/00, A61P 25/14, A61P 25/16, A61P 25/28

(54) **PREVENTATIVE AGENT OR THERAPEUTIC AGENT FOR AMYOTROPHIC LATERAL SCLEROSIS, PARKINSON'S DISEASE, HUNTINGTON'S DISEASE, SPINOCEREBELLAR ATAXIA, AGING-RELATED DEGENERATIVE OR NEUROLOGICAL DISEASE, BRAIN AGING, OR DISEASES ASSOCIATED WITH BRAIN AGING**

(30) Priority: 07.06.2022 JP 2022092342
(71) Applicant: Institute of Science Tokyo, Tokyo 152-8550 (JP)
(72) Inventor: OKAZAWA, Hitoshi, Tokyo 152-8550 (JP)
(74) Representative: D Young & Co LLP
(86) International application number: PCT/JP2023/021039
(87) International publication number: WO 2023/238869

(57) **Abstract**

The present invention addresses the problem of providing an agent for preventing or treating amyotrophic lateral sclerosis (ALS), Parkinson's disease (PD), Huntington's disease (HD), spinocerebellar ataxia (SCA), aging-related degenerative or neurological disease, brain aging, or diseases associated with brain aging, as well as a more stable antibody that exhibits an effect of preventing or treating these diseases, Alzheimer's disease (AD), or frontotemporal lobar degeneration (FTLD). A human monoclonal antibody that specifically binds to human HMGB1, wherein the human monoclonal antibody (anti-human HMGB1 antibody) comprises a heavy chain CDR1, heavy chain CDR2, and heavy chain CDR3 each consisting of a specific amino acid sequence and a light chain CDR1, light chain CDR2, and light chain CDR3 each consisting of a specific amino acid sequence, is used as an agent for preventing or treating ALS, PD, HD, SCA, aging-related degenerative or neurological disease, brain aging, or diseases associated with brain aging. An antibody in which the light chain complementarity determining region (CDR) 3 of the anti-human HMGB1 antibody has been modified is used.

## Description

### Technical Field

The present invention relates to a drug product for preventing or treating amyotrophic lateral sclerosis (hereinafter may be referred to as "ALS"), Parkinson's disease (hereinafter may be referred to as "PD"), Huntington's disease (hereinafter may be referred to as "HD"), spinocerebellar ataxia (hereinafter may be referred to as "SCA"), degenerative or neurological diseases related to aging (more specifically, cellular senescence), brain aging, or diseases associated with brain aging (hereinafter, these may be collectively referred to as "the diseases of the present invention and the like"); an anti-human HMGB1 antibody; or a drug product for preventing or treating Alzheimer's disease (hereinafter may be referred to as "AD") or frontotemporal lobar degeneration (hereinafter may be referred to as "FTLD"), and the like.

### Background Art

ALS is an adult-onset neurodegenerative disease, and exhibits phylogenetic disorders in upper (motor cerebral cortex) and lower (spinal anterior horn cells and brainstem motor nuclei) motor neurons. Approximately 10% of ALS patients have a familial disease (gene mutation), whereas the remaining 90% have an idiopathic (sporadic) disease. The cause of ALS onset remains unknown, and an ALS therapeutic agent riluzole (Patent Document 1) has effects that merely delay progression of the symptoms for several months, indicating there is no effective method for treating ALS.

On the other hand, FTLD is the second or third most common early-onset neurodegenerative dementia following AD and is considered closely related to motor neuron diseases. FTLD has symptoms of noticeable changes in behavior and personality which are often accompanied by language impairment, and these symptoms gradually progress to cognitive impairment and dementia. As causative genes of familial FTLD, valosin-containing protein (VCP), progranulin (PGRN), charged multivesicular body protein 2B (CHMP2B), and transactive response DNA-binding protein of 43 kD (TDP-43) are known. Studies of FTLD are under way, but the whole picture of the onset mechanism thereof is yet to be clarified as with ALS.

Up to 50% of ALS patients develop frontotemporal dysfunction, whereas 15% of FTLD patients develop motor neuron dysfunction. As such, although ALS and FTLD exhibit clearly different symptoms, they have a significant overlap at the molecular level. Therefore, ALS and FTLD develop due to a combination of various genetic and environmental factors, and more than 20 genes are thought to be involved in these diseases.

Meanwhile, high mobility group box 1 (HMGB1) protein is known as one of non-histone chromatin-associated proteins that are involved in maintenance of the DNA structure and transcriptional regulation. Recently, this HMGB1 is drawing attention because it not only functions in the nucleus but also functions as a so-called damage-associated molecule pattern (DAMP) when it is released out of a cell owing to cellular necrosis or actively secreted out of a cell owing to a vascular inflammatory response to a signal. The present inventors found that, in AD, HMGB1 leaking out of a cell owing to neuronal necrosis induces phosphorylation of serine at position 46 (Ser46) of MARCKS, which is a substrate of a phosphorylating enzyme, thereby inducing degeneration of neurites, prepared a mouse monoclonal antibody against HMGB1, and found that this mouse monoclonal antibody inhibits phosphorylation of Ser46 of MARCKS (Non-Patent Document 1). Further, the present inventors have reported that this mouse monoclonal antibody and a human monoclonal antibody resolve cognitive impairment in Alzheimer's disease model mice (Patent Documents 2 and 3).

### Prior Art Documents

### Patent Documents

Patent Document 1: Japanese Patent No. 2713384
Patent Document 2: International Publication No. WO 2018/030405
Patent Document 3: International Publication No. WO 2020/059847

### Non-Patent Document

Non Patent Document 1: SCIENTIFIC REPORT, Aug 25, 2016; 6:31895

### Summary of the Invention

### Object to be Solved by the Invention

An object of the present invention is to provide an agent for preventing or treating ALS, PD, HD, SCA, aging-related degenerative or neurological disease, brain aging, or diseases associated with brain aging, and an antibody that is more stable and exhibits an effect of preventing or treating AD or FTLD.

### Means to Solve the Object

The present inventor is continuing to study assiduously to solve the above-described object. During this process, a human monoclonal antibody #129 prepared in the examples of International Publication No. WO 2020/059847 (Patent Document 3) was found to exhibit a treatment effect on four types of gene mutations (VCP^{T262A} mutation, PGRN^{R504X} mutation, CHMP2B^{Q165X} mutation, and TDP43^{N267S} mutation) in the FTLD-ALS spectrum (hereinafter may be referred to as "FTLD-ALS"), and suppress transcriptional repression-induced atypical cell death (TRIAD) necrosis in nerve cells (may be referred to "neurons") as well as further having an effect of inhibiting reciprocal amplification of TRIAD necrosis that is based on HMGB1-induced DNA damage. Also, in the pathological conditions of HD, PD, SCA, and ALS, when taking into consideration of the conventional technique in which TRIAD necrosis of nerve cells and the underlying DNA damage occur and the conventional technique in which the above-mentioned four types of gene mutations are the cause of not only FTLD but also ALS, it can be said that the above-described human monoclonal antibody #129 and the HMGB1 antibody having common complementarity determining regions (CDRs) have a treatment effect on general neurodegenerative diseases including ALS, PD, HD, and SCA. Further, the above-described human monoclonal antibody #129 was confirmed to reduce the number of senescent cells in the cerebral cortex of two types of AD model mice (5 × FAD mice and APP-KI mice), and also to suppress the cellular senescence of neurons, astrocytes and microglia. In addition, since TRIAD necrosis was found to be age-related cell death itself, the human monoclonal antibody #129 and the anti-HMGB1 antibody having common CDRs thereto can be said to have an effect of treating aging-related degenerative or neurological diseases, brain aging itself, and diseases associated with brain aging.

Further, as a result of analyzing treatment effects on five types of model mice (HD model mice, PD model mice, ALS model mice, spinocerebellar ataxia [hereinafter may be referred to as "SCA"] model mice, and aging model mice) using, in addition to the human monoclonal antibody #129, a human monoclonal antibody #129-L2, which is a modified antibody of the light chain CDR3 of the human monoclonal antibody #129, the human monoclonal antibody #129 and the human monoclonal antibody #129-L2 were confirmed to exhibit treatment effects on HD, PD, ALS, SCA, and aging-associated cellular changes, cell death, cognitive function (memory), and motor function.

Furthermore, it was confirmed that the human monoclonal antibody #129-L2 has similar binding strength for human disulfide HMGB1 (ds-HMGB1) to the human monoclonal antibody #129 and treatment effects on FTLD-ALS model mice and AD model mice, and that the binding strength for human reduced HMGB1 is as weak as or weaker than that of the human monoclonal antibody #129. Also, it was confirmed that, unlike the human monoclonal antibody #129, the human monoclonal antibody #129-L2 does undergo N-glycan glycosylation, and that the stability of the antibody when stored is rather higher than that of the human monoclonal antibody #129.

The present invention was accomplished based on these findings.

Specifically, the present invention provides the following.
[1] An agent for preventing or treating amyotrophic lateral sclerosis, Parkinson's disease, Huntington's disease, spinocerebellar ataxia, aging-related degenerative or neurological diseases, brain aging, or diseases associated with brain aging, comprising a human monoclonal antibody that specifically binds to human HMGB1 and comprises:
   a heavy chain complementarity determining region (CDR) 1 consisting of the amino acid sequence shown in SEQ ID No: 1 or an amino acid sequence in which one or more amino acids are substituted, deleted, added, and/or inserted in the amino acid sequence shown in SEQ ID No: 1; a heavy chain CDR2 consisting of the amino acid sequence shown in SEQ ID No: 2 or an amino acid sequence in which one or more amino acids are substituted, deleted, added, and/or inserted in the amino acid sequence shown in SEQ ID No: 2; and a heavy chain CDR3 consisting of the amino acid sequence shown in SEQ ID No: 3 or an amino acid sequence in which one or more amino acids are substituted, deleted, added, and/or inserted in the amino acid sequence shown in SEQ ID No: 3; and
   a light chain CDR1 consisting of the amino acid sequence shown in SEQ ID No: 4 or an amino acid sequence in which one or more amino acids are substituted, deleted, added, and/or inserted in the amino acid sequence shown in SEQ ID No: 4; a light chain CDR2 consisting of the amino acid sequence shown in SEQ ID No: 5 or an amino acid sequence in which one or more amino acids are substituted, deleted, added, and/or inserted in the amino acid sequence shown in SEQ ID No: 5;
   and a light chain CDR3 consisting of the amino acid sequence shown in SEQ ID No: 6, 20 or 17, or an amino acid sequence in which one or more amino acids are substituted, deleted, added, and/or inserted in the amino acid sequence shown in SEQ ID No: 6, 20 or 17.
[2] The agent according to the above-described [1], wherein the human monoclonal antibody comprises a heavy chain variable region consisting of an amino acid sequence having at least 80% or more sequence identity to the amino acid sequence shown in SEQ ID No: 7, and a light chain variable region consisting of an amino acid sequence having at least 80% or more sequence identity to the amino acid sequence shown in SEQ ID No: 8, SEQ ID No: 21, or SEQ ID No: 18.
[3] The agent according to the above-described [1] or [2], wherein the human monoclonal antibody comprises a heavy chain consisting of an amino acid sequence having at least 80% or more sequence identity to the amino acid sequence shown in SEQ ID No: 9, and a light chain consisting of an amino acid sequence having at least 80% or more sequence identity to the amino acid sequence shown in SEQ ID No: 10, SEQ ID No: 22, or SEQ ID No: 19.
[4] The agent according to any of the above-described [1] to [3], wherein the agent is intravenously administered.
[5] A human monoclonal antibody that specifically binds to human HMGB1, comprising:
   a heavy chain complementarity determining region (CDR) 1 consisting of the amino acid sequence shown in SEQ ID No: 1; a heavy chain CDR2 consisting of the amino acid sequence shown in SEQ ID No: 2; and a heavy chain CDR3 consisting of the amino acid sequence shown in SEQ ID No: 3; and
   a light chain CDR1 consisting of the amino acid sequence shown in SEQ ID No: 4; a light chain CDR2 consisting of the amino acid sequence shown in SEQ ID No: 5; and a light chain CDR3 consisting of the amino acid sequence shown in SEQ ID No: 20 or 17.
[6] The human monoclonal antibody according to the above-described [5], comprising a heavy chain variable region consisting of an amino acid sequence having at least 80% or more sequence identity to the amino acid sequence shown in SEQ ID No: 7 and a light chain variable region consisting of an amino acid sequence having at least 80% or more sequence identity to the amino acid sequence shown in SEQ ID No: 21 or 18.
[7] The human monoclonal antibody according to the above-described [5] or [6], comprising a heavy chain consisting of an amino acid sequence having at least 80% or more sequence identity to the amino acid sequence shown in SEQ ID No: 9, and a light chain consisting of an amino acid sequence having at least 80% or more sequence identity to the amino acid sequence shown in SEQ ID No: 22 or 19.
[8] An agent for preventing or treating Alzheimer's disease or frontotemporal lobar degeneration, comprising the human monoclonal antibody according to any one of the above-described [5] to [7].
[9] The agent according to the above-described [8], wherein the agent is intravenously administered.
[10] An agent for preventing or treating amyotrophic lateral sclerosis, Parkinson's disease, Huntington's disease, spinocerebellar ataxia, aging-related degenerative or neurological diseases, brain aging, or diseases associated with brain aging, comprising the human monoclonal antibody according to any one of the above-described [5] to [7].
[11] The agent according to the above-described [10], wherein the agent is intravenously administered.

Further, examples of other embodiments of the present invention include the following:
a method for preventing or treating the diseases of the present invention and the like, comprising a step of administering a human monoclonal antibody (hereinafter may be referred to as "the human monoclonal antibody of the present invention") that specifically binds to human HMGB1 and comprises:
a heavy (H) chain complementarity determining region (CDR) 1 consisting of the amino acid sequence shown in SEQ ID No: 1 or an amino acid sequence in which one or more amino acids are substituted, deleted, added, and/or inserted in the amino acid sequence shown in SEQ ID No: 1, an H chain CDR2 consisting of the amino acid sequence shown in SEQ ID No: 2 or an amino acid sequence in which one or more amino acids are substituted, deleted, added, and/or inserted in the amino acid sequence shown in SEQ ID No: 2, and an H chain CDR3 consisting of the amino acid sequence shown in SEQ ID No: 3 or an amino acid sequence in which one or more amino acids are substituted, deleted, added, and/or inserted in the amino acid sequence shown in SEQ ID No: 3; and
a light (L) chain CDR1 consisting of the amino acid sequence shown in SEQ ID No: 4 or an amino acid sequence in which one or more amino acids are substituted, deleted, added, and/or inserted in the amino acid sequence shown in SEQ ID No: 4, an L chain CDR2 consisting of the amino acid sequence shown in SEQ ID No: 5 or an amino acid sequence in which one or more amino acids are substituted, deleted, added, and/or inserted in the amino acid sequence shown in SEQ ID No: 5, and an L chain CDR3 consisting of the amino acid sequence shown in SEQ ID No: 6, 20 or 17, or an amino acid sequence in which one or more amino acids are substituted, deleted, added, and/or inserted in the amino acid sequence shown in SEQ ID No: 6, 20 or 17,
to a subject in need of the prevention or treatment of the diseases of the present invention and the like;
the human monoclonal antibody of the present invention for use as an agent for preventing or treating the diseases of the present invention and the like;
the human monoclonal antibody of the present invention for use in prevention or treatment of the diseases of the present invention and the like; and
use of the human monoclonal antibody of the present invention for manufacturing an agent for preventing or treating the diseases of the present invention and the like.

Further, examples of other embodiments of the present invention include the following:
a method for preventing or treating Alzheimer's disease or frontotemporal lobar degeneration, and/or the diseases of the present invention and the like, comprising a step of administering a human monoclonal antibody (hereinafter may be referred to as "the human monoclonal antibody L1/L2 of the present invention") that specifically binds to human HMGB1 and comprises:
an H chain CDR1 consisting of the amino acid sequence shown in SEQ ID No: 1, an H chain CDR2 consisting of the amino acid sequence shown in SEQ ID No: 2, and an H chain CDR3 consisting of the amino acid sequence shown in SEQ ID No: 3; and
an L chain CDR1 consisting of the amino acid sequence shown in SEQ ID No: 4, an L chain CDR2 consisting of the amino acid sequence shown in SEQ ID No: 5, and an L chain CDR3 consisting of the amino acid sequence shown in SEQ ID No: 20 or 17,
to a subject in need of the prevention or treatment of Alzheimer's disease or frontotemporal lobar degeneration, and/or the diseases of the present invention and the like;
the human monoclonal antibody L1/L2 of the present invention for use as an agent for preventing or treating Alzheimer's disease or frontotemporal lobar degeneration, and/or the diseases of the present invention and the like;
the human monoclonal antibody L1/L2 of the present invention for use in prevention or treatment of Alzheimer's disease or frontotemporal lobar degeneration, and/or the diseases of the present invention and the like; and
use of the human monoclonal antibody L1/L2 of the present invention for manufacturing an agent for preventing or treating Alzheimer's disease or frontotemporal lobar degeneration, and/or the diseases of the present invention and the like.

### Effect of the Invention

When the human monoclonal antibody of the present invention is used, the diseases of the present invention and the like can be prevented or treated without causing noticeable adverse reactions. Also, when the human monoclonal antibody L1/L2 of the present invention is used, Alzheimer's disease or frontotemporal lobar degeneration, and/or the diseases of the present invention and the like can be prevented or treated without causing noticeable adverse reactions.

### Brief Description of Drawings

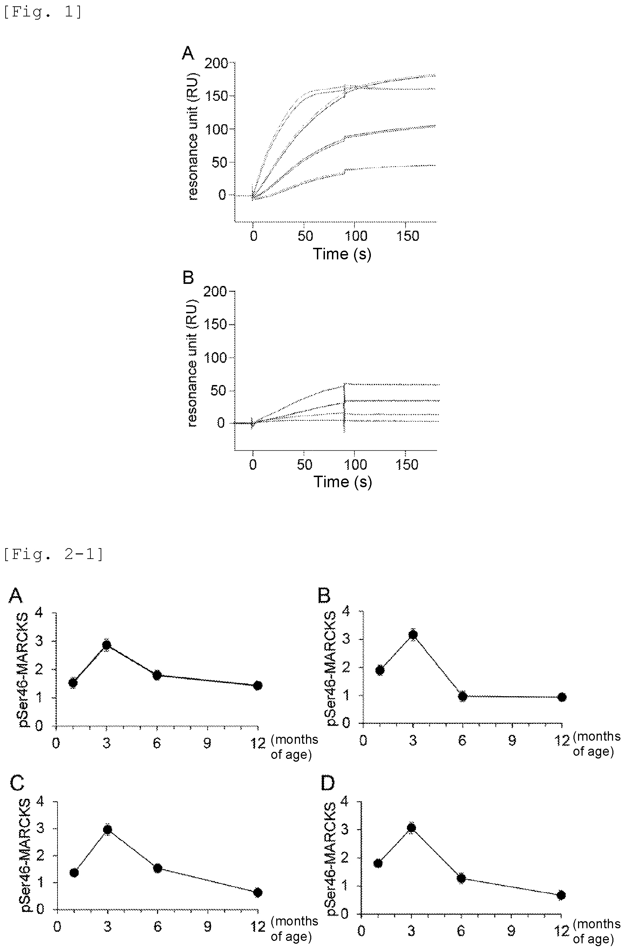

[Figure 1] Figure 1 shows the results of a surface plasmon resonance (SPR) analysis of interactions between two types of anti-human HMGB1 antibodies (the human monoclonal antibody #129 of the present invention [Figure 1A] and a mouse monoclonal antibody 2C8C [Figure 1B]) and the human HMGB1 protein.

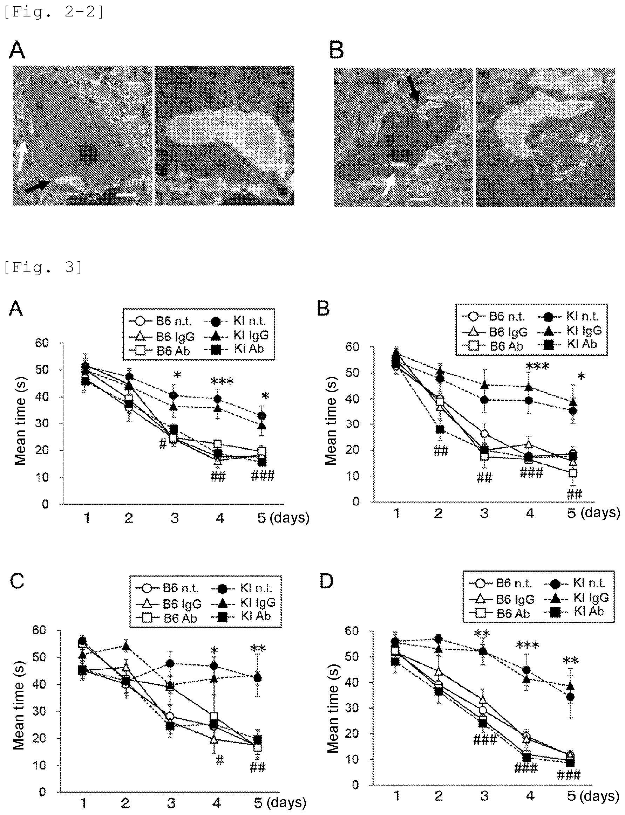

[Figure 2-1] Figure 2-1 shows the results of an immunohistochemical staining analysis of pSer46-MARCKS levels in the cerebral cortex in four types of FTLD-ALS model mice (VCP^{T262A}-KI mice [Figure 2-1A], PGRN^{R504X}-KI mice [Figure 2-1B], CHMP2B^{Q165x}-KI mice [Figure 2-1C], and TDP-43^{N267S}-KI mice [Figure 2-1D]). Of note, all the similarly quantified pSer46-MARCKS levels in C57BL/6J mice were 0 (zero).
[Figure 2-2] Figure 2-2 shows the results of a Nissl's staining analysis of the brain tissue in two types of FTLD-ALS model mice (VCP^{T262A}-KI mice [Figure 2-2A] and PGRN^{R504X}-KI mice [Figure 2-2B]). The arrow in the left image points at a vacuole. The right image is a highly magnified image of the region pointed at by the black arrow in the left image.
[Figure 3] Figure 3A shows the results of a Morris water maze test performed at one to five days from 10 months of age in TDP-43^{N267S}-KI mice ("KI Ab" in the figure [n = 12]) and C57BL/6J mice ("B6 Ab" in the figure [n = 8]) to which the human monoclonal antibody #129 of the present invention was administered, TDP-43^{N267S}-KI mice ("KI IgG" in the figure [n = 11]) and C57BL/6J mice ("B6 IgG" in the figure [n = 6]) to which a control human IgG was administered, and TDP-43^{N267S}-KI mice ("KI n.t." in the figure [n = 10]) and C57BL/6J mice ("B6 n.t." in the figure [n = 6]) which were not treated. Figure 3B shows the results of a Morris water maze test performed at one to five days from seven months of age in PGRN^{R504X}-KI mice ("KI Ab" in the figure [n = 11]) and C57BL/6J mice ("B6 Ab" in the figure [n = 6]) to which the human monoclonal antibody #129 of the present invention was administered, PGRN^{R504X}-KI mice ("KI IgG" in the figure [n = 7]) and C57BL/6J mice ("B6 IgG" in the figure [n = 7]) to which a control human IgG was administered, and PGRN^{R504X}-KI mice ("KI n.t." in the figure [n = 11]) and C57BL/6J mice ("B6 n.t." in the figure [n = 7]) which were not treated. Figure 3C shows the results of a Morris water maze test performed at one to five days from 10 months of age in VCP^{T262A}-KI mice ("KI Ab" in the figure [n = 13]) and C57BL/6J mice ("B6 Ab" in the figure [n = 6]) to which the human monoclonal antibody #129 of the present invention was administered, VCP^{T262A}-KI mice ("KI IgG" in the figure [n = 5]) and C57BL/6J mice ("B6 IgG" in the figure [n = 7]) to which a control human IgG was administered, and VCP^{T262A}-KI mice ("KI n.t." in the figure [n = 7]) and C57BL/6J mice ("B6 n.t." in the figure [n = 6]) which were not treated. Figure 3D shows the results of a Morris water maze test performed at one to five days from six months of age in CHMP2B^{Q165X}-KI mice ("KI Ab" in the figure [n = 7]) and C57BL/6J mice ("B6 Ab" in the figure [n = 6]) to which the human monoclonal antibody #129 of the present invention was administered, CHMP2B^{Q165X}-KI mice ("KI IgG" in the figure [n = 5]) and C57BL/6J mice ("B6 IgG" in the figure [n = 7]) to which a control human IgG was administered, and CHMP2B^{Q165X}-KI mice ("KI n.t." in the figure [n = 5]) and C57BL/6J mice ("B6 n.t." in the figure [n = 6]) which were not treated. In the figures, "*," "**," and "***" indicate that the results of "KI n.t." showed a statistically significant difference from those of "B6 n.t." in a Tukey's HSD test (p < 0.05, p < 0.01, and p < 0.001, respectively). Further, in the figures, "#," "##," and "###" indicate that the results of "KI Ab" showed a statistically significant difference from those of "KI n.t." in a Tukey's HSD test (p < 0.05, p < 0.01, and p < 0.001, respectively).

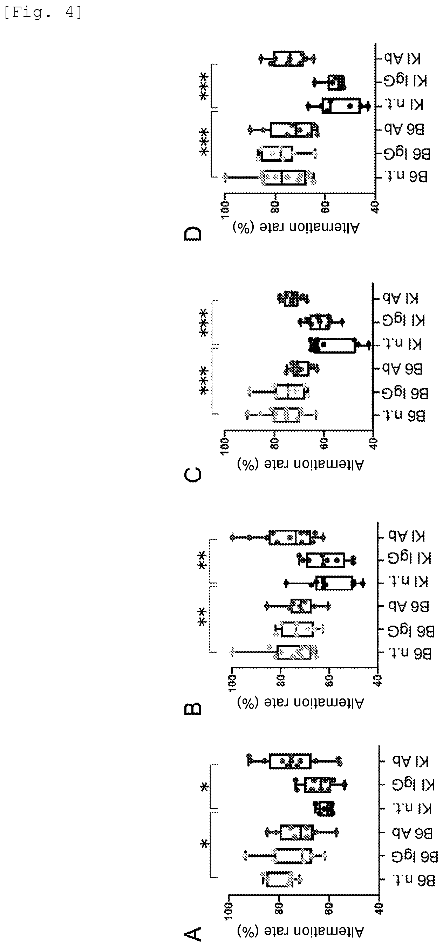

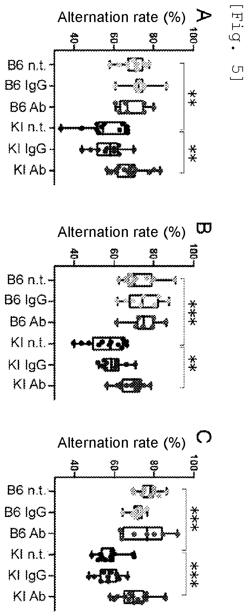

[Figure 4] Figure 4 shows the results of a Y-maze test performed at seven months of age (Figure 4A), eight months of age (Figure 4B), nine months of age (Figure 4C), and 10 months of age (Figure 4D) in TDP-43^{N267S}-KI mice ("KI Ab" in the figures [n = 12 (Figures 4A to 4C); n = 9 (Figure 4D)]) and C57BL/6J mice ("B6 Ab" in the figures [n = 8 (Figures 4A to 4D)]) to which the human monoclonal antibody #129 of the present invention was administered, TDP-43^{N267S}-KI mice ("KI IgG" in the figures [n = 9 (Figures 4A to 4C); n = 6 (Figure 4D)]) and C57BL/6J mice ("B6 IgG" in the figures [n = 7 (Figures 4A to 4D)]) to which a control human IgG was administered, and TDP-43^{N267S}-KI mice ("KI n.t." in the figures [n = 6 (Figure 4A); n = 10 (Figure 4B); n = 9 (Figure 4C); n = 7 (Figure 4D)]) and C57BL/6J mice ("B6 n.t." in the figures [n = 6 (Figure 4A); n = 12 (Figures 4B to 4D)]) which were not treated. In the figures, "*," "**," and "***" indicate that a statistically significant difference was detected in a Tukey's HSD test (p < 0.05, p < 0.01, and p < 0.001, respectively) (hereunder, same in Figures 5 to 16).
[Figure 5] Figure 5 shows the results of a Y-maze test performed at five months of age (Figure 5A), six months of age (Figure 5B), and seven months of age (Figure 5C) in PGRN^{R504X}-KI mice ("KI Ab" in the figures [n = 15 (Figures 5A and 5B); n = 14 (Figure 5C)]) and C57BL/6J mice ("B6 Ab" in the figures [n = 7 (Figures 5A to 5C)]) to which the human monoclonal antibody #129 of the present invention was administered, PGRN^{R504X}-KI mice ("KI IgG" in the figures [n = 10 (Figures 5A to 5C)]) and C57BL/6J mice ("B6 IgG" in the figures [n = 8 (Figures 5A to 5C)]) to which a control human IgG was administered, and PGRN^{R504X}-KI mice ("KI n.t." in the figures [n = 11 (Figures 5A and 5C); n = 12 (Figure 5B)]) and C57BL/6J mice ("B6 n.t." in the figure [n = 7 (Figure 5A); n = 11 (Figures 5B and 5C)]) which were not treated.

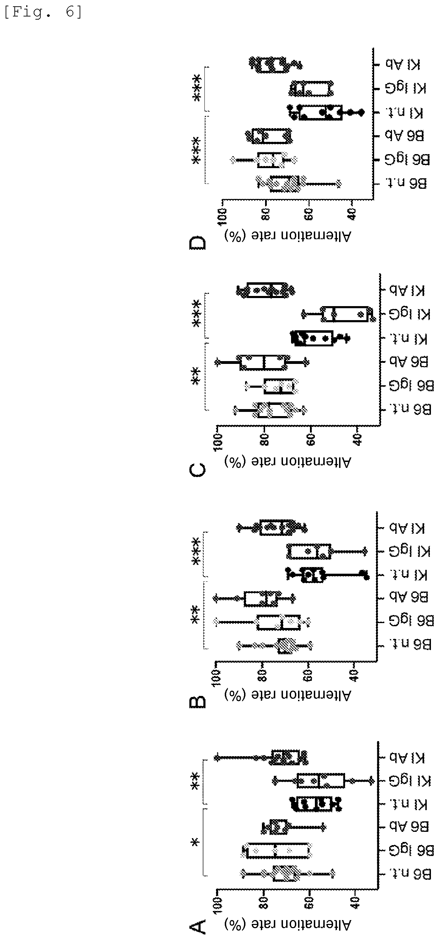

[Figure 6] Figure 6 shows the results of a Y-maze test performed at seven months of age (Figure 6A), eight months of age (Figure 6B), nine months of age (Figure 6C), and 10 months of age (Figure 6D) in VCP^{T262A}-KI mice ("KI Ab" in the figures [n = 15 (Figures 6A to 6C); n = 12 (Figure 6D)]) and C57BL/6J mice ("B6 Ab" in the figures [n = 8 (Figures 6A to 6C); n = 5 (Figure 6D)]) to which the human monoclonal antibody #129 of the present invention was administered, VCP^{T262A}-KI mice ("KI IgG" in the figures [n = 8 (Figure 6A); n = 7 (Figures 6B and 6C); n = 5 (Figure 6D)]) and C57BL/6J mice ("B6 IgG" in the figures [n = 7 (Figures 6A to 6C); n = 6 (Figure 6D)]) to which a control human IgG was administered, and VCP^{T262A}-KI mice ("KI n.t." in the figures [n = 11 (Figures 6A to 6C); n = 5 (Figure 6D)]) and C57BL/6J mice ("B6 n.t." in the figures [n = 17 (Figure 6A); n = 16 (Figure 6B); n = 13 (Figure 6C); n = 12 (Figure 6D)]) which were not treated.

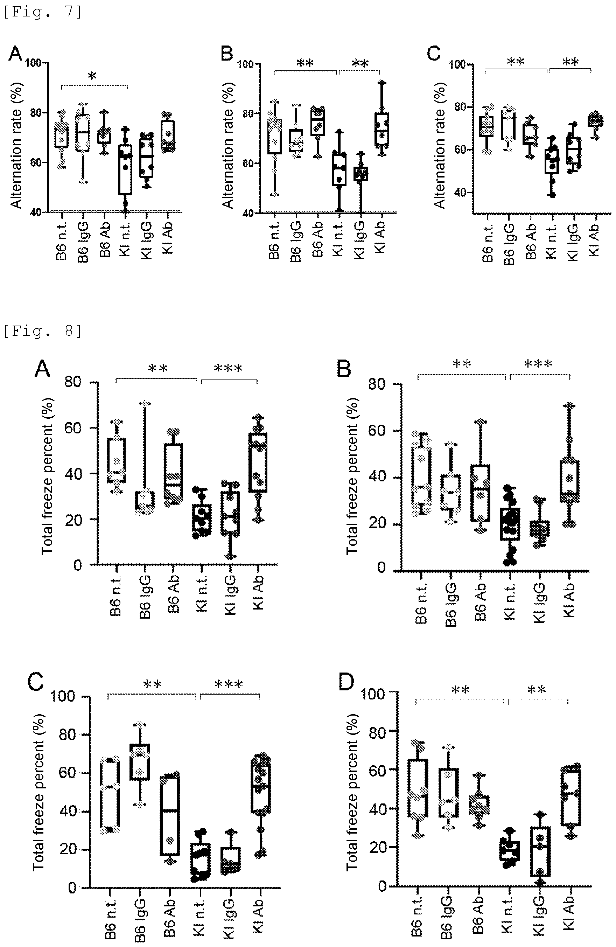

[Figure 7] Figure 7 shows the results of a Y-maze test performed at four months of age (Figure 7A), five months of age (Figure 7B), and six months of age (Figure 7C) in CHMP2B^{Q165x}-KI mice ("KI Ab" in the figures [n = 8 (Figures 7A to 7C)]) and C57BL/6J mice ("B6 Ab" in the figures [n = 8 (Figures 7A to 7C)]) to which the human monoclonal antibody #129 of the present invention was administered, CHMP2B^{Q165X}-KI mice ("KI IgG" in the figures [n = 8 (Figures 7A to 7C)]) and C57BL/6J mice ("B6 IgG" in the figures [n = 8 (Figures 7A to 7C)]) to which a control human IgG was administered, and CHMP2B^{Q165X}-KI mice ("KI n.t." in the figures [n = 8 (Figures 7A to 7C)]) and C57BL/6J mice ("B6 n.t." in the figures [n = 12 (Figure 7A); n = 10 (Figure 7B); n = 10 (Figure 7C)]) which were not treated.
[Figure 8] Figure 8A shows the results of a fear-conditioning test performed at 10 months of age in TDP-43^{N267S}-KI mice ("KI Ab" in the figure [n = 12]) and C57BL/6J mice ("B6 Ab" in the figure [n = 8]) to which the human monoclonal antibody #129 of the present invention was administered, TDP-43^{N267S}-KI mice ("KI IgG" in the figure [n = 9]) and C57BL/6J mice ("B6 IgG" in the figure [n = 7]) to which a control human IgG was administered, and TDP-43^{N267S}-KI mice ("KI n.t." in the figure [n = 9]) and C57BL/6J mice ("B6 n.t." in the figure [n = 7]) which were not treated. Figure 8B shows the results of a fear-conditioning test performed at seven months of age in PGRN^{R504X}-KI mice ("KI Ab" in the figure [n = 11]) and C57BL/6J mice ("B6 Ab" in the figure [n = 6]) to which the human monoclonal antibody #129 of the present invention was administered, PGRN^{R504X}-KI mice ("KI IgG" in the figure [n = 10]) and C57BL/6J mice ("B6 IgG" in the figure [n = 7]) to which a control human IgG was administered, and PGRN^{R504X}-KI mice ("KI n.t." in the figure [n = 17]) and C57BL/6J mice ("B6 n.t." in the figure [n = 11]) which were not treated. Figure 8C shows the results of a fear-conditioning test performed at 10 months of age in VCP^{T262A}-KI mice ("KI Ab" in the figure [n = 15]) and C57BL/6J mice ("B6 Ab" in the figure [n = 4]) to which the human monoclonal antibody #129 of the present invention was administered, VCP^{T262A}-KI mice ("KI IgG" in the figure [n = 5]) and C57BL/6J mice ("B6 IgG" in the figure [n = 6]) to which a control human IgG was administered, and VCP^{T262A}-KI mice ("KI n.t." in the figure [n = 9]) and C57BL/6J mice ("B6 n.t." in the figure [n = 6]) which were not treated. Figure 8D shows the results of a fear-conditioning test performed at six months of age in CHMP2B^{Q165X}-KI mice ("KI Ab" in the figure [n = 7]) and C57BL/6J mice ("B6 Ab" in the figure [n = 8]) to which the human monoclonal antibody #129 of the present invention was administered, CHMP2B^{Q165X}-KI mice ("KI IgG" in the figure [n = 5]) and C57BL/6J mice ("B6 IgG" in the figure [n = 6]) to which a control human IgG was administered, and CHMP2B^{Q165X}-KI mice ("KI n.t." in the figure [n = 7]) and C57BL/6J mice ("B6 n.t." in the figure [n = 8]) which were not treated.

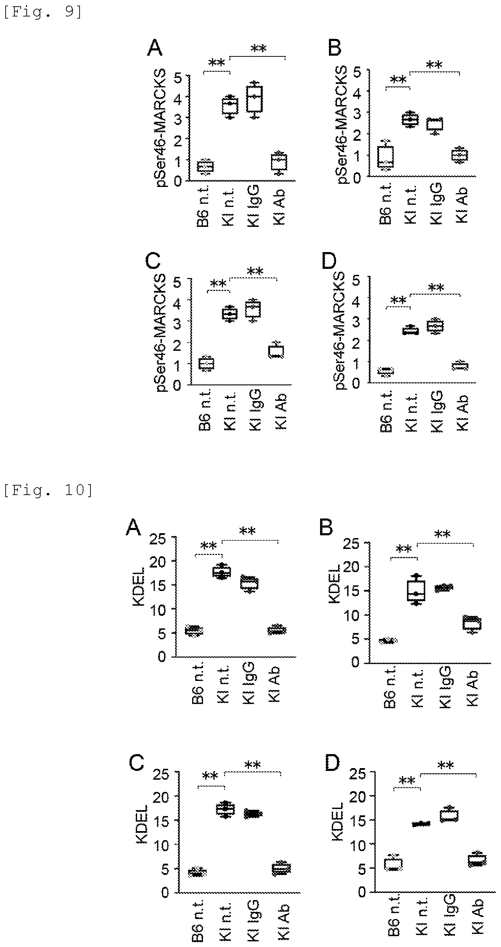

[Figure 9] Figure 9 shows the results (n = 3) of an immunohistochemical staining analysis of pSer46-MARCKS levels in the cerebral cortex in four types of FTLD-ALS model mice (PGRN^{R504X}-KI mice [Figure 9A], TDP-43^{N267S}-KI mice [Figure 9B], VCP^{T262A}-KI mice [Figure 9C], and CHMP2B^{Q165X}-KI mice [Figure 9D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .
[Figure 10] Figure 10 shows the results (n = 3) of an immunohistochemical staining analysis of KDEL fluorescence levels in the cerebral cortex in four types of FTLD-ALS model mice (PGRN^{R504X}-KI mice [Figure 10A], TDP-43^{N267S}-KI mice [Figure 10B], VCP^{T262A}-KI mice [Figure 10C], and CHMP2B^{Q165X}-KI mice [Figure 10D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figure), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .

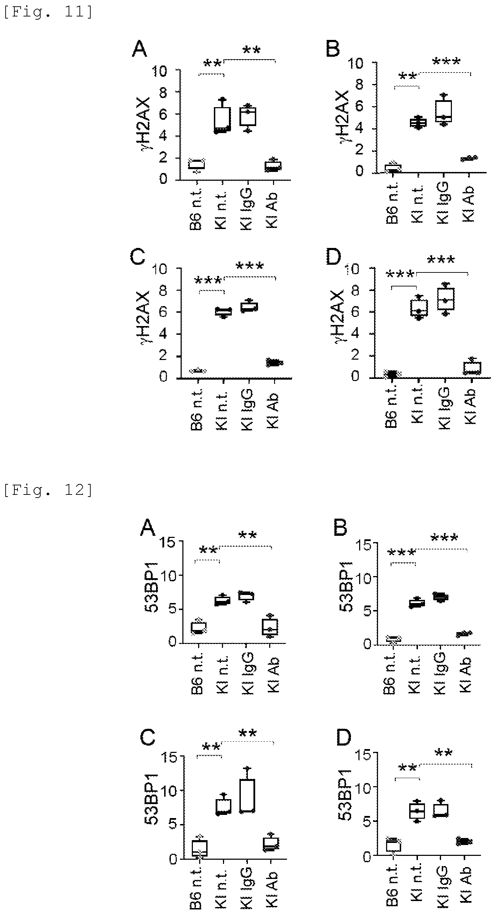

[Figure 11] Figure 11 shows the results (n = 3) of an immunohistochemical staining analysis of γH2AX fluorescence levels in the cerebral cortex in four types of FTLD-ALS model mice (PGRN^{R504X}-KI mice [Figure 11A], TDP-43^{N267S}-KI mice [Figure 11B], VCP^{T262A}-KI mice [Figure 11C], and CHMP2B^{Q165X}-KI mice [Figure 11D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .
[Figure 12] Figure 12 shows the results (n = 3) of an immunohistochemical staining analysis of 53BP1 fluorescence levels in the cerebral cortex in four types of FTLD-ALS model mice (PGRN^{R504X}-KI mice [Figure 12A], TDP-43^{N267S}-KI mice [Figure 12B], VCP^{T262A}-KI mice [Figure 12C], and CHMP2B^{Q165X}-KI mice [Figure 12D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .

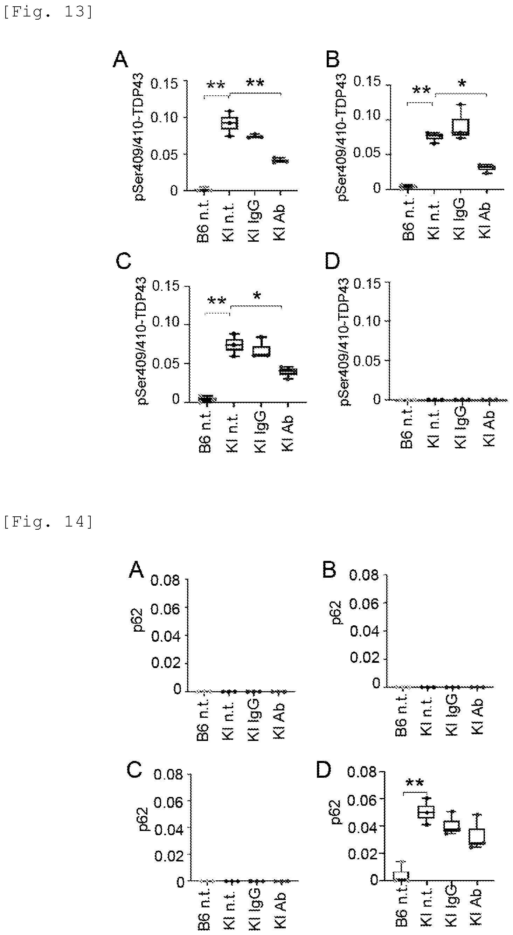

[Figure 13] Figure 13 shows the results (n = 3) of an immunohistochemical staining analysis of pSer409/410-TDP-43 fluorescence levels in the cerebral cortex in four types of FTLD-ALS model mice (PGRNR^{R504X}-KI mice [Figure 13A], TDP-43^{N267S}-KI mice [Figure 13B], VCP^{T262A}-KI mice [Figure 13C], and CHMP2B^{Q165X}-KI mice [Figure 13D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KT mice), and 10 months of age (C57BL/6J mice, TDP-43^{N267S}-KI mice, and VCP^{T262A}-KI mice).
[Figure 14] Figure 14 shows the results (n = 3) of an immunohistochemical staining analysis of p62 fluorescence levels in the cerebral cortex in four types of FTLD-ALS model mice (PGRN^{R504X}-KI mice [Figure 14A], TDP-43^{N267S}-KI mice [Figure 14B], VCP^{T262A}- KI mice [Figure 14C], and CHMP2B^{Q165X}-KI mice [Figure 14D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .

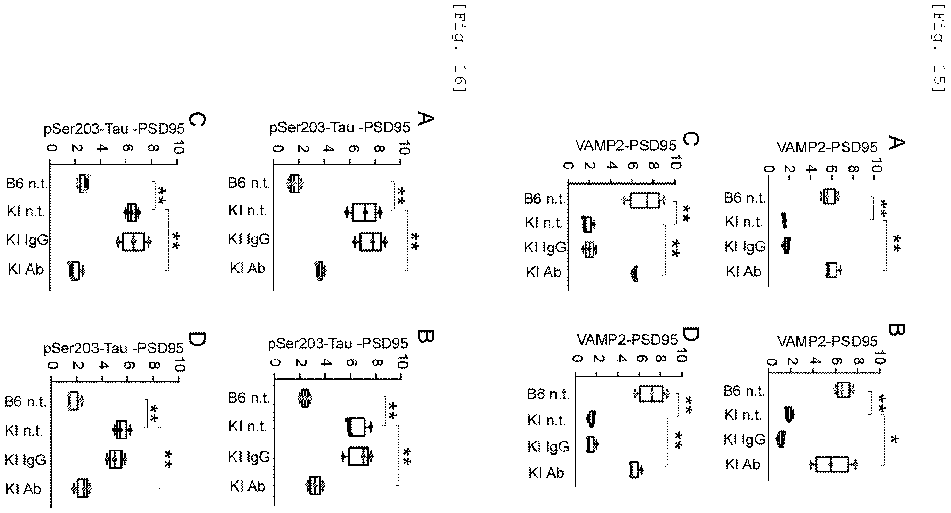

[Figure 15] Figure 15 shows the results (n = 3) of an immunohistochemical staining analysis of the proportion of cells having a VAMP2 fluorescence signal and a PSD95 fluorescence signal colocalized therein in the cerebral cortex in four types of FTLD-ALS model mice (PGRN^{R504X}-KI mice [Figure 15A], TDP-43^{N267S}-KI mice [Figure 15B], VCP^{T262A}-KI mice [Figure 15C], and CHMP2B^{Q165X}-KI mice [Figure 15D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KT mice), and 10 months of age (C57BL/6J mice, TDP-43^{N267S}-KI mice, and VCP^{T262A}- KI mice) .
[Figure 16] Figure 16 shows the results (n = 3) of an immunohistochemical staining analysis of the proportion of cells having a pSer203-tau fluorescence signal and a PSD95 fluorescence signal colocalized therein in the cerebral cortex in four types of FTLD-ALS model mice (PGRN^{R504X}-KI mice [Figure 16A], TDP-43^{N267S}-KI mice [Figure 16B], VCP^{T262A}-KI mice [Figure 16C], and CHMP2B^{Q165X}-KI mice [Figure 16D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}- KI mice) .

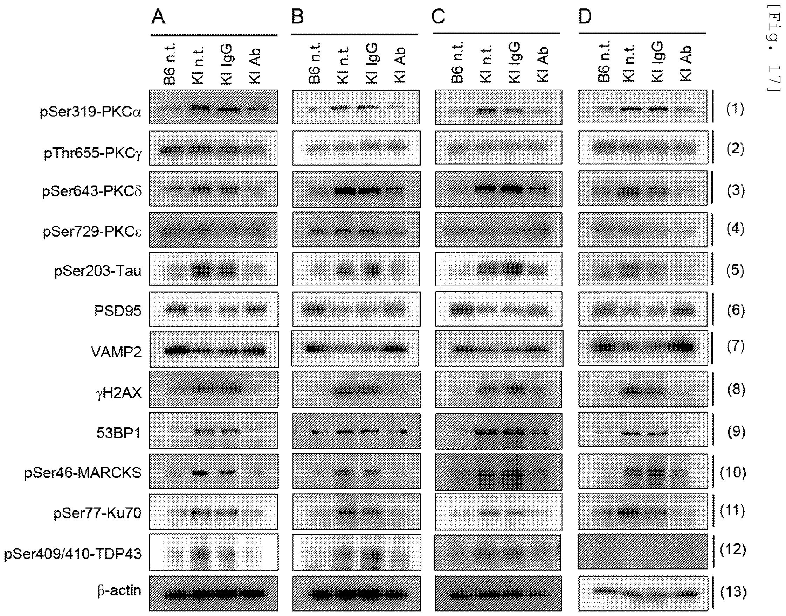

[Figure 17] Figure 17 shows the results of a Western blot analysis of the phosphorylation levels of four types of proteins associated with the core signaling in AD-FTLD (pSer319-PKCα, pThr655-PKCγ, pSer643-PKCδ, and pSer729-PKCε: Lanes (1) to (4), respectively), the phosphorylation level of one type of synapse instability-associated protein (pSer203-tau: Lane (5)), the expression levels of two types of synapse-associated proteins (PSD95 and VAMP2: Lanes (6) and (7), respectively), the expression levels of two types of DNA damage-associated proteins (γH2AX and 53BP1: Lanes (8) and (9), respectively), the phosphorylation level of one type of cell death-associated protein (pSer46-MARCKS: Lane (10)), the phosphorylation level of one type of DNA repair-associated protein (pSer77/78-Ku70: Lane (11)), the level of one type of protein agglutination marker (pSer409/410-TDP-43: Lane (12)), and the expression level of β-actin, which is an internal standard protein (Lane (13)), in the cerebral cortex in four types of FTLD-ALS model mice (TDP-43^{N267S}-KI mice [Figure 17A], PGRN^{R504X}-KI mice [Figure 17B], VCP^{T262A}-KI mice [Figure 17C], and CHMP2B^{Q165X}-KI mice [Figure 17D]) to which the human monoclonal antibody #129 of the present invention was administered ("KI Ab" in the figures), the four types of FTLD-ALS model mice to which a control human IgG was administered ("KI IgG" in the figures), the four types of FTLD-ALS model mice which were not treated ("KI n.t." in the figures), and C57BL/6J mice which were not treated ("B6 n.t." in the figures) at six months of age (C57BL/6J mice and CHMP2B^{Q165X}-KI mice), seven months of age (C57BL/6J mice and PGRN^{R504X}-KI mice), and 10 months of age (C57BL/6J mice, TDP-43^{N267S}-KI mice, and VCP^{T262A}-KI mice) .

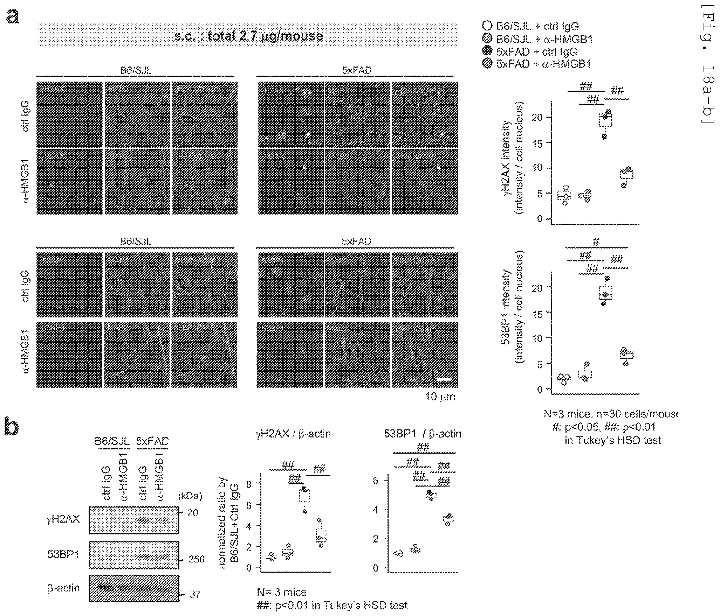

[Figures 18a-b] Figure 18a shows the results of an immunohistochemical staining analysis of DNA double-strand break levels of MAP2-positive neurons in the cerebral cortex in four groups of mice (non-transgenic sibling mice [B6/SJL mice] to which the human monoclonal antibody #129 of the present invention was subcutaneously administered into the back and neck region [hereinafter simply referred to as "subcutaneously administered"]; B6/SJL mice to which a control IgG was subcutaneously administered; 5 × FAD mice to which the human monoclonal antibody #129 of the present invention was subcutaneously administered; and 5 × FAD mice to which the control IgG was subcutaneously administered), using antibodies for two types of markers (γH2AX and 53BP1). The right graphs show the results (N = 3 mice, and n = 30 cells) of quantified fluorescence intensity detected using the antibodies. Figure 18b shows the results (n = 3) of a Western blot analysis of the entire cerebral cortex in the above-mentioned four groups of mice, using an anti-γH2AX antibody and an anti-53BP1 antibody.

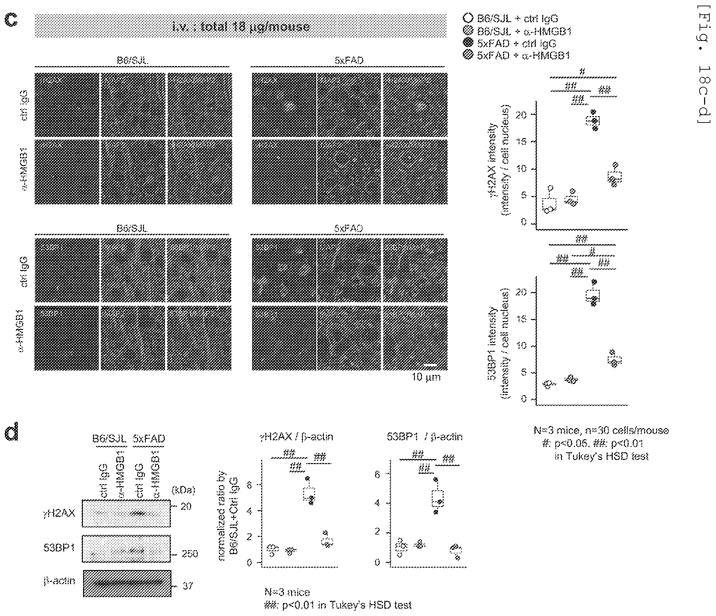

[Figures 18c-d] Figure 18c shows the results of an immunohistochemical staining analysis of DNA double-strand break levels of MAP2-positive neurons in the occipital cortical neurons in four groups of mice (B6/SJL mice to which the human monoclonal antibody #129 of the present invention was intravenously administered; B6/SJL mice to which a control IgG was intravenously administered; 5 × FAD mice to which the human monoclonal antibody #129 of the present invention was intravenously administered; and 5 × FAD mice to which the control IgG was intravenously administered), using antibodies for two types of markers (γH2AX and 53BP1). The right graphs show the results (N = 3 mice, and n = 30 cells) of quantified fluorescence intensity detected using the antibodies. Figure 18d shows the results (n = 3) of a Western blot analysis of the entire cerebral cortex in the above-mentioned four groups of mice, using the anti-γH2AX antibody and the anti-53BP1 antibody.

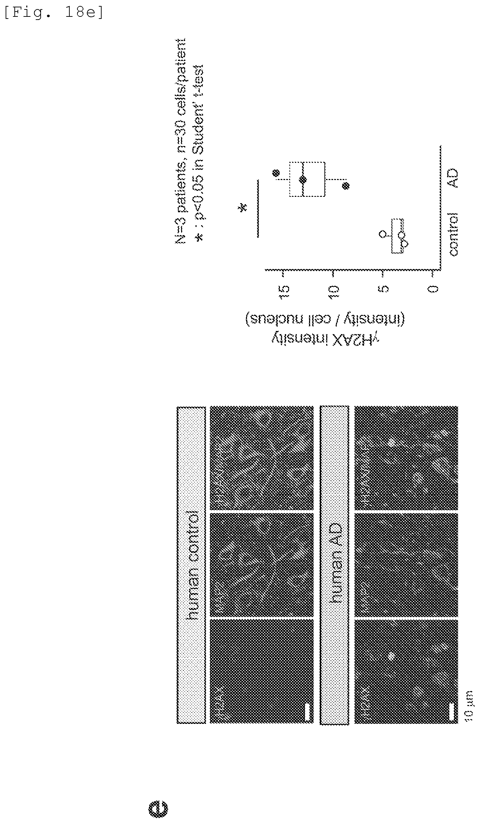

[Figure 18e] Figure 18e shows the results of an immunohistochemical staining analysis of the brain tissue samples (occipital lobe) in patients without neurological disease and the brain tissue samples (occipital lobe) in AD patients, using the anti-γH2AX antibody.

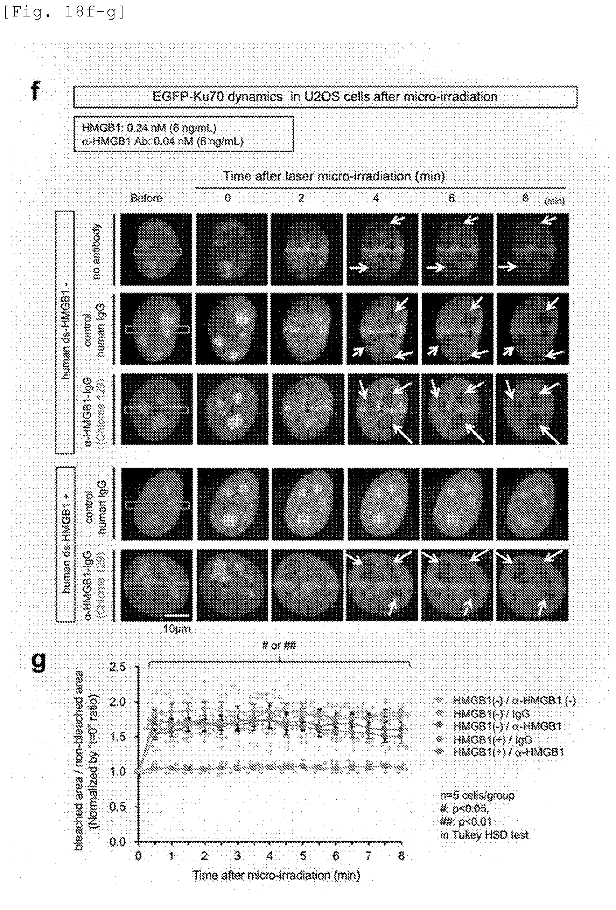

[Figures 18f-g] Figure 18f shows the results of an imaging analysis of the dynamic localization of Ku70 to DNA damage sites in which EGFP-Ku70 was transiently expressed in U2OS cells cultured in the presence (+) or absence (-) of 6 ng/mL (0.24 nM) ds-HMGB1 and that were induced by micro-irradiation. Figure 18g shows the results (n = 5, mean value ± S.E.M.) of a quantitative analysis of the localization levels of EGFP-Ku70 to DNA damage sites, based on time-lapse images of the U2OS cells (n = 5) obtained in Figure 18f.

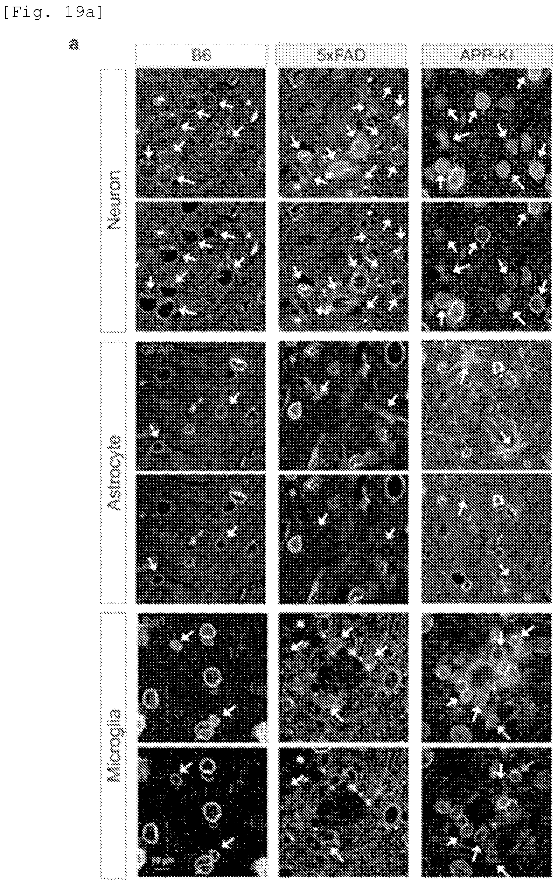

[Figure 19a] Figure 19a shows the results of an immunohistochemical staining analysis of the nerve cells, astrocytes and microglia in three types of mice (C57BL/6J mice, 5 × FAD mice, and APP-KI mice), using an anti-Lamin B1 antibody.

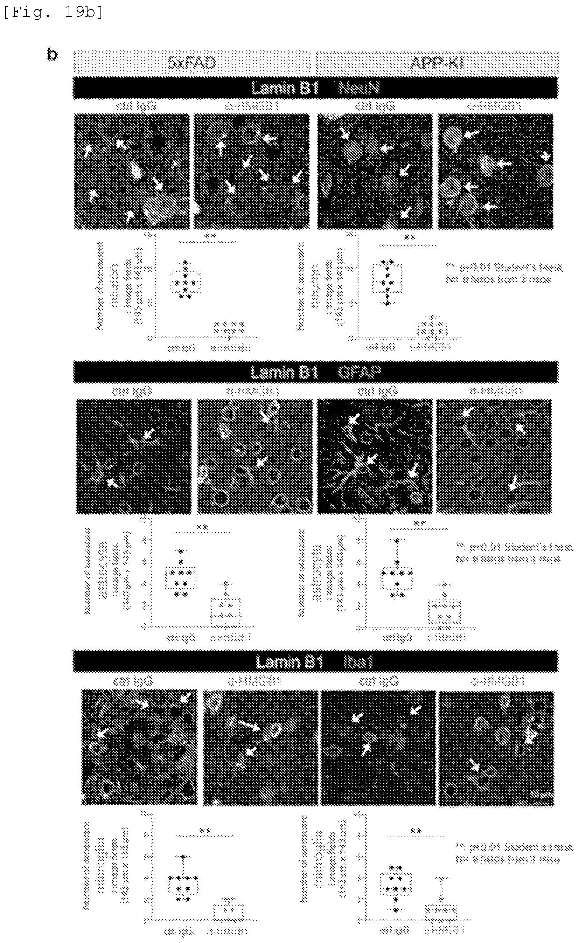

[Figure 19b] Figure 19b shows the results of an immunohistochemical staining analysis of the nerve cells, astrocytes and microglia in two types of AD model mice (5 × FAD mice and APP-KI mice) to which the human monoclonal antibody #129 of the present invention or the control IgG was intravenously administered, using the anti-Lamin B1 antibody and antibodies for three types of senescence markers ("NeuN" in the nerve cells, "GFAP" in the astrocytes and "Iba1" in the microglia). The graphs in the figure show the results obtained by measuring the proportions of these senescence marker-positive cells to show the results of measured proportions of the senescent cells.

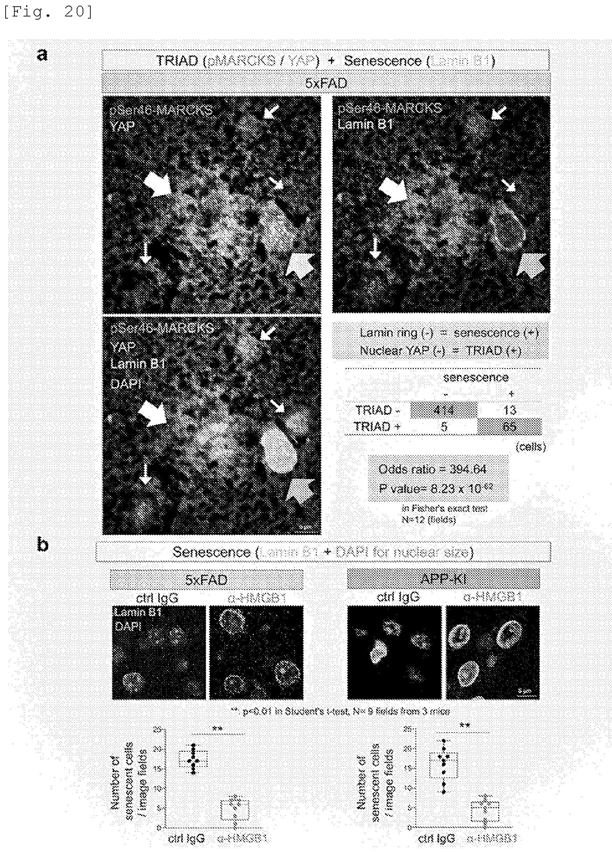

[Figure 20] Figure 20a shows the results of an immunohistochemical staining analysis of the cerebral cortex in AD model mice at eight months of age (5 × FAD mice), using three types of antibodies (anti-YAP antibody, anti-pSer46-MARCKS antibody and anti-Lamin B1 antibody). Figure 20b shows the results of an immunohistochemical staining analysis of the cerebral cortex in two types of AD model mice (5 × FAD mice and APP-KI mice) to which the human monoclonal antibody #129 of the present invention or the control IgG was intravenously administered, using the anti-Lamin B1 antibody to analyze proportions of the senescent cells (that is, Lamin B1 ring-negative cells).

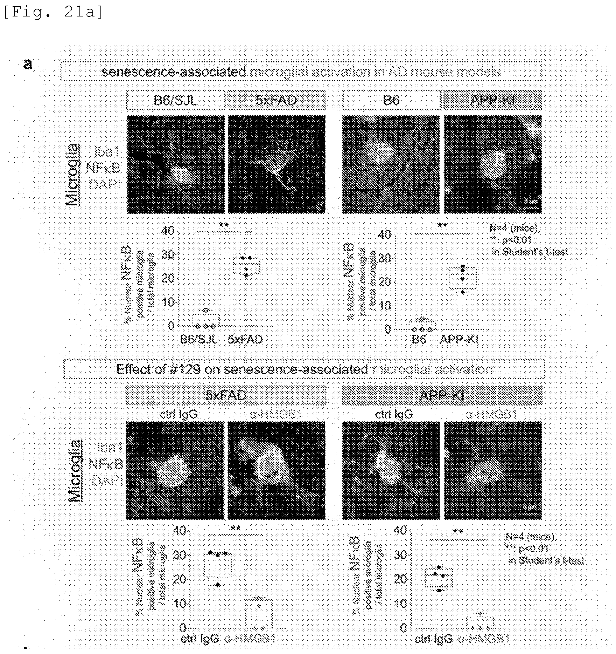

[Figure 21a] Figure 21a shows in the upper part the results of an immunohistochemical staining analysis of the NFκB nuclear translocation levels of the microglia in two types of normal mice (B6/SJL mice or C57BL/6 mice [B6]) or two types of AD model mice (5 × FAD mice and APP-KI mice) both at eight months of age, using an anti-NFκB antibody. Figure 21a shows in the lower part the results of an immunohistochemical staining analysis of the NFκB nuclear translocation levels of the microglia in the above-mentioned two types of AD model mice to which the human monoclonal antibody #129 of the present invention or the control IgG was intravenously administered, using the anti-NFκB antibody.

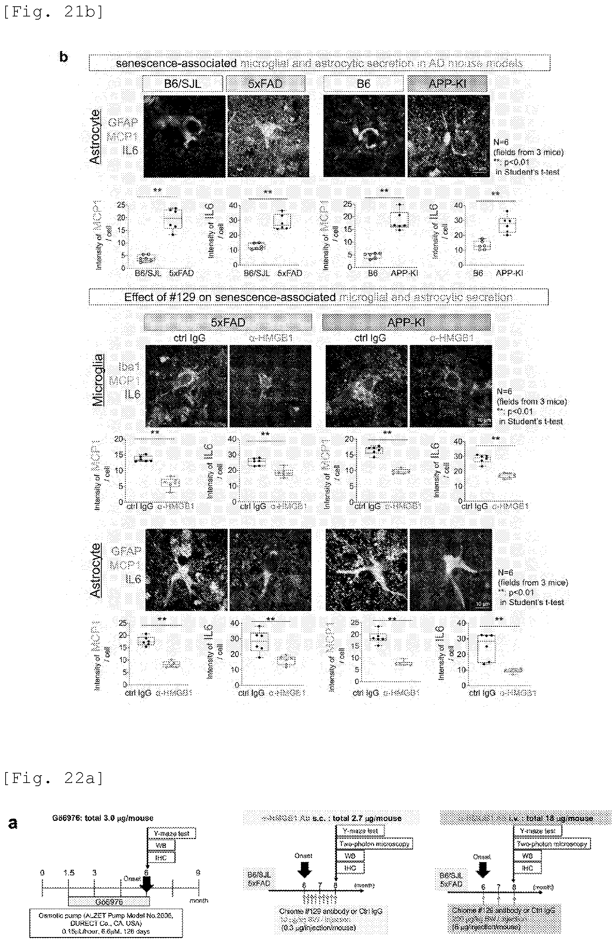

[Figure 21b] Figure 21b shows in the upper part the results of an immunohistochemical staining analysis of the MCP1 and IL6 expression levels around the astrocytes and the microglia in two types of normal mice (B6/SJL mice or C57BL/6 mice [B6]) or two types of AD model mice (5 × FAD mice and APP-KI mice) both at eight months of age, using an anti-MCP1 antibody and an anti-IL6 antibody. Figure 21a shows in the lower part the results of an immunohistochemical staining analysis of the MCP1 and IL6 expression levels around the astrocytes and the microglia in the above-mentioned two types of AD model mice to which the human monoclonal antibody #129 of the present invention or the control IgG was intravenously administered, using the anti-MCP1 antibody and the anti-IL6 antibody.

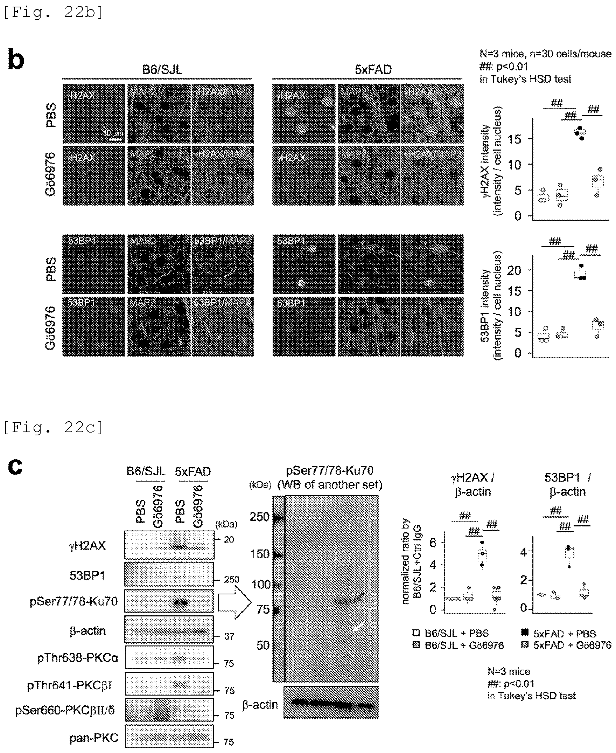

[Figure 22a] Figure 22a schematically shows the experimental protocol of Figures 22b to f.
[Figure 22b] Figure 22b shows the results of an immunohistochemical staining analysis of DNA double-strand break levels of MAP2-positive neurons in the occipital cortical neurons in B6/SJL mice or 5 × FAD mice (left diagram of Figure 22a) to which the PKC inhibitor (Go6976) was subcutaneously administered, using antibodies for two types of markers (γH2AX and 53BP1). The right graphs show the results (N = 3 mice, and n = 30 cells) of quantified fluorescence intensity detected using the antibodies.
[Figure 22c] Figure 22c shows the results of a Western blot analysis of the expression levels of eight types of proteins (yH2AX, 53BP1, pSer77/78-Ku70, β-actin, pThr638-PKCα, pThr641-PKCβI, pSer660-PKCβII/δ, and pan-PKC) in the entire cerebral cortex in B6/SJL mice or 5 × FAD mice (left diagram of Figure 22a) to which the PKC inhibitor (Go6976) was subcutaneously administered.

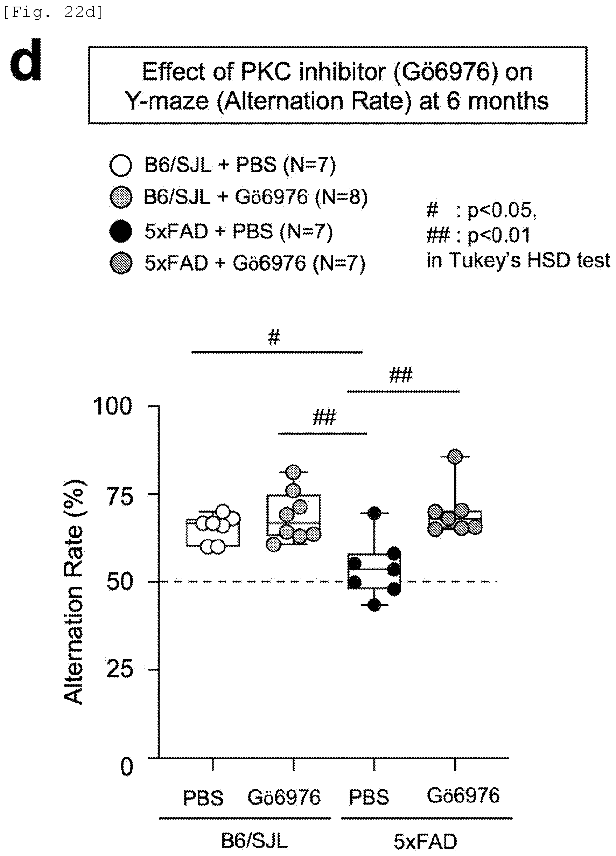

[Figure 22d] Figure 22d shows the results of a Y-maze test performed to B6/SJL mice or 5 × FAD mice (left diagram of Figure 22a) to which the PKC inhibitor (Go6976) was subcutaneously administered.

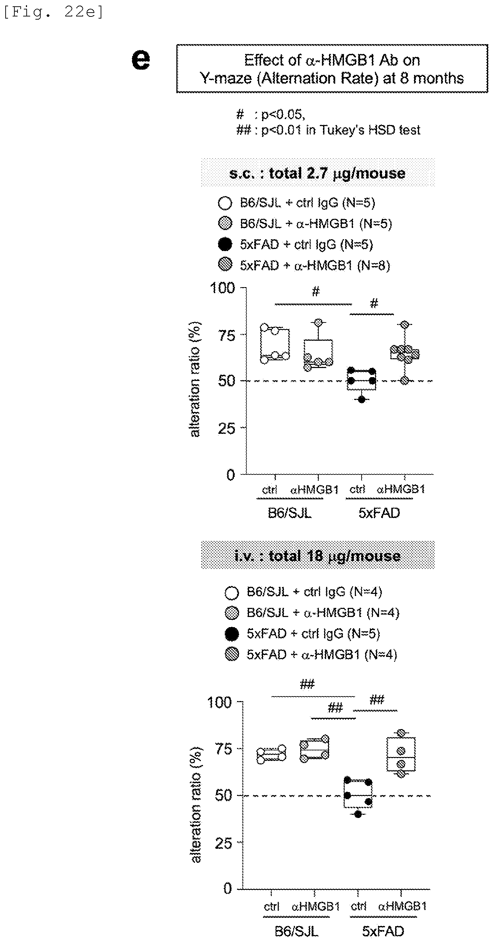

[Figure 22e] Figure 22e shows the results of a Y-maze test performed to B6/SJL mice or 5 × FAD mice (center graph of Figure 22a) to which the human monoclonal antibody #129 of the present invention or a control human IgG was subcutaneously administered.

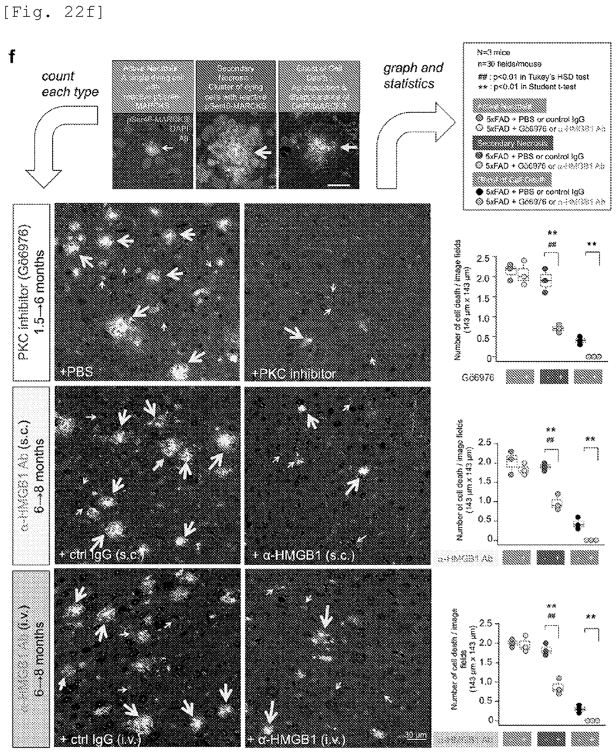

[Figure 22f] Figure 22f shows the results of an immunohistochemical staining of the cerebral cortex in B6/SJL mice or 5 × FAD mice (left diagram of Figure 22a) to which the PKC inhibitor (Go6976) was subcutaneously administered, the cerebral cortex in B6/SJL mice or 5 × FAD mice (center diagram of Figure 22a) to which the human monoclonal antibody #129 of the present invention or the control human IgG was subcutaneously administered, and the cerebral cortex in B6/SJL mice or 5 × FAD mice (right diagram of Figure 22a) to which the human monoclonal antibody #129 of the present invention or the control human IgG was intravenously administered, using an anti-pSer46-MARCKS antibody and an anti-amyloid (A) β antibody to analyze the proportion of active necrosis (two sets on the left side in each graph), secondary necrosis (two sets in the center in each graph), and ghost of cell death (two sets on the right side in each graph).

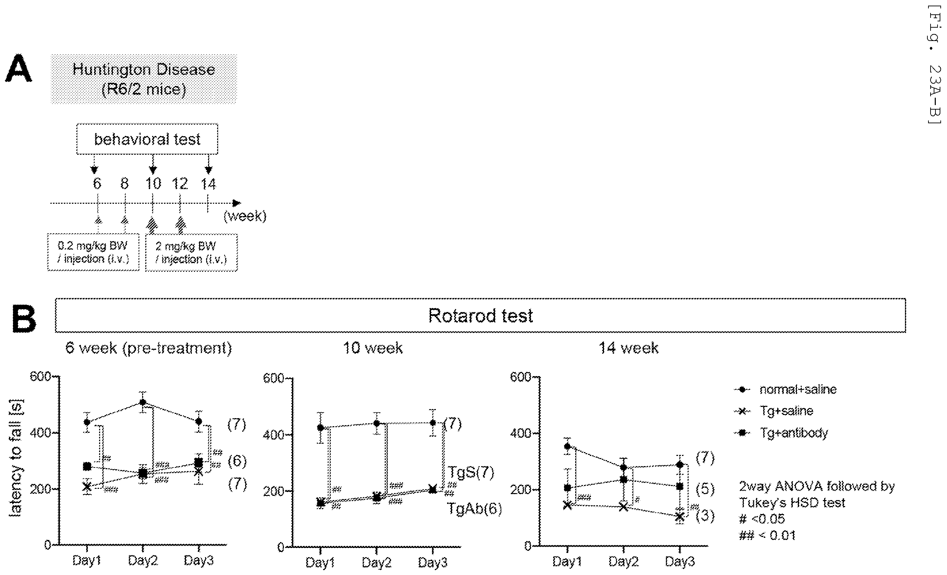

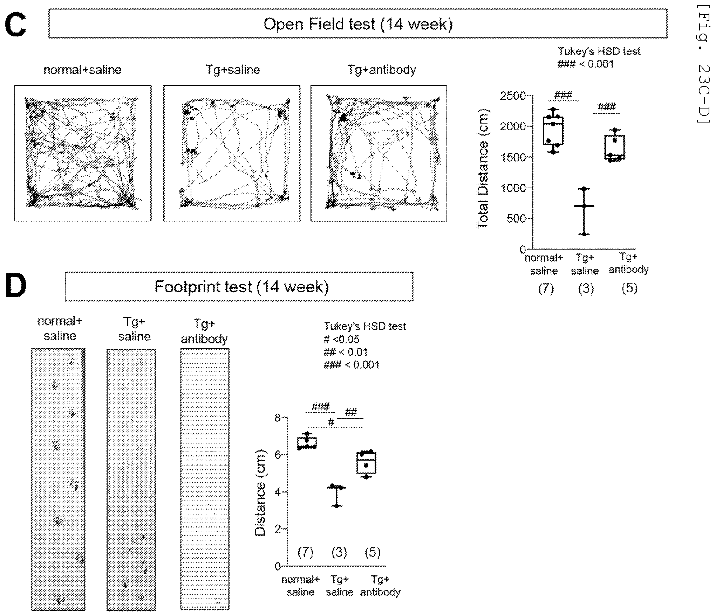

[Figures 23A-B] Figure 23A schematically shows the therapeutic experimental protocol for Huntington's disease model mice (R6/2 mice). Figure 23B shows the results of examining treatment effects of the human monoclonal antibody #129 on Huntington's disease model mice, using a rotarod test. Figure 23B shows the results (mean value ± standard error) of three groups of physiological saline-administered normal mice ("Normal+saline" in the figure; same in Figures 23 to 28), physiological saline-administered R6/2 mice ("Tg+saline" in the figure; same in Figures 23 to 28), and human monoclonal antibody #129-administered R6/2 mice ("Tg+antibody" in the figure; same in Figures 23 to 28) (hereinafter may be referred to as "three groups for analyzing Huntington's disease model mice") for three consecutive days of testing in each week. Numerical values in the brackets indicate the number of animals used for the measurement.
[Figures 23C-D] Figure 23C shows the results of examining treatment effects of the human monoclonal antibody #129 on Huntington's disease model mice, using an open field test. Figure 23D shows the results of examining treatment effects of the human monoclonal antibody #129 on Huntington's disease model mice, using a footprint test. Figures 23C and 23D show the representative examples of the three groups for analyzing Huntington's disease model mice and the measurement results, the box plots of the graphs indicate the maximum value, minimum value, median value, and quartiles (75% and 25%) (the same applies to the box plots in the following graphs), and the numerical values in the brackets indicate the number of animals used for the measurement. Of note, in Figure 23D, strides (distance) were measured in the three groups for analyzing Huntington's disease model mice.

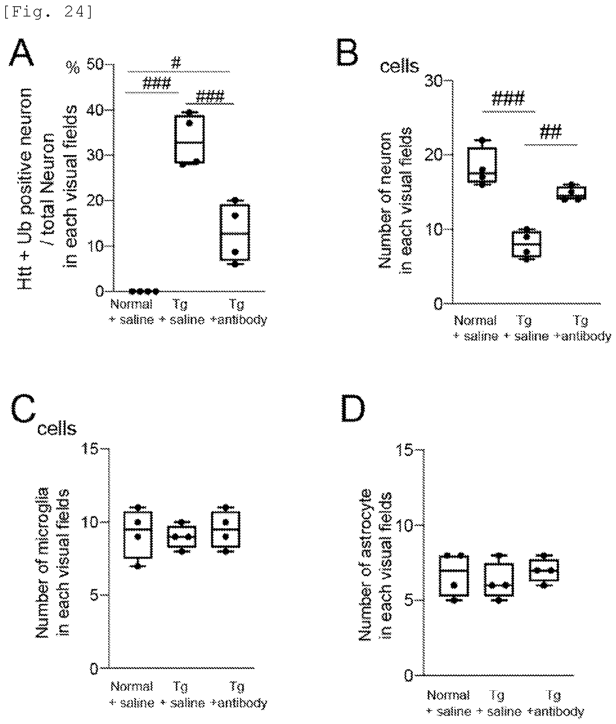

[Figure 24] Figure 24A shows the results (n = 4) obtained by immunohistochemical staining that detects huntingtin (Htt), ubiquitin (Ub) and MAP2 in the cerebral cortex in the three groups for analyzing Huntington's disease model mice and by measuring the percentage (%) of the cells having inclusions positive for both Htt and Ub in the MAP2-positive neurons. Figure 24B shows the results (n = 4) of comparison of the number of MAP2-positive cells (the number of neurons) in the cerebral cortex between the three groups for analyzing Huntington's disease model mice. Figure 24C shows the results (n = 4) of comparison of the number of Iba1-positive cells (the number of microglia) in the cerebral cortex between the three groups for analyzing Huntington's disease model mice. Figure 24D shows the results (n = 4) of comparison of the number of GFAP-positive cells (the number of astrocytes) in the cerebral cortex between the three groups for analyzing Huntington's disease model mice. In the figures, "#," "##," and "###" indicate a statistically significant difference in a Tukey's HSD test (p < 0.05, p < 0.01, and p < 0.001, respectively).

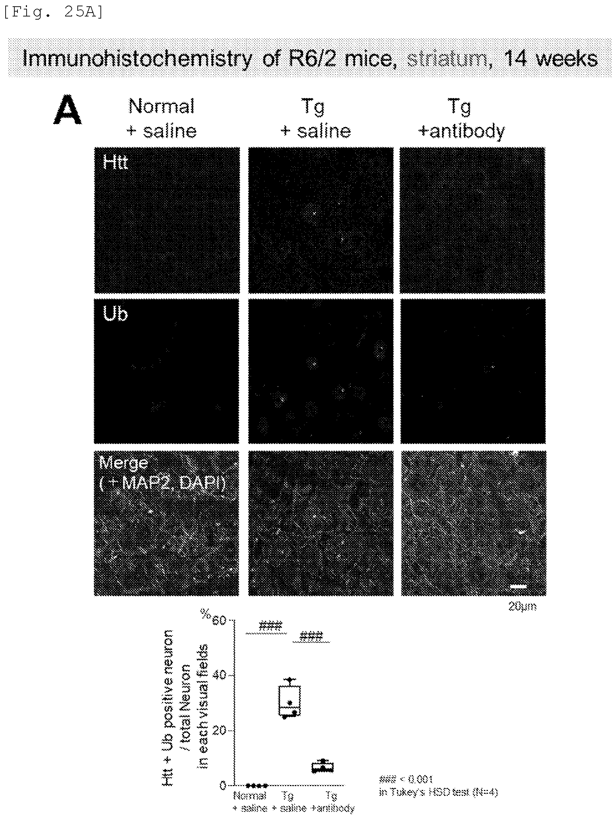

[Figure 25A] Figure 25A shows the results (n = 4) obtained by immunohistochemical staining that detects Htt, Ub and MAP2 in the corpus striatum in the three groups for analyzing Huntington's disease model mice and by measuring the percentage (%) of the cells having inclusions positive for both Htt and Ub in the MAP2-positive neurons.

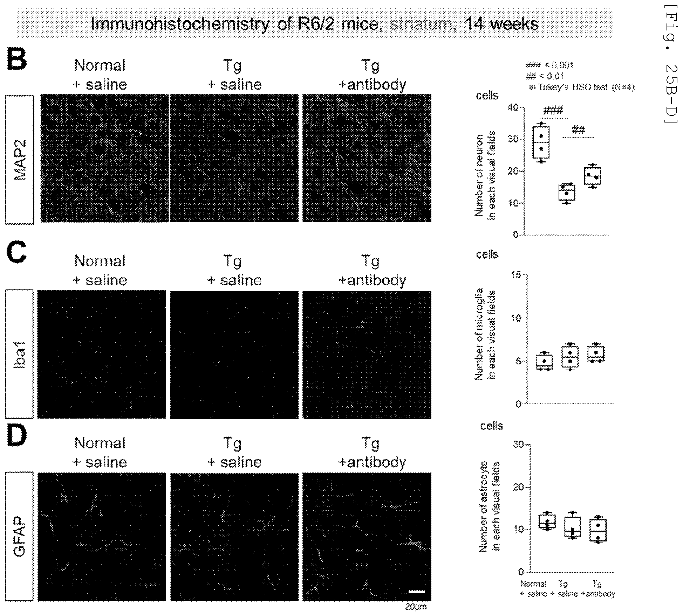

[Figures 25B-D] Figure 25B shows the results (n = 4) of comparison of the number of MAP2-positive cells (the number of neurons) in the corpus striatum between the three groups for analyzing Huntington's disease model mice. Figure 25C shows the results (n = 4) of comparison of the number of Iba1-positive cells (the number of microglia) in the corpus striatum between the three groups for analyzing Huntington's disease model mice. Figure 25D shows the results (n = 4) of comparison of the number of GFAP-positive cells (the number of astrocytes) in the corpus striatum between the three groups for analyzing Huntington's disease model mice. In the figures, "##" and "###" indicate a statistically significant difference in a Tukey's HSD test (p < 0.01 and p < 0.001, respectively).

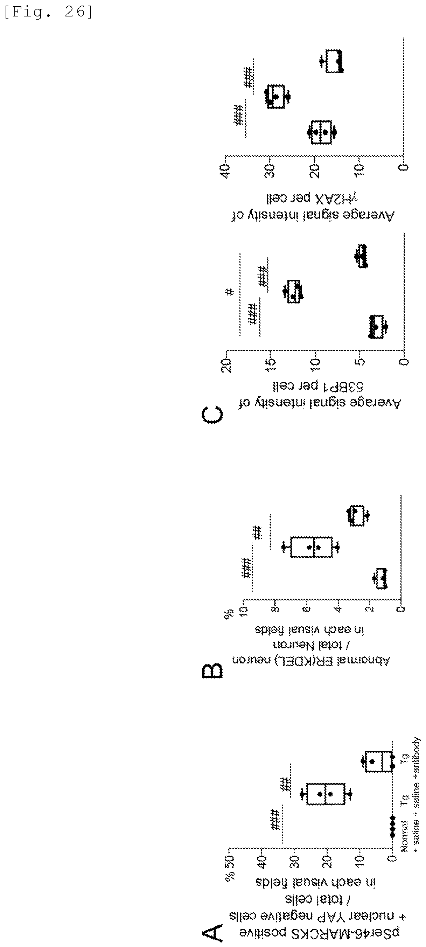

[Figure 26] Figure 26A shows the results (n = 4) obtained by immunohistochemical staining that detects YAP and pSer46-MARCKS in the cerebral cortex in the three groups for analyzing Huntington's disease model mice and by measuring the percentage (%) of the cells that indicates the characteristics of TRIAD cell death (nuclear YAP disappearance and pSer46-MARCKS positive). Figure 26B shows the results (n = 4) of comparison of the number of MAP2-positive neurons (the number of neurons) that indicates abnormal KDEL staining images in the cerebral cortex between the three groups for analyzing Huntington's disease model mice. Figure 26C shows the results of comparison of the signal intensity derived from 53BP1 (left graph) and the signal intensity derived from γH2AX (right graph) for 53BP1-positive, yH2AX-positive, and MAP2-positive neurons (neurons) in the cerebral cortex between the three groups for analyzing Huntington's disease model mice. In the figures, "#," "##," and "###" indicate a statistically significant difference in a Tukey's HSD test (p < 0.05, p < 0.01, and p < 0.001, respectively).

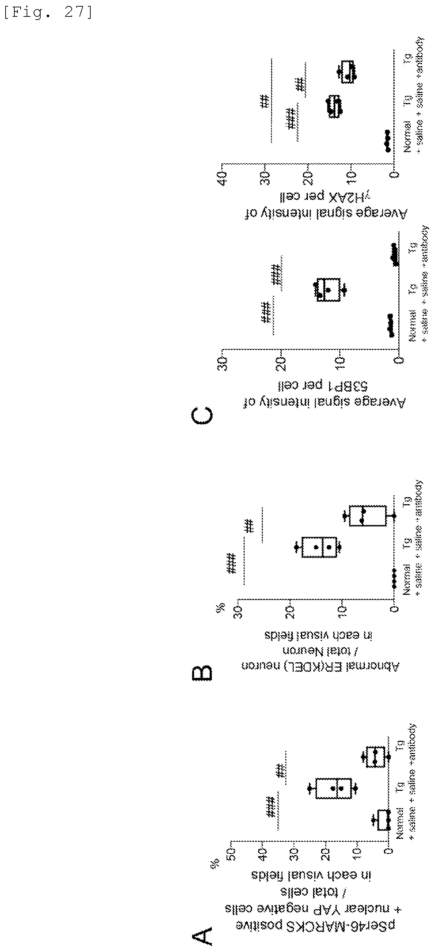

[Figure 27] Figure 27A shows the results (n = 4) obtained by immunohistochemical staining that detects YAP and pSer46-MARCKS in the corpus striatum in the three groups for analyzing Huntington's disease model mice and by measuring the percentage (%) of the cells that indicate the characteristics of TRIAD cell death (nuclear YAP disappearance and pSer46-MARCKS positive). Figure 27B shows the results (n = 4) of comparison of the number of MAP2-positive neurons (the number of neurons) that indicate abnormal KDEL staining images in the corpus striatum between the three groups for analyzing Huntington's disease model mice. Figure 27C shows the results of comparison of the signal intensity derived from 53BP1 (left graph) and the signal intensity derived from γH2AX (right graph) for 53BP1-positive, yH2AX-positive, and MAP2-positive neurons (neurons) in the corpus striatum between the three groups for analyzing Huntington's disease model mice. In the figures, "##" and "###" indicate a statistically significant difference in a Tukey's HSD test (p < 0.01 and p < 0.001, respectively).

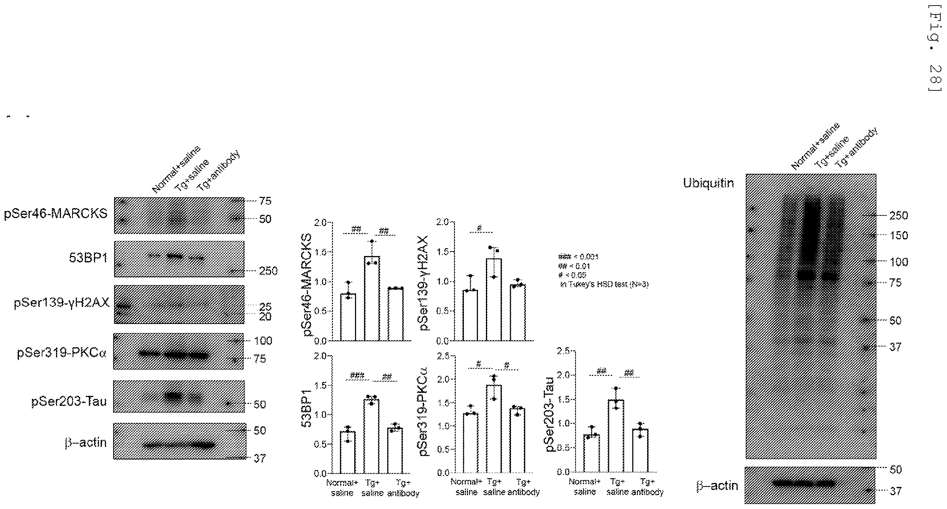

[Figure 28] Figure 28 shows the results of a Western blot that detects pSer46-MARCKS, 53BP1, pSer139-γH2AX, pSer319-PKCa, pSer203-Tau, Ub, and control β-actin in the three groups for analyzing Huntington's disease model mice, using the whole brain including basal ganglia as a sample. Quantification and statistical comparison were performed on bands of marker molecules except Ub, which forms a ladder or smear.

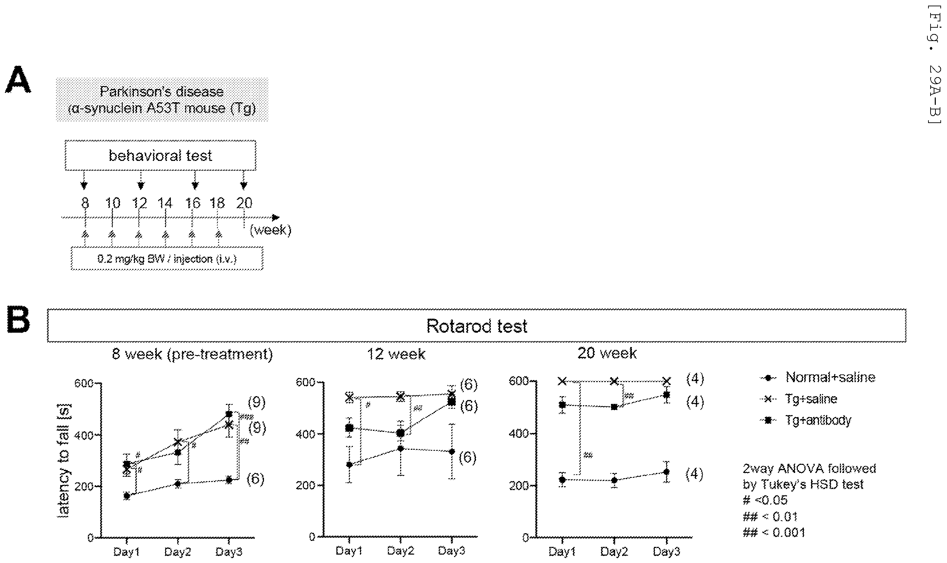

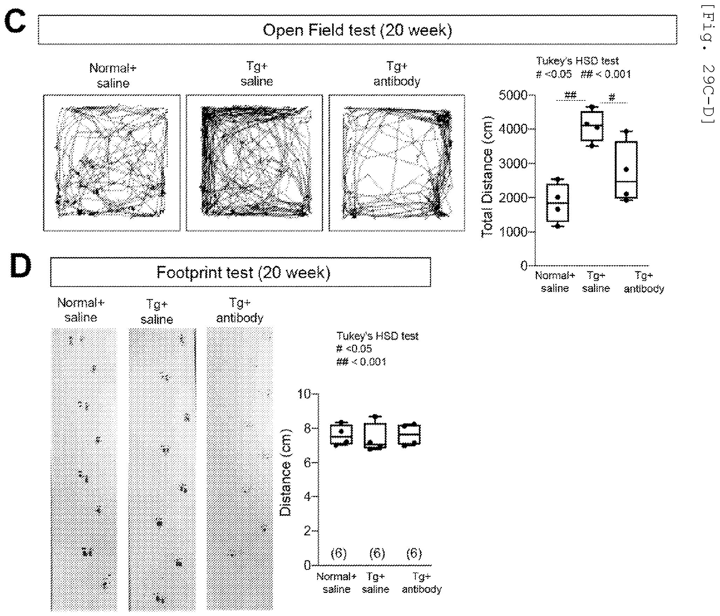

[Figures 29A-B] Figure 29A schematically shows the therapeutic experimental protocol for Parkinson's disease model mice (α-synuclein A53T mice). Figure 29B shows the results of examining treatment effects of the human monoclonal antibody #129 on α-synuclein A53T mice, using a rotarod test. Figure 29B shows the results (mean value ± standard error) of three groups of physiological saline-administered normal mice ("Normal+saline" in the figure; same in Figures 29 to 32), physiological saline-administered α-synuclein A53T mice ("Tg+saline" in the figure; same in Figures 29 to 32), and human monoclonal antibody #129-administered α-synuclein A53T mice ("Tg+antibody" in the figure; same in Figures 29 to 32) (hereinafter may be referred to as "three groups for analyzing Parkinson's disease model mice") for three consecutive days of testing in each week. Numerical values in the brackets indicate the number of animals used for the measurement.
[Figures 29C-D] Figure 29C shows the results of examining treatment effects of the human monoclonal antibody #129 on α-synuclein A53T mice, using an open field test. Figure 29D shows the results of examining treatment effects of the human monoclonal antibody #129 on α-synuclein A53T mice, using a footprint test. Figures 29C and 29D show the representative examples of the three groups for analyzing Parkinson's disease model mice and the measurement results, and the numerical values in the brackets indicate the number of animals used for the measurement. Of note, in Figure 29D, strides (distance) were measured in the three groups for analyzing Parkinson's disease model mice.

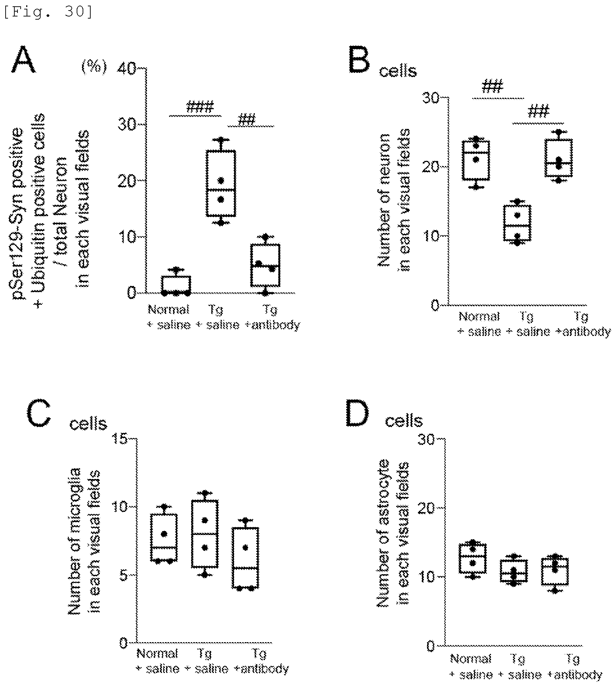

[Figure 30] Figure 30A shows the results (n = 4) obtained by immunohistochemical staining that detects pSer129-Syn (Ser129 phosphorylated synuclein), Ub and MAP2 in the cerebral cortex in the three groups for analyzing Parkinson's disease model mice and by measuring the percentage (%) of the cells having inclusions positive for both pSer129-Syn and Ub in the MAP2-positive neurons. Figure 30B shows the results (n = 4) of comparison of the number of MAP2-positive cells (the number of neurons) in the cerebral cortex between the three groups for analyzing Parkinson's disease model mice. Figure 30C shows the results (n = 4) of comparison of the number of Iba1-positive cells (the number of microglia) in the cerebral cortex between the three groups for analyzing Parkinson's disease model mice. Figure 30D shows the results (n = 4) of comparison of the number of GFAP-positive cells (the number of astrocytes) in the cerebral cortex between the three groups for analyzing Parkinson's disease model mice. In the figures, "##" and "###" indicate a statistically significant difference in a Tukey's HSD test (p < 0.01 and p < 0.001, respectively).

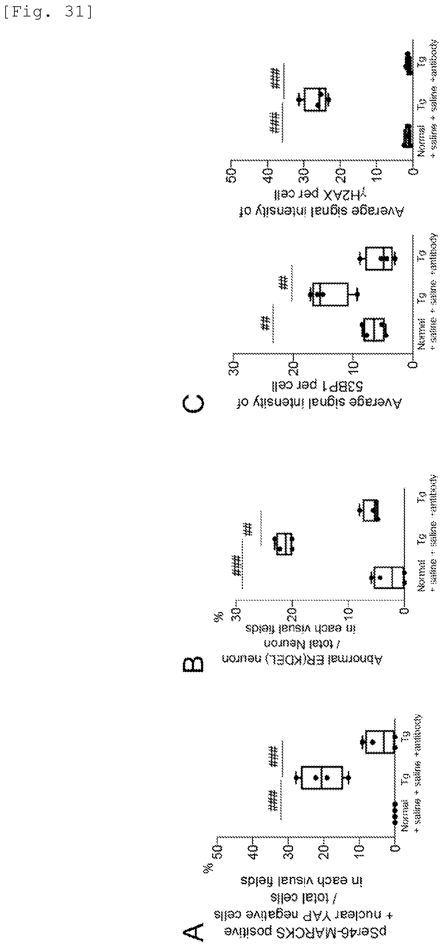

[Figure 31] Figure 31A shows the results (n = 4) obtained by immunohistochemical staining that detects YAP and pSer46-MARCKS in the cerebral cortex in the three groups for analyzing Parkinson's disease model mice and by measuring the percentage (%) of the cells that indicate the characteristics of TRIAD cell death (nuclear YAP disappearance and pSer46-MARCKS positive). Figure 31B shows the results (n = 4) of comparison of the number of MAP2-positive neurons (the number of neurons) that indicates abnormal KDEL staining images in the cerebral cortex between the three groups for analyzing Parkinson's disease model mice. Figure 31C shows the results of comparison of the signal intensity derived from 53BP1 (left graph) and the signal intensity derived from γH2AX (right graph) for 53BP1-positive, yH2AX-positive, and MAP2-positive neurons (neurons) in the cerebral cortex between the three groups for analyzing Parkinson's disease model mice. In the figures, "##" and "###" indicate a statistically significant difference in a Tukey's HSD test (p < 0.01 and p < 0.001, respectively).

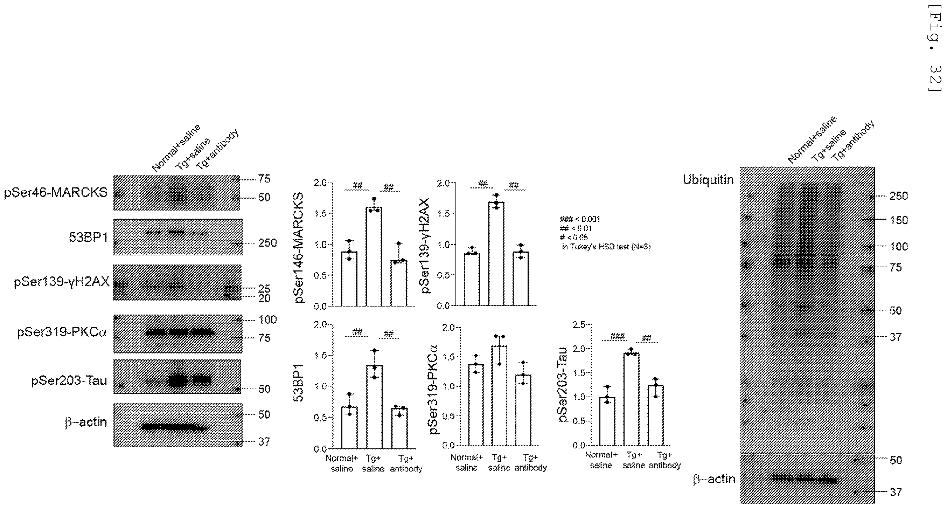

[Figure 32] Figure 32 shows the results of a Western blot that detects pSer46-MARCKS, 53BP1, pSer139-γH2AX, pSer319-PKCa, pSer203-Tau, Ub, and control β-actin in the three groups for analyzing Parkinson's disease model mice, using the whole brain including basal ganglia as a sample. Quantification and statistical comparison were performed on bands of marker molecules except Ub, which forms a ladder or smear.

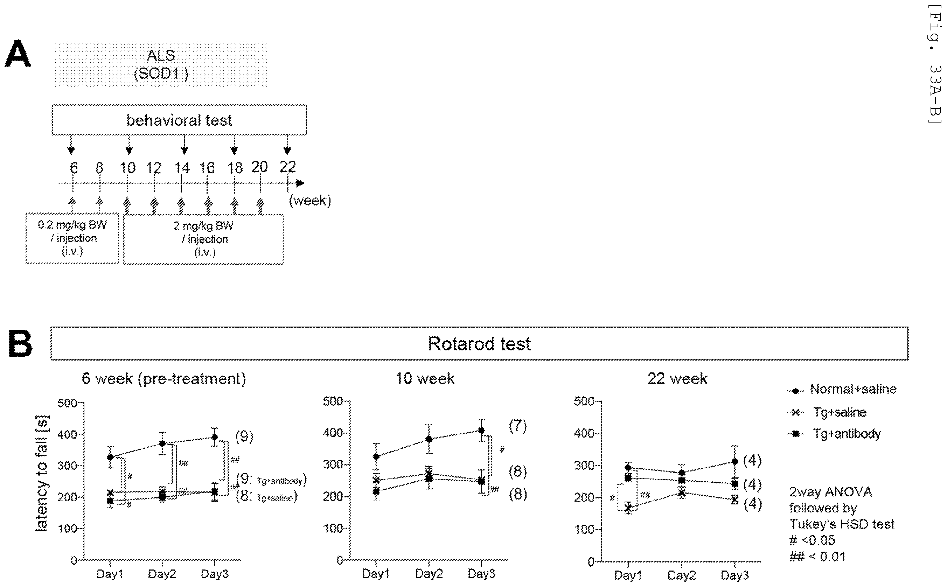

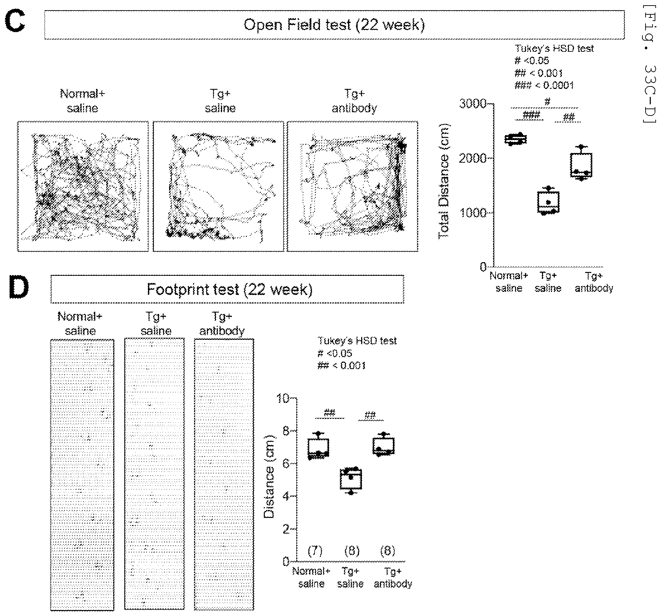

[Figures 33A-B] Figure 33A schematically shows the therapeutic experimental protocol for amyotrophic lateral sclerosis model mice (SOD1-Tg mice). Figure 33B shows the results of examining treatment effects of the human monoclonal antibody #129-L2 on SOD1-Tg mice, using a rotarod test. Figure 33B shows the results (mean value ± standard error) of three groups of physiological saline-administered normal mice ("Normal+saline" in the figure; same in Figures 33 to 37), physiological saline-administered SOD1-Tg mice ("Tg+saline" in the figure; same in Figures 33 to 37), and human monoclonal antibody #129-L2-administered SOD1-Tg mice ("Tg+antibody" in the figure; same in Figures 33 to 37) (hereinafter may be referred to as "three groups for analyzing amyotrophic lateral sclerosis model mice") for three consecutive days of testing in each week. Numerical values in the brackets indicate the number of animals used for the measurement.
[Figures 33C-D] Figure 33C shows the results of examining treatment effects of the human monoclonal antibody #129-L2 on SOD1-Tg mice, using an open field test. Figure 33D shows the results of examining treatment effects of the human monoclonal antibody #129-L2 on SOD1-Tg mice, using a footprint test. Figures 33C and 33D show the representative examples of the three groups for analyzing amyotrophic lateral sclerosis model mice and the measurement results, and the numerical values in the brackets indicate the number of animals used for the measurement. Of note, in Figure 33D, strides (distance) were measured in the three groups for analyzing amyotrophic lateral sclerosis model mice.

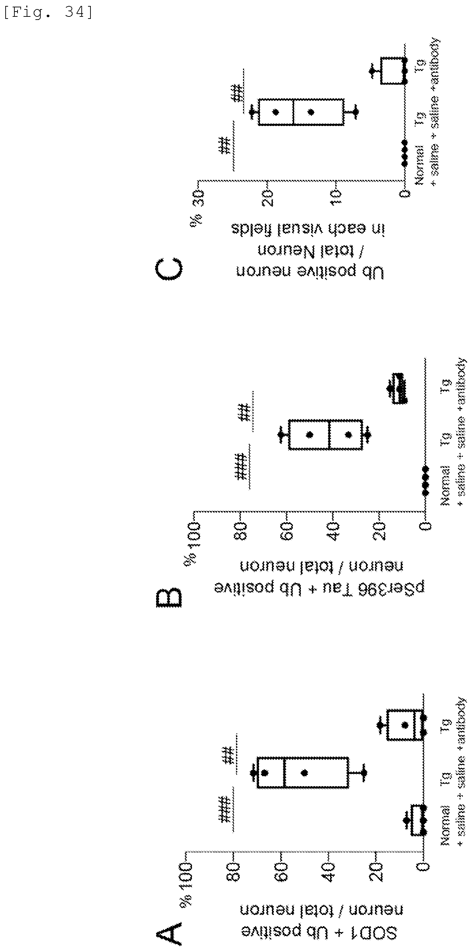

[Figure 34] Figure 34A shows the results (n = 4) obtained by immunohistochemical staining that detects SOD1, Ub and MAP2 in the cerebral cortex in the three groups for analyzing amyotrophic lateral sclerosis model mice and by measuring the percentage (%) of the cells showing positive for both SOD1 and Ub in the MAP2-positive neurons. Figure 34B shows the results (n = 4) obtained by immunohistochemical staining that detects pSer386-Tau, Ub and MAP2 in the cerebral cortex in the three groups for analyzing amyotrophic lateral sclerosis model mice and by measuring the percentage (%) of the cells showing positive for both pSer386-Tau and Ub in the MAP2-positive neurons. Figure 34C shows the results (n = 4) obtained by immunohistochemical staining that detects TDP43, Ub and MAP2 in the cerebral cortex in the three groups for analyzing amyotrophic lateral sclerosis model mice and by measuring the percentage (%) of the cells showing positive for both TDP43 and Ub in the MAP2-positive neurons. In the figures, "##" and "###" indicate a statistically significant difference in a Tukey's HSD test (p < 0.01 and p < 0.001, respectively).

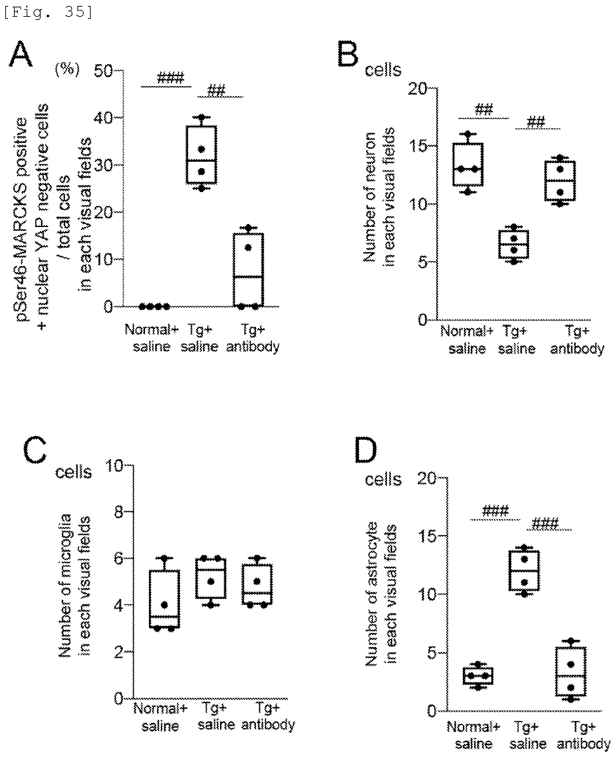

[Figure 35] Figure 35A shows the results (n = 4) obtained by immunohistochemical staining that detects YAP and pSer46-MARCKS in the lumbar cord in the three groups for analyzing amyotrophic lateral sclerosis model mice and by measuring the percentage (%) of the cells that indicate the characteristics of TRIAD cell death (nuclear YAP disappearance and pSer46-MARCKS positive). Figure 35B shows the results (n = 4) of comparison of the number of MAP2-positive cells (the number of neurons) in the lumbar anterior horn between the three groups for analyzing amyotrophic lateral sclerosis model mice. Figure 35C shows the results (n = 4) of comparison of the number of Iba1-positive cells (the number of microglia) in the lumbar anterior horn between the three groups for analyzing amyotrophic lateral sclerosis model mice. Figure 35D shows the results (n = 4) of comparison of the number of GFAP-positive cells (the number of astrocytes) in the lumbar anterior horn between the three groups for analyzing amyotrophic lateral sclerosis model mice. In the figures, "##" and "###" indicate a statistically significant difference in a Tukey's HSD test (p < 0.01 and p < 0.001, respectively).

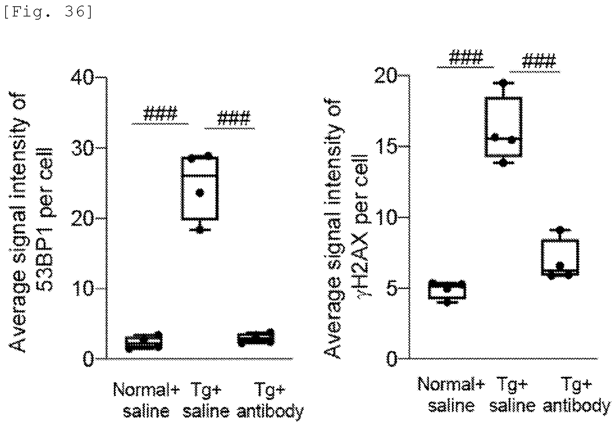

[Figure 36] Figure 36 shows the results of comparison of the signal intensity derived from 53BP1 (left graph) and the signal intensity derived from γH2AX (right graph) for 53BP1-positive, yH2AX-positive, and MAP2-positive neurons (neurons) in the cerebral cortex between the three groups for analyzing amyotrophic lateral sclerosis model mice. In the figures, "###" indicates a statistically significant difference in a Tukey's HSD test (p < 0.001).

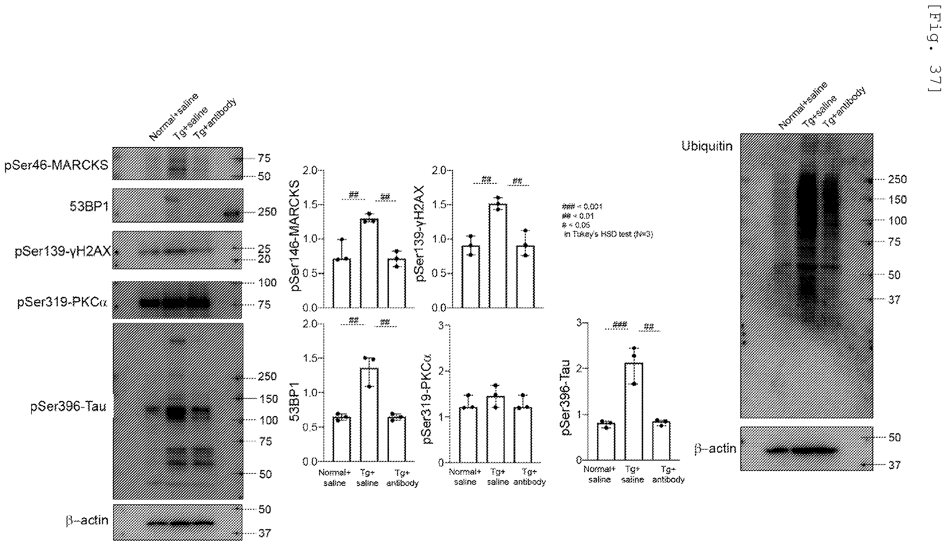

[Figure 37] Figure 37 shows the results of a Western blot that detects pSer46-MARCKS, 53BP1, pSer139-γH2AX, pSer319-PKCa, pSer396-Tau, Ub, and control β-actin in the three groups for analyzing amyotrophic lateral sclerosis model mice, using the whole brain including basal ganglia as a sample. Quantification and statistical comparison were performed on bands of marker molecules except Ub, which forms a ladder or smear.

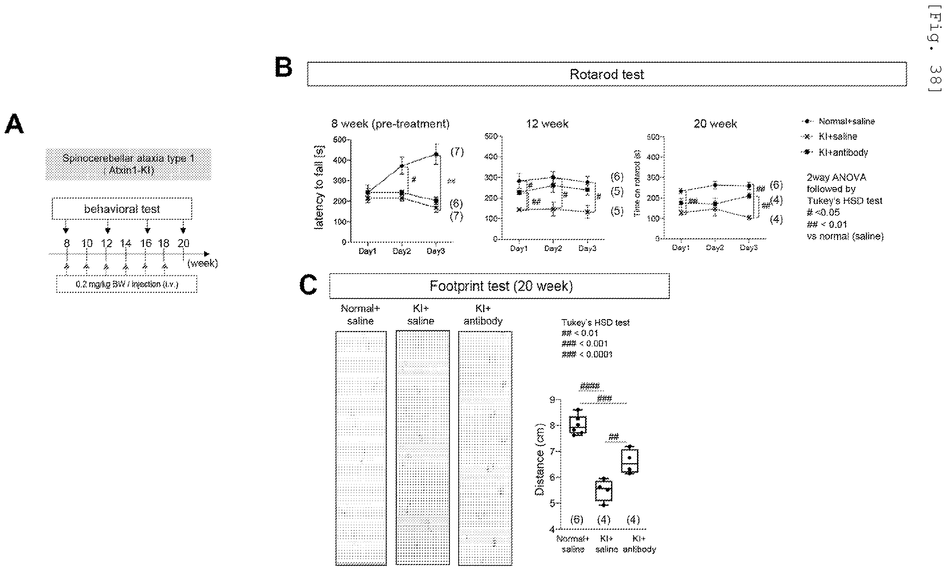

[Figure 38] Figure 38A schematically shows the therapeutic experimental protocol for spinocerebellar ataxia model mice (mutant ataxin-1 knock-in mice). Figure 38B shows the results of examining treatment effects of the human monoclonal antibody #129 on mutant ataxin-1 knock-in mice, using a rotarod test. Figure 38B shows the results (mean value ± standard error) of three groups of physiological saline-administered normal mice ("Normal+saline" in the figure; same in Figures 38 to 41), physiological saline-administered mutant ataxin-1 knock-in mice ("KI+saline" in the figure; same in Figures 38 to 41), and human monoclonal antibody #129-administered mutant ataxin-1 knock-in mice ("KI+antibody" in the figure; same in Figures 38 to 41) (hereinafter may be referred to as "three groups for analyzing spinocerebellar ataxia model mice") for three consecutive days of testing in each week. Numerical values in the brackets indicate the number of animals used for the measurement. Figure 38C shows the results of examining treatment effects of the human monoclonal antibody #129 on mutant ataxin-1 knock-in mice, using a footprint test. Figure 38C shows the representative examples of the three groups for analyzing spinocerebellar ataxia model mice and the measurement results, and the numerical values in the brackets indicate the number of animals used for the measurement. Of note, in Figure 38C, strides (distance) were measured in the three groups for analyzing spinocerebellar ataxia model mice.

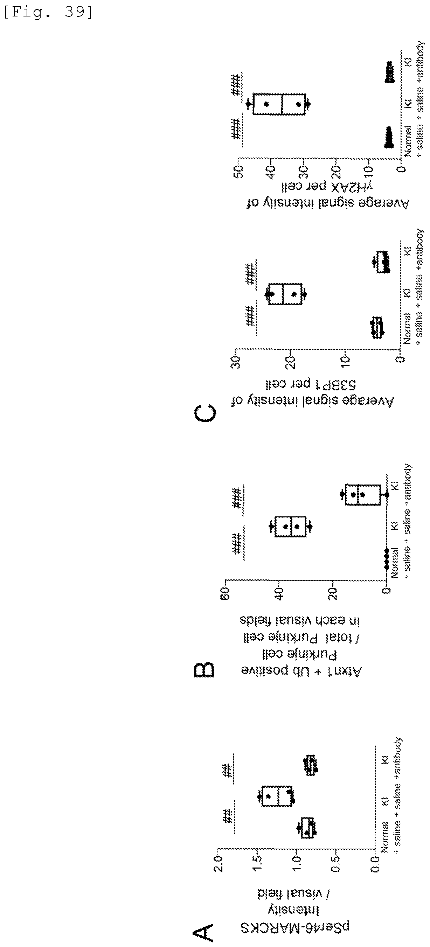

[Figure 39] Figure 39A shows the results (n = 4) obtained by immunohistochemistry with YAP, pSer46-MARCKS, and DAPI in the cerebellar cortex in the three groups for analyzing spinocerebellar ataxia model mice and by quantifying and comparing the signals of pSer46-MARCKS staining in the granule cell layer of the cerebellar cortex. Figure 39B shows the results (n = 4) obtained by immunohistochemical staining that detects Ataxin-1 (Atxn1), Ub and MAP2 in the cerebral cortex in the three groups for analyzing spinocerebellar ataxia model mice and by measuring the percentage (%) of the cells showing positive for both Atxn1 and Ub in the MAP2-positive neurons. Figure 39C shows the results of comparison of the signal intensity derived from 53BP1 (left graph) and the signal intensity derived from γH2AX (right graph) in Purkinje cells between the three groups for analyzing spinocerebellar ataxia model mice. In the figures, "##" and "###" indicate a statistically significant difference in a Tukey's HSD test (p < 0.01 and p < 0.001, respectively).

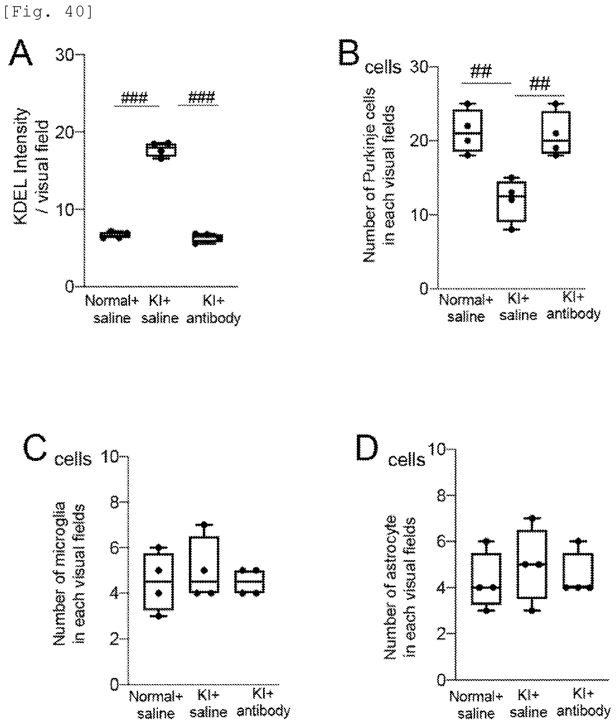

[Figure 40] Figure 40A shows the results (n = 4) of comparison of the number of Calbindin-positive Purkinje cells that indicate abnormal KDEL staining images between the three groups for analyzing spinocerebellar ataxia model mice. Figure 40B shows the results (n = 4) of comparison of the number of Purkinje cells (the number of neurons) in the cerebellar cortex between the three groups for analyzing spinocerebellar ataxia model mice. Figure 40C shows the results (n = 4) of comparison of the number of Iba1-positive cells (the number of microglia) in the cerebellar cortex between the three groups for analyzing spinocerebellar ataxia model mice. Figure 40D shows the results (n = 4) of comparison of the number of GFAP-positive cells (the number of astrocytes) in the cerebellar cortex between the three groups for analyzing spinocerebellar ataxia model mice.

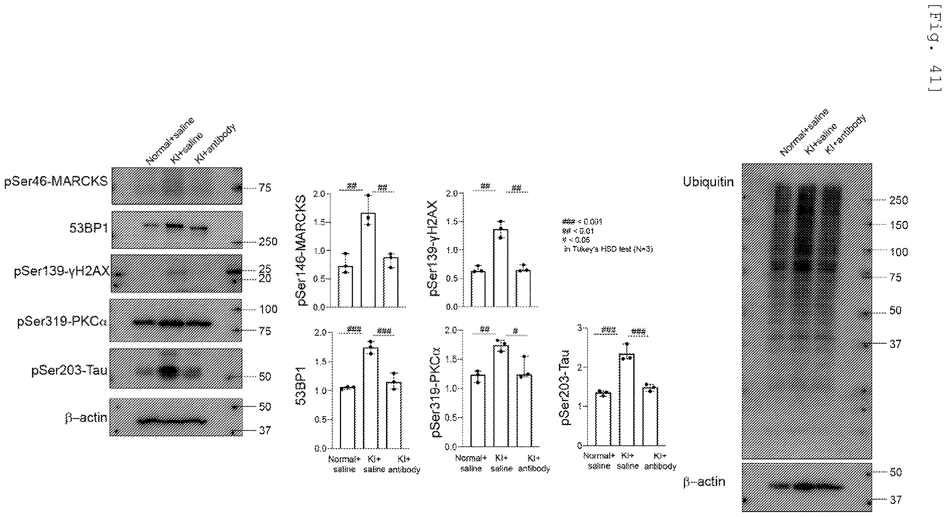

[Figure 41] Figure 41 shows the results of a Western blot that detects pSer46-MARCKS, 53BP1, pSer139-γH2AX, pSer319-PKCa, pSer203-Tau, Ub, and control β-actin in the three groups for analyzing spinocerebellar ataxia model mice, using the whole brain including basal ganglia as a sample. Quantification and statistical comparison were performed on bands of marker molecules except Ub, which forms a ladder or smear.

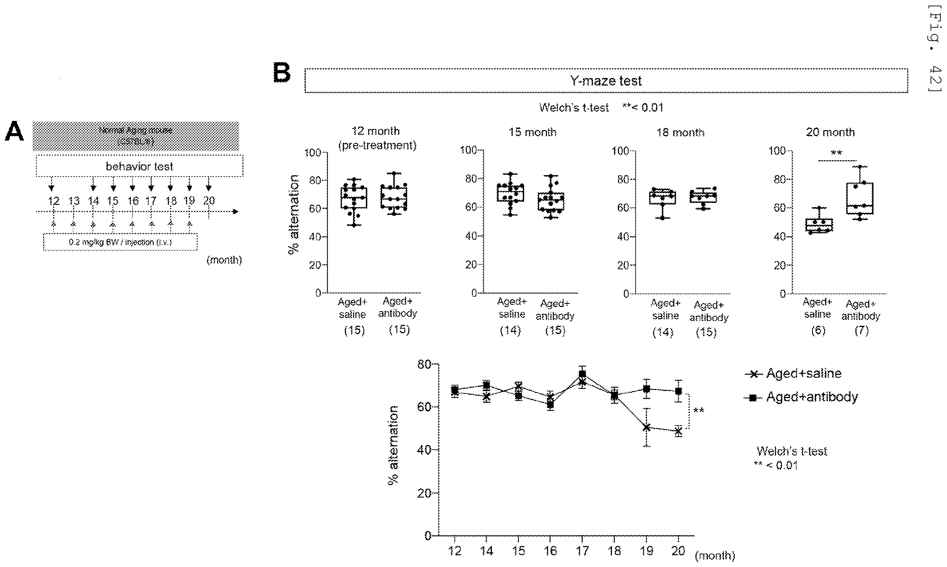

[Figure 42] Figure 42A schematically shows the therapeutic experimental protocol for aging model mice (C57BL/6 mice at 12 months of age). Figure 42B shows the results of examining treatment effects of the human monoclonal antibody #129-L2 on decreased cognitive function of aging model mice, using a Y-maze test. The upper part shows the results of the Y-maze test at each month of age of two groups of physiological saline-administered C57BL/6 mice at 12 months of age ("Aged+saline" in the figure), and human monoclonal antibody #129-L2-administered C57BL/6 mice at 12 months of age ("Aged+antibody" in the figure). The lower part is a summary of the results obtained in the upper part. Numerical values in the brackets indicate the number of animals used for the measurement.

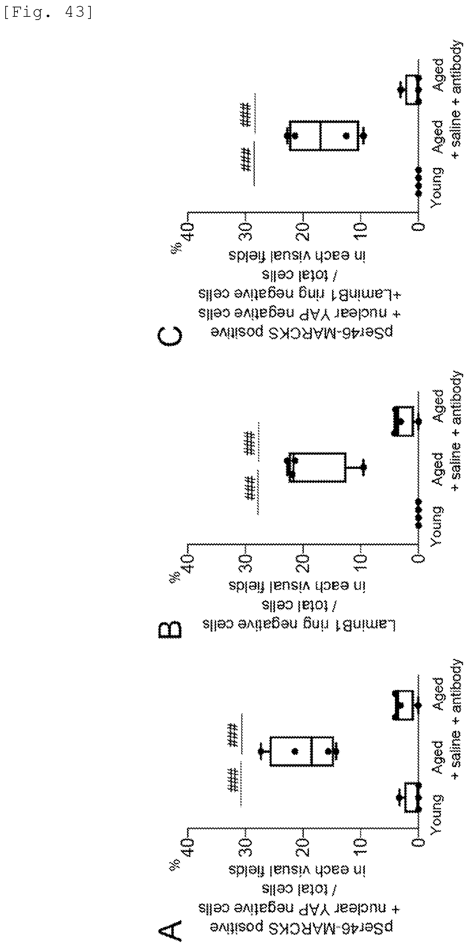

[Figure 43] Figure 43 shows the results (n = 4) obtained by immunohistochemical staining that detects YAP, pSer46-MARCKS and Lamin B1 in the cerebral cortex in three groups of young mice (C57BL/6 mice at 3 months of age) ("Young" in the figure; same in Figures 43 to 45), physiological saline-administered C57BL/6 mice at 20 months of age ("Aged+saline" in the figure; same in Figures 43 to 45), and human monoclonal antibody #129-L2-administered C57BL/6 mice at 20 months of age ("Aged+antibody" in the figure; same in Figures 43 to 45) (hereinafter may be referred to as "three groups for analyzing aging model mice at 20 months of age"). Figure 43A shows the results (n = 4) of measuring the percentage (%) of the TRIAD necrosis cells being positive for cytoplasmic pSer46-MARCK and determined to be the nuclear YAP disappearance. Figure 43B shows the results (n = 4) of measuring the percentage (%) of the senescent cells in which a nuclear ring of Lamin B1 has disappeared. Figure 43C shows the results (n = 4) of measuring the percentage (%) of the cells having the characteristics of both TRIAD necrosis and aging.

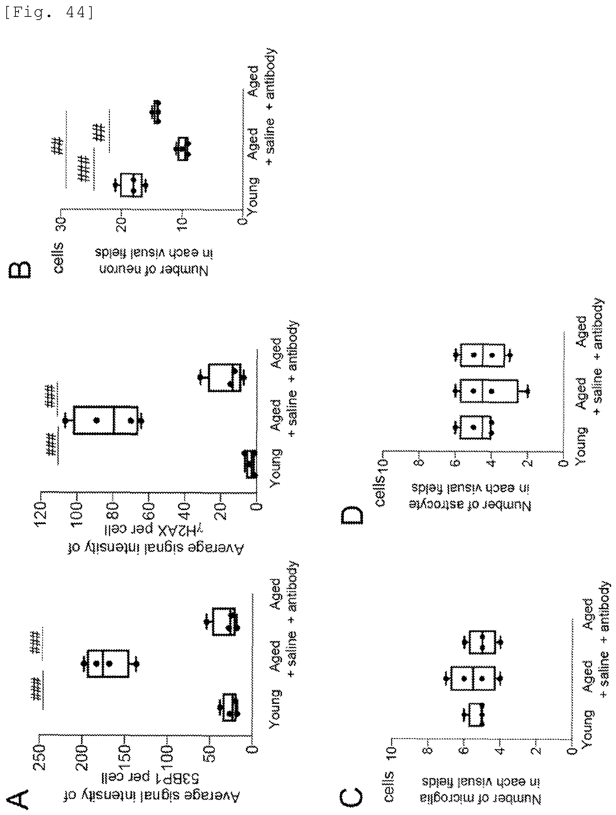

[Figure 44] Figure 44A shows the results (n = 4) of comparison of the signal intensity derived from 53BP1 (left graph) and the signal intensity derived from γH2AX (right graph) for 53BP1-positive, yH2AX-positive, and MAP2-positive neurons (neurons) in the cerebral cortex between the three groups for analyzing aging model mice at 20 months of age. Figure 44B shows the results (n = 4) of comparison of the number of MAP2-positive cells (the number of neurons) in the cerebral cortex between the three groups for analyzing aging model mice at 20 months of age. Figure 44C shows the results (n = 4) of comparison of the number of Iba1-positive cells (the number of microglia) in the cerebral cortex between the three groups for analyzing aging model mice at 20 months of age. Figure 44D shows the results (n = 4) of comparison of the number of GFAP-positive cells (the number of astrocytes) in the cerebral cortex between the three groups for analyzing aging model mice at 20 months of age. In the figures, "##" and "###" indicate a statistically significant difference in a Tukey's HSD test (p < 0.01 and p < 0.001, respectively).

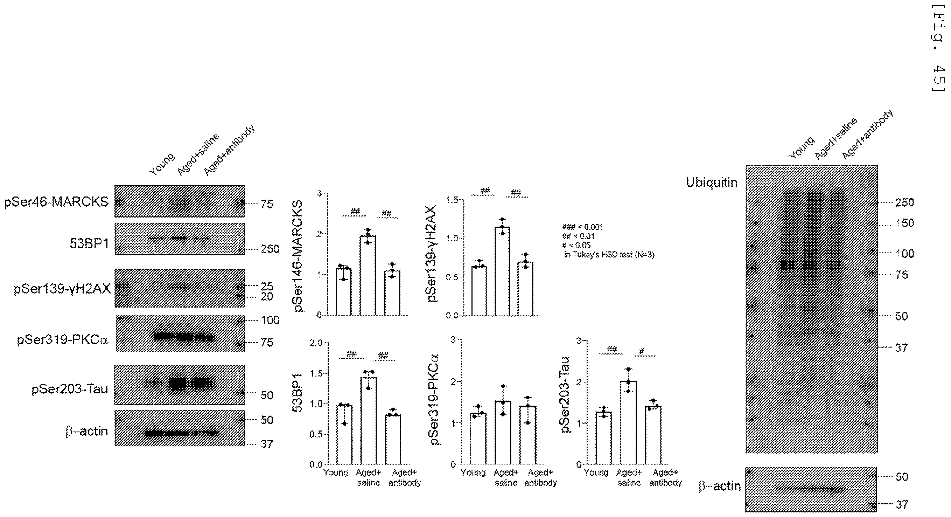

[Figure 45] Figure 45 shows the results of a Western blot that detects pSer46-MARCKS, 53BP1, pSer139-γH2AX, pSer319-PKCa, pSer396-Tau, Ub, and control β-actin in the three groups for analyzing aging model mice at 20 months of age, using the whole brain including basal ganglia as a sample. Quantification and statistical comparison were performed on bands of marker molecules except Ub, which forms a ladder or smear.

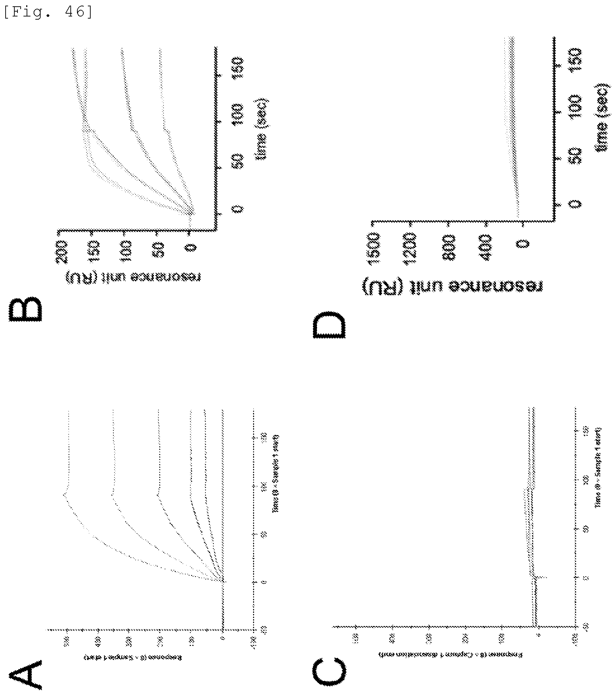

[Figure 46] Figures 46A and B show the results of SPR analysis of interactions between two types of anti-human HMGB1 antibodies of the present invention (the human monoclonal antibody #129-L2 [Figure 46A] and the human monoclonal antibody #129 [Figure 46B]) and the human dsHMGB1. Figures 46C and D show the results of SPR analysis of interactions between two types of anti-human HMGB1 antibodies of the present invention (the human monoclonal antibody #129-L2 [Figure 46C] and the human monoclonal antibody #129 [Figure 46D]) and the human reduced HMGB1.

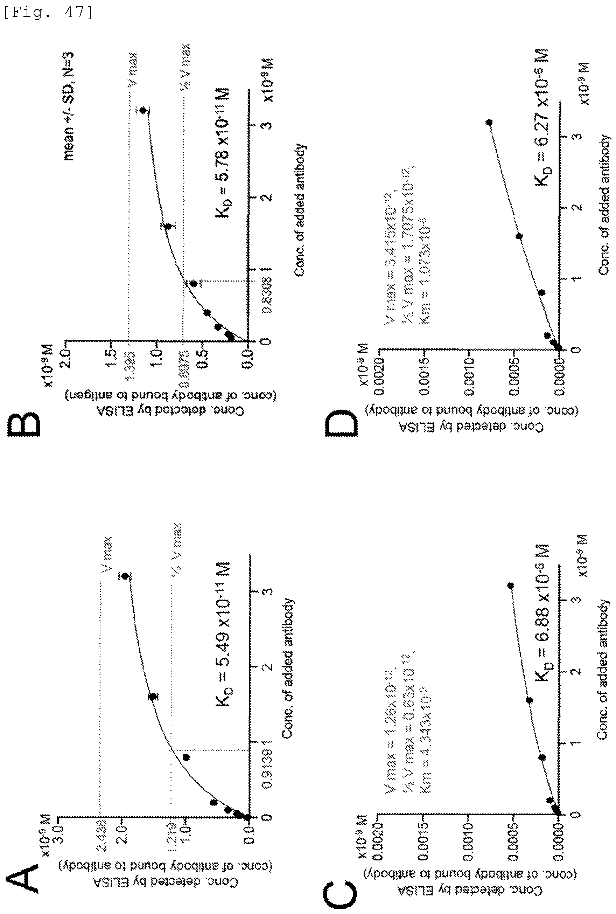

[Figure 47] Figures 47A and B show the results of ELISA analysis of interactions between two types of anti-human HMGB1 antibodies of the present invention (the human monoclonal antibody #129-L2 [Figure 47A] and the human monoclonal antibody #129 [Figure 47B]) and the human dsHMGB1. Figures 47C and D show the results of ELISA analysis of interactions between two types of anti-human HMGB1 antibodies of the present invention (the human monoclonal antibody #129-L2 [Figure 47C] and the human monoclonal antibody #129 [Figure 47D]) and the human reduced HMGB1.

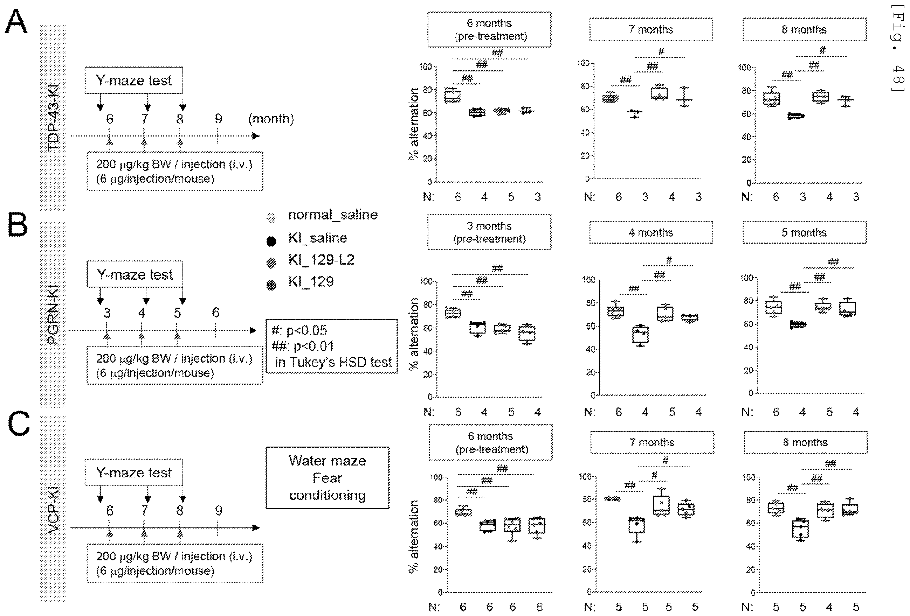

[Figure 48] Figure 48 shows the results of a Y-maze test performed at six months of age, seven months of age, and eight months of age of three types of FTLD-ALS model mice (TDP43^{N267S}-KI mice [Figure 48A], PGRN^{R504X}-KI mice [Figure 48B], and VCP^{T262A}-KI mice [Figure 48C]) to which the above-described two types of anti-human HMGB1 antibodies of the present invention were administered. In order from the left of each graph, the results of normal mice (normal_saline), respective physiological saline-administered FTLD-ALS model mice (KI_saline), respective human monoclonal antibody #129-L2-administered FTLD-ALS model mice (KI_129-L2), and respective human monoclonal antibody #129-administered FTLD-ALS model mice (KI_129) are shown.

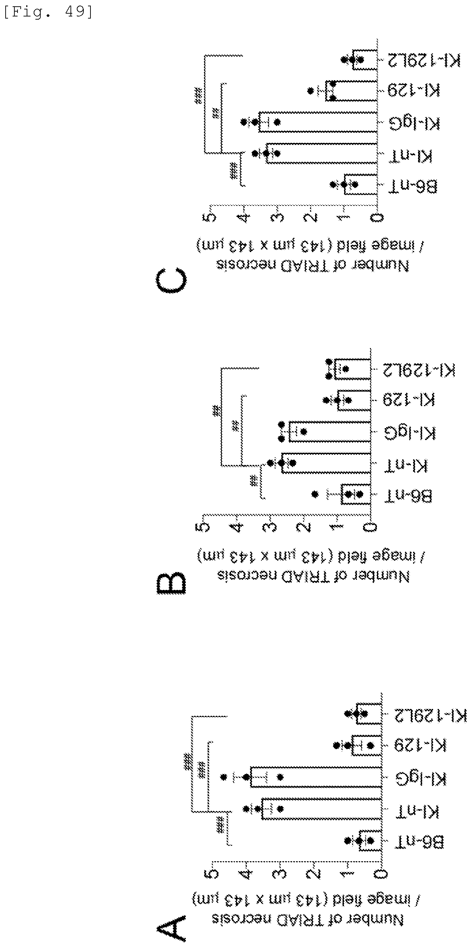

[Figure 49] Figure 49 shows the results (n = 3) obtained by immunohistochemical staining that detects YAP and pSer46-MARCKS in the cerebral cortex in the three types of FTLD-ALS model mice (PGRN^{R504X}-KI mice [Figure 49A], TDP43^{N267S}-KI mice [Figure 49B], and VCP^{T262A}-KI mice [Figure 49C]) to which the above-described two types of anti-human HMGB1 antibodies of the present invention were administered and by measuring the percentage (%) of the cells that indicate the characteristics of TRIAD cell death (nuclear YAP disappearance and pSer46-MARCKS positive). In order from the left of each graph, the results of normal mice ("B6-nT" in the figure), respective physiological saline-administered FTLD-ALS model mice ("KI-nT" in the figure), respective control human IgG-administered FTLD-ALS model mice ("KI-IgG" in the figure), respective human monoclonal antibody #129-administered FTLD-ALS model mice ("KI-129" in the figure), and respective human monoclonal antibody #129-L2-administered FTLD-ALS model mice ("KI-129L2" in the figure) are shown. In the figures, "##" and "###" indicate a statistically significant difference in a Tukey's HSD test (p < 0.01 and p < 0.001, respectively).

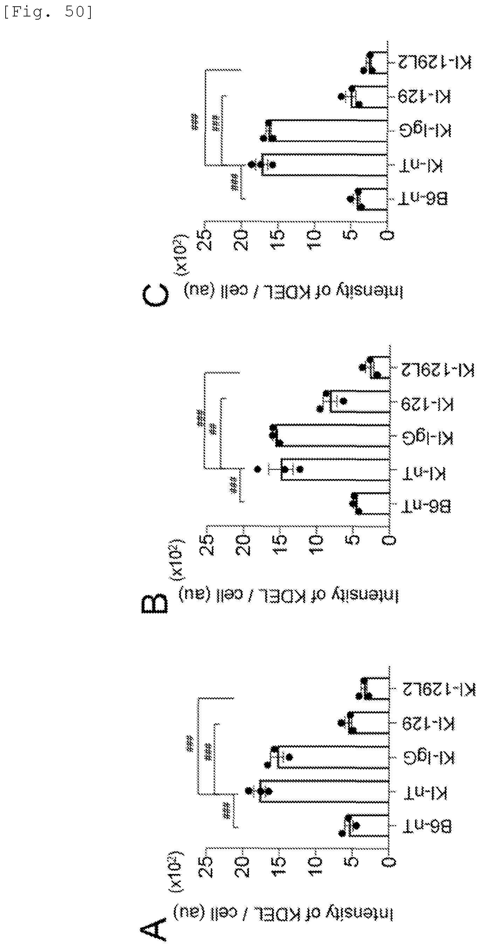

[Figure 50] Figure 50 shows the results (n = 3) of comparison of the number of MAP2-positive neurons (the number of neurons) that indicate abnormal KDEL staining images in the cerebral cortex in the three types of FTLD-ALS model mice (PGRN^{R504X}-KI mice [Figure 50A], TDP43^{N267S}-KI mice [Figure 50B], and VCP^{T262A}-KI mice [Figure 50C]) to which the above-described two types of anti-human HMGB1 antibodies of the present invention were administered. In order from the left of each graph, the results of normal mice ("B6-nT" in the figure), respective physiological saline-administered FTLD-ALS model mice ("KI-nT" in the figure), respective control human IgG-administered FTLD-ALS model mice ("KI-IgG" in the figure), respective human monoclonal antibody #129-administered FTLD-ALS model mice ("KI-129" in the figure), and respective human monoclonal antibody #129-L2-administered FTLD-ALS model mice ("KI-129L2" in the figure) are shown. In the figures, "###" indicates a statistically significant difference in a Tukey's HSD test (p < 0.001).

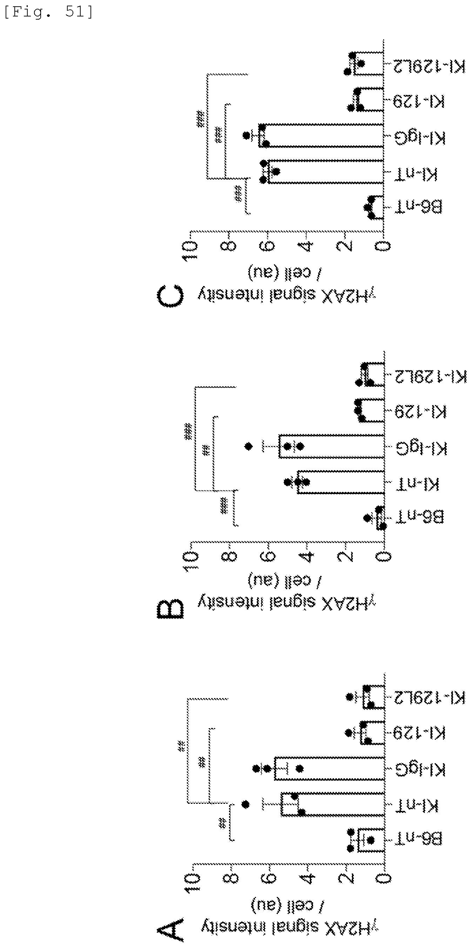

[Figure 51] Figure 51 shows the results (n = 3) of comparison of the signal intensity derived from γH2AX for the number of γH2AX-positive and MAP2-positive neurons (the number of neurons) in the cerebral cortex in the three types of FTLD-ALS model mice (PGRN^{R504X}-KI mice [Figure 51A], TDP43^{N267S}-KI mice [Figure 51B], and VCP^{T262A}-KI mice [Figure 51C]) to which the above-described two types of anti-human HMGB1 antibodies of the present invention were administered. In order from the left of each graph, the results of normal mice ("B6-nT" in the figure), respective physiological saline-administered FTLD-ALS model mice ("KI-nT" in the figure), respective control human IgG-administered FTLD-ALS model mice ("KI-IgG" in the figure), respective human monoclonal antibody #129-administered FTLD-ALS model mice ("KI-129" in the figure), and respective human monoclonal antibody #129-L2-administered FTLD-ALS model mice ("KI-129L2" in the figure) are shown. In the figures, "##" and "###" indicate a statistically significant difference in a Tukey's HSD test (p < 0.01 and p < 0.001, respectively).

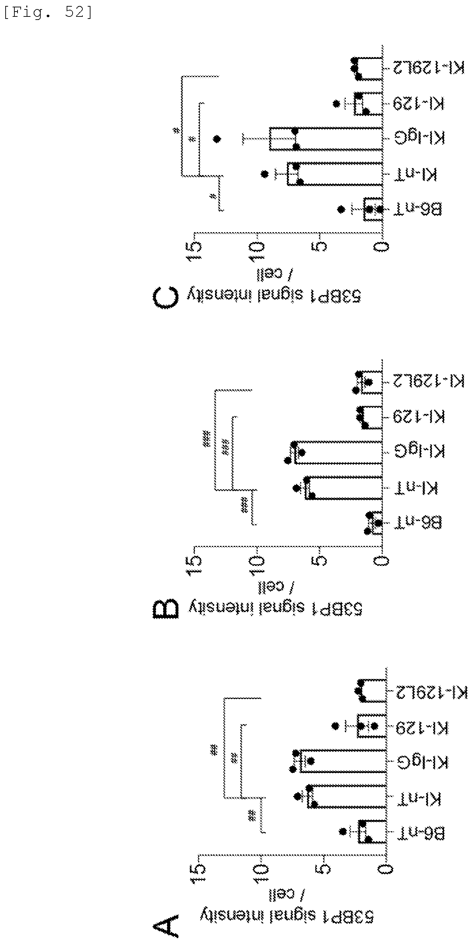

[Figure 52] Figure 52 shows the results (n = 3) of comparison of the signal intensity derived from 53BP1 for the number of 53BP1-positive and MAP2-positive neurons (the number of neurons) in the cerebral cortex in the three types of FTLD-ALS model mice (PGRN^{R504X}-KI mice [Figure 52A], TDP43^{N267S}-KI mice [Figure 52B], and VCP^{T262A}-KI mice [Figure 52C]) to which the above-described two types of anti-human HMGB1 antibodies of the present invention were administered. In order from the left of each graph, the results of normal mice ("B6-nT" in the figure), respective physiological saline-administered FTLD-ALS model mice ("KI-nT" in the figure), respective control human IgG-administered FTLD-ALS model mice ("KI-IgG" in the figure), respective human monoclonal antibody #129-administered FTLD-ALS model mice ("KI-129" in the figure), and respective human monoclonal antibody #129-L2-administered FTLD-ALS model mice ("KI-129L2" in the figure) are shown. In the figures, "##" and "###" indicate a statistically significant difference in a Tukey's HSD test (p < 0.01 and p < 0.001, respectively).

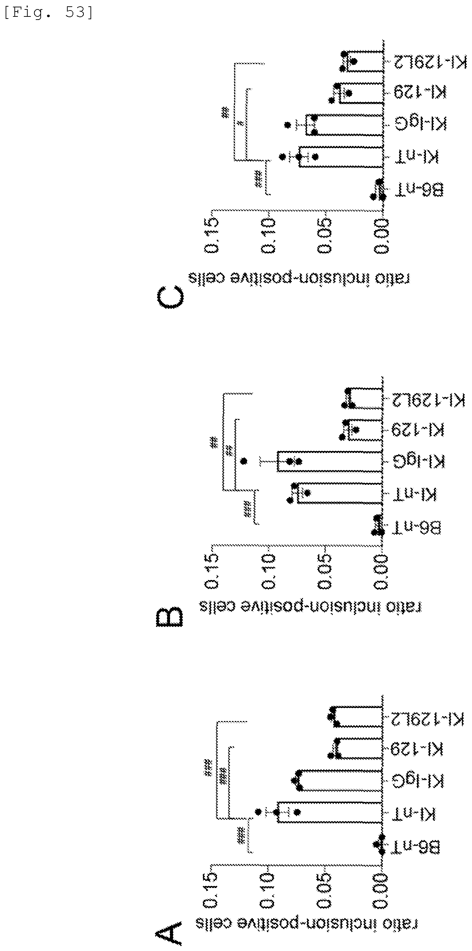

[Figure 53] Figure 53 shows the results (n = 3) obtained by immunohistochemical staining that detects pSer409/410-TDP43 and Ub in the cerebral cortex in the three types of FTLD-ALS model mice (PGRN^{R504X}-KI mice [Figure 53A], TDP43^{N267S}-KI mice [Figure 53B], and VCP^{T262A}-KI mice [Figure 53C]) to which the above-described two types of anti-human HMGB1 antibodies of the present invention were administered and by measuring the percentage (%) of the cells having inclusions positive for both pSer409/410-TDP43 and Ub. In order from the left of each graph, the results of normal mice ("B6-nT" in the figure), respective physiological saline-administered FTLD-ALS model mice ("KI-nT" in the figure), respective control human IgG-administered FTLD-ALS model mice ("KI-IgG" in the figure), respective human monoclonal antibody #129-administered FTLD-ALS model mice ("KI-129" in the figure), and respective human monoclonal antibody #129-L2-administered FTLD-ALS model mice ("KI-129L2" in the figure) are shown. In the figures, "#," "##," and "###" indicate a statistically significant difference in a Tukey's HSD test (p < 0.05, p < 0.01, and p < 0.001, respectively).

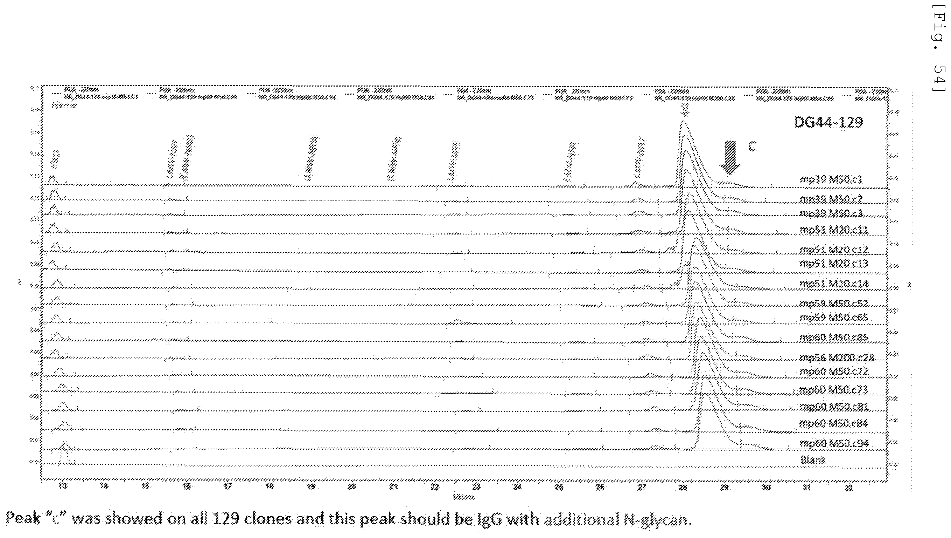

[Figure 54] Figure 54 shows the results obtained by purifying human monoclonal antibody #129 from CHO (Chinese Hamster Ovary) cell line that stably expresses human monoclonal antibody #129 and by a non-reducing capillary electrophoresis analysis.

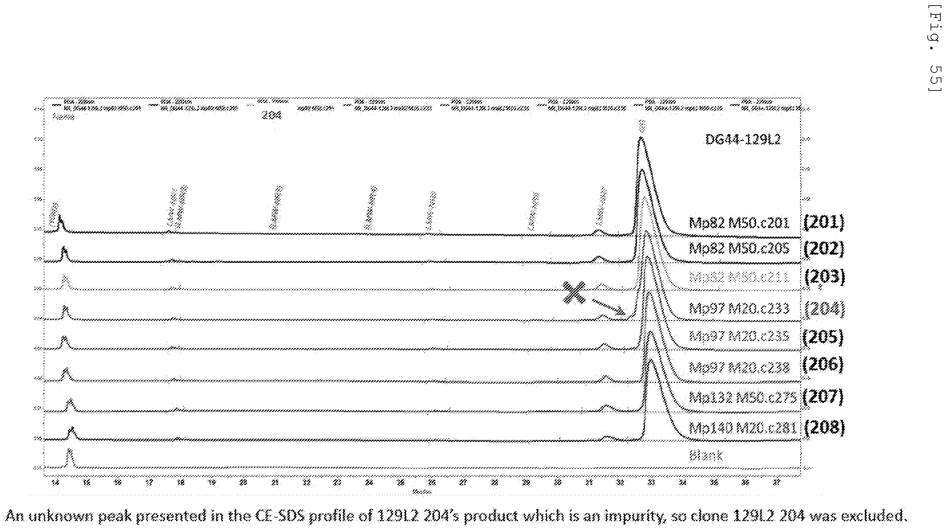

[Figure 55] Figure 55 shows the results obtained by purifying human monoclonal antibody #129-L2 from CHO cell line that stably expresses human monoclonal antibody #129-L2 and by a non-reducing capillary electrophoresis analysis.

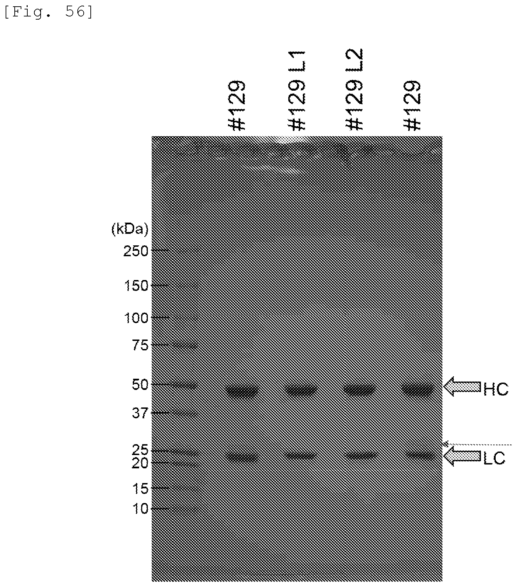

[Figure 56] Figure 56 shows the results of SDS-PAGE of human monoclonal antibody #129 purified from two clones of CHO cell line that stably expresses human monoclonal antibody #129 ("#129" in the figure), human monoclonal antibody #129-L1 purified from one clone of CHO cell line that stably expresses human monoclonal antibody #129-L1 ("#129L1" in the figure), and human monoclonal antibody #129-L2 purified from one clone of CHO cell line that stably expresses human monoclonal antibody #129-L2, respectively. In the figure, "HC" indicates a band derived from an antibody heavy chain, and "LC" indicates a band derived from an antibody light chain.

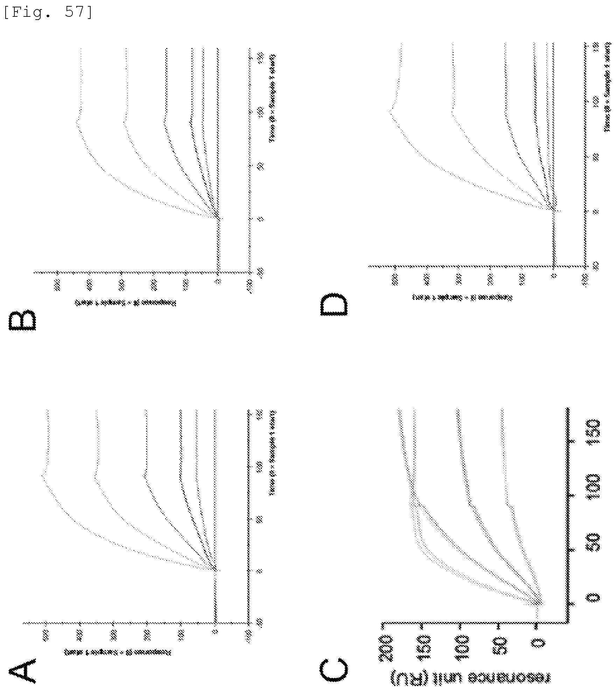

[Figure 57] Figure 57 shows the results of SPR analysis of interactions between two types of anti-human HMGB1 antibodies of the present invention (the human monoclonal antibody #129-L2 [Figures 57A and B] and the human monoclonal antibody #129 [Figures 57C and D]) before six-month storage at 4°C (left graphs) and after six-month storage at 4°C (right graphs), and the human dsHMGB1.

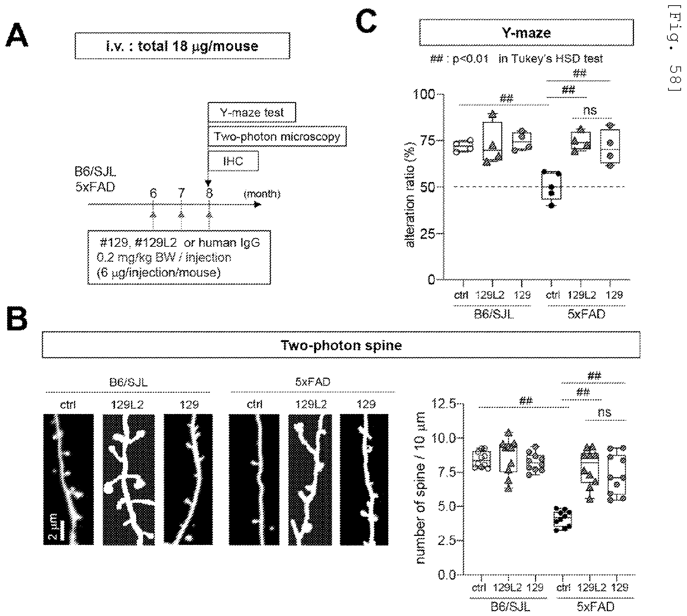

[Figure 58] Figure 58A schematically shows the therapeutic experimental protocol for AD model mice (5 × FAD mice). Figure 58B shows the results obtained by measuring the number of synapses of the cerebral cortex in the AD model mice and by examining treatment effects of the two types of anti-human HMGB1 antibodies of the present invention (the human monoclonal antibody #129-L2 and the human monoclonal antibody #129). Figure 58C shows the results of examining treatment effects of the above-described two types of anti-human HMGB1 antibodies of the present invention on decreased cognitive function of AD model mice, using a Y-maze test. The graphs in Figures 58B and C show, in order from the left, the results of control human IgG-administered normal mice ("ctrl B6/SJL" in the figure), human monoclonal antibody #129-L2-administered normal mice ("129L2 B6/SJL" in the figure), human monoclonal antibody #129-administered normal mice ("129 B6/SJL" in the figure), control human IgG-administered AD model mice ("ctrl 5 × FAD" in the figure), human monoclonal antibody #129-L2-administered AD model mice ("129L2 5 × FAD" in the figure), and human monoclonal antibody #129-administered AD model mice ("129 5 × FAD" in the figure).

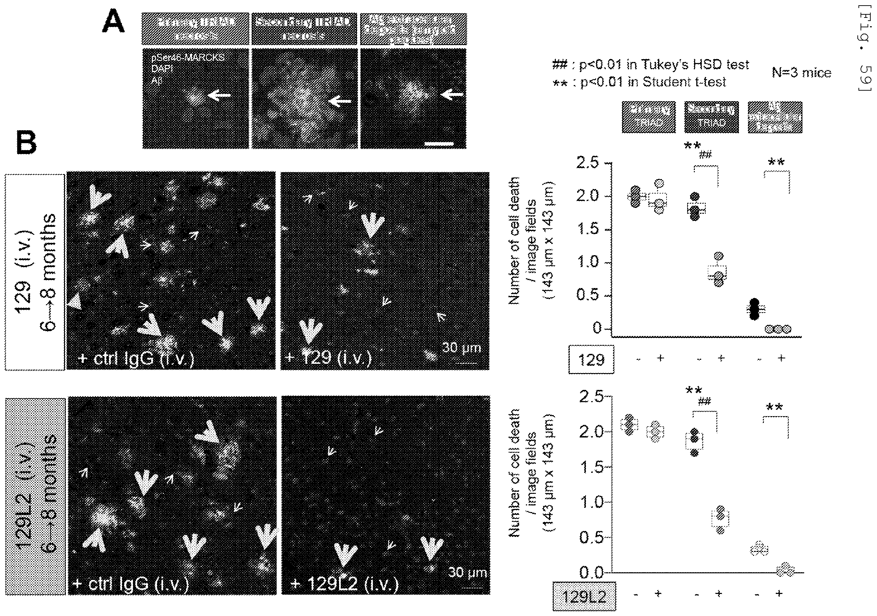

[Figure 59] Figure 59 shows the results of an immunohistochemical staining analysis of the cerebral cortex in AD model mice (5 × FAD mice) to which the two types of the anti-human HMGB1 antibodies of the present invention (human monoclonal antibody #129-L2 and human monoclonal antibody #129) were administered, using an anti-pSer46-MARCKS antibody and anti-Aβ antibody and to analyze the proportions of primary TRIAD necrosis, secondary TRIAD necrosis, and Aβ extracellular deposits (amyloid plaques). In the graph, the symbol "-" shows the results of administering control human IgG, and the symbol "+" shows the results of administering the human monoclonal antibody #129-L2 ("129L2" in the figure), or the human monoclonal antibody #129 ("129" in the figure).

### Mode of Carrying Out the Invention

### <Preventing or Treating Agent of The Present Invention>

A first aspect of the preventing or treating agent of the present invention is an agent containing the human monoclonal antibody of the present invention (hereinafter may be referred to as "the agent 1 for preventing or treating of the present invention") used for a specific purpose of "preventing or treating one or more of amyotrophic lateral sclerosis; Parkinson's disease; Huntington's disease; aging-related degenerative or neurological disease; brain aging; and diseases associated with brain aging". Further, a second aspect of the preventing or treating agent of the present invention is an agent containing the human monoclonal antibody L1/L2 of the present invention (hereinafter may be referred to as "the agent 2 for preventing or treating of the present invention") used for a specific purpose of "preventing or treating one or more of Alzheimer's disease; frontotemporal lobar degeneration; amyotrophic lateral sclerosis; Parkinson's disease; Huntington's disease; aging-related degenerative or neurological disease; brain aging; and diseases associated with brain aging" (hereinafter, "the agent 1 for preventing or treating of the present invention" and "the agent 2 for preventing or treating of the present invention" may be collectively referred to as "the agent for preventing or treating of the present invention"). The agent for preventing or treating of the present invention may be used solely as a medicine (drug product) or may be used in a form of a composition (pharmaceutical composition) by further mixing additives. Of note, the "treatment of brain aging" may be referred to as "improvement in brain aging".

### <Human Monoclonal Antibody L1/L2 of The Present Invention>

The human monoclonal antibody L1/L2 of the present invention is a human monoclonal antibody that specifically binds to human HMGB1, comprises an H chain CDR1 consisting of the amino acid sequence shown in SEQ ID No: 1, an H chain CDR2 consisting of the amino acid sequence shown in SEQ ID No: 2, and an H chain CDR3 consisting of the amino acid sequence shown in SEQ ID No: 3; and an L chain CDR1 consisting of the amino acid sequence shown in SEQ ID No: 4, an L chain CDR2 consisting of the amino acid sequence shown in SEQ ID No: 5, and an L chain CDR3 consisting of the amino acid sequence shown in SEQ ID No: 20 or 17, and falls within the scope of the human monoclonal antibody of the present invention.

As used herein, the expression "prevention of amyotrophic lateral sclerosis, Parkinson's disease, Huntington's disease, aging-related degenerative or neurological disease, brain aging, or diseases associated with brain aging" includes not only suppression of presence or development of amyotrophic lateral sclerosis, Parkinson's disease, Huntington's disease, aging-related degenerative or neurological disease, brain aging, or diseases associated with brain aging, but also delay of presence or onset time of amyotrophic lateral sclerosis, Parkinson's disease, Huntington's disease, aging-related degenerative or neurological disease, brain aging, or diseases associated with brain aging. Further, the expression "treatment (improvement) of amyotrophic lateral sclerosis, Parkinson's disease, Huntington's disease, aging-related degenerative or neurological disease, brain aging, or diseases associated with brain aging" includes not only resolution or improvement of lesions and symptoms of amyotrophic lateral sclerosis, Parkinson's disease, Huntington's disease, aging-related degenerative or neurological disease, brain aging, or diseases associated with brain aging, but also suppression of their progression.

As demonstrated in the example described later, the human monoclonal antibody of the present invention contained in the agent 1 for preventing or treating of the present invention, and the human monoclonal antibody L1/L2 of the present invention contained in the agent 2 for preventing or treating of the present invention have high affinity for human HMGB1 protein and have an effect of reducing or suppressing the phosphorylation level of serine at position 46 (Ser46) in MARCKS (pSer46-MARCKS); an effect of recovering the repair function of DNA damage in nerve cells; an effect of inhibiting TRIAD necrosis or its amplification (may be referred to as "propagation"); and an effect of reducing protein (for example, TDP43) aggregates in cerebral cortical neurons. Therefore, those caused by one or more selected from HMGB1 and/or pSer46-MARCKS; decrease in the repair function of DNA damage in nerve cells; TRIAD necrosis or its amplification; and protein (for example, TDP43) aggregates in cerebral cortical neurons can be mentioned as a preferred example of the diseases of the present invention and the like to be prevented or treated with the agent 1 for preventing or treating of the present invention, and/or Alzheimer's disease or frontotemporal lobar degeneration, and/or the diseases of the present invention and the like to be prevented or treated with the agent 2 for preventing or treating of the present invention.

Examples of the above-mentioned "aging-related degenerative or neurological disease" include Lewy body dementia, progressive supranuclear palsy, basal ganglionic degeneration, striatonigral degeneration, neuronal intranuclear inclusion disease, and multiple system degeneration. Further, the above-mentioned "brain aging" means reduced cranial nerves associated with aging, increased neuronal death, and/or decreased cognitive function. In addition, the examples of the above-mentioned "diseases associated with brain aging" include multiple sclerosis, viral encephalitis, acute demyelinating encephalomyelitis, and neuromyelitis optica.

As used herein, the term "TRIAD (necrosis)" means slow necrosis-like cell death occurred in nerve cells due to specific inhibition of RNA polymerase II, which is a basic molecule in transcription, and it is caused by decreased function of yes-associated protein (YAP) ("J Cell Biol (2006) 172(4): 589-604"). TRIAD does not have morphological and biochemical features of apoptosis, and unlike autophagic cell death, it does not have autophagosome expansion and increase, but does have a morphological feature of swelling of endoplasmic reticulum.

Target diseases of the agent for preventing or treating of the present invention may be a genetic disease (for example, diseases caused by genetic mutation, for example, valosin-containing protein (VCP) mutation such as VCP^{T262A} mutation; progranulin (PGRN) mutation such as PGRN^{R504X} mutation; charged multivesicular body protein 2B (CHMP2B) mutation such as CHMP2B^{Q165}X mutation; and transactive response DNA-binding protein of 43 kD (TDP43) mutation such as TDP43^{N267S} mutation), or may be a sporadic disease.

The human monoclonal antibody of the present invention is preferably a human monoclonal antibody comprising an H chain variable region consisting of an amino acid sequence having at least 80% or more sequence identity to the amino acid sequence shown in SEQ ID No: 7 and an L chain variable region consisting of an amino acid sequence having at least 80% or more sequence identity to the amino acid sequence shown in SEQ ID No: 8, SEQ ID No: 21, or SEQ ID No: 18, more preferably a human monoclonal antibody comprising a heavy chain consisting of an amino acid sequence having at least 80% or more sequence identity to the amino acid sequence shown in SEQ ID No: 9 and a light chain consisting of an amino acid sequence having at least 80% or more sequence identity to the amino acid sequence shown in SEQ ID No: 10, SEQ ID No: 22, or SEQ ID No: 19. Further, the human monoclonal antibody L1/L2 of the present invention is preferably a human monoclonal antibody comprising a heavy chain variable region consisting of an amino acid sequence having at least 80% or more sequence identity to the amino acid sequence shown in SEQ ID No: 7 and a light chain variable region consisting of an amino acid sequence having at least 80% or more sequence identity to the amino acid sequence shown in SEQ ID No: 21 or 18, and more preferably a human monoclonal antibody comprising a heavy chain consisting of an amino acid sequence having at least 80% or more sequence identity to the amino acid sequence shown in SEQ ID No: 9 and a light chain consisting of an amino acid sequence having at least 80% or more sequence identity to the amino acid sequence shown in SEQ ID No: 22 or 19.

As used herein, "high mobility group box 1 (HMGB1)" is a protein also called HMG1, HMG3, SBP-1, or HMG-1. Human-derived HMGB1 is typically a protein consisting of an amino acid sequence identified as NCBI reference sequence: NP_002119.1 (a protein encoded by the nucleotide sequence identified as NCBI reference sequence: NM_002128.5). However, the DNA sequence of the gene is mutated in nature (that is, non-artificially) by a mutation thereof or the like, and the amino acid sequence of the protein encoded thereby is also modified along with the mutation. Therefore, in addition to the protein consisting of the amino acid sequence identified by NCBI reference sequence: NP_002119.1, variants of this protein existing in nature also fall within the scope of HMGB1, to which the human monoclonal antibody of the present invention or the human monoclonal antibody L1/L2 of the present invention binds.

As used herein, the term "antibody that specifically binds to human HMGB1" means an antibody that recognizes and binds to human HMGB1 through a highly specific antigen-antibody recognition mechanism. The human monoclonal antibody of the present invention or the human monoclonal antibody L1/L2 of the present invention is preferably in an isolated condition. The term "isolated" used herein means that an antibody exists in a condition different from the condition in which it originally exists, achieved by extracting the antibody by artificial manipulation from the environment where it originally exists, expressing the antibody in an environment that is not the environment where it originally exists antibody, or the like. That is, the term "isolated antibody" does not include an antibody derived from a certain individual and contained in the body of the individual or in a tissue or body fluid (for example, blood, plasma, or serum) derived from the body without undergoing an external operation (artificial manipulation). Further, the human monoclonal antibody of the present invention or the human monoclonal antibody L1/L2 of the present invention is preferably an antibody produced from an organism or a cell prepared by artificial manipulation (for example, an antibody produced from hybridoma). The "antibody produced from an organism or a cell prepared by artificial manipulation" does not include an antibody produced from an organism or a B cell that exists naturally (not undergoing artificial manipulation).

As used herein, the term "monoclonal antibody" means an antibody (including a functional fragment of an antibody) obtained from a group of substantially uniform antibodies. A monoclonal antibody recognizes a single determinant on an antigen. The human monoclonal antibody of the present invention or the human monoclonal antibody L1/L2 of the present invention includes human immunoglobulin classes and subclasses, as well as forms of functional fragments of these antibodies. The classes and subclasses of the human monoclonal antibody of the present invention or the human monoclonal antibody L1/L2 of the present invention include IgG such as IgG1, IgG2, IgG3, and IgG4, IgA such as IGA1 and IGA2, IgD, IgE, and IgM.

The human monoclonal antibody of the present invention and the human monoclonal antibody L1/L2 of the present invention typically comprise heavy chain CDR1, heavy chain CDR2, heavy chain CDR3, light chain CDR1, light chain CDR2, and light chain CDR3, and have a framework region (FR) linked to the amino (N) terminus and the carboxyl (C) terminus of each region of these CDR1 to CDR3, and comprise a heavy chain variable region and light chain variable region.

Among the above-mentioned FRs, examples of a heavy chain FR include heavy chain FR1 linked to the N terminus of heavy chain CDR1, heavy chain FR2 linked between the C terminus of heavy chain CDR1 and the N terminus of heavy chain CDR2, heavy chain FR3 linked between the C terminus of heavy chain CDR2 and the N terminus of heavy chain CDR3, and heavy chain FR4 linked to the C terminus of heavy chain CDR3. Further, among the above-mentioned FRs, examples of a light chain FR include light chain FR1 linked to the N terminus of the light chain CDR1, light chain FR2 linked between the C terminus of light chain CDR1 and the N terminus of light chain CDR2, light chain FR3 linked between the C terminus of light chain CDR2 and the N terminus of light chain CDR3, and light chain FR4 linked to the C terminus of light chain CDR3.

Specific examples of the above-mentioned heavy chain FR1 include (HF1) a polypeptide consisting of amino acid residues 1 to 30 of the amino acid sequence shown in SEQ ID No: 7 and (HF1') a polypeptide consisting of an amino acid sequence having at least 80% or more sequence identity to this polypeptide;
specific examples of the above-mentioned heavy chain FR2 include (HF2) a polypeptide consisting of amino acid residues 36 to 49 of the amino acid sequence shown in SEQ ID No: 7 and (HF2') a polypeptide consisting of an amino acid sequence having at least 80% or more sequence identity to this polypeptide;
specific examples of the above-mentioned heavy chain FR3 include (HF3) a polypeptide consisting of amino acid residues 67 to 98 of the amino acid sequence shown in SEQ ID No: 7, a polypeptide consisting of amino acid residues 67 to 98 of the amino acid sequence shown in SEQ ID No: 11, and (HF3') a polypeptide consisting of an amino acid sequence having at least 80% or more sequence identity to either of these polypeptides; and
specific examples of the above-mentioned heavy chain FR4 include (HF4) a polypeptide consisting of amino acid residues 105 to 115 of the amino acid sequence shown in SEQ ID No: 7 and (HF4') a polypeptide consisting of an amino acid sequence having at least 80% or more sequence identity to this polypeptide.

Specific examples of the above-mentioned light chain FR1 include (LF1) a polypeptide consisting of amino acid residues 1 to 23 of the amino acid sequence shown in SEQ ID No: 8 and (LF1') a polypeptide consisting of an amino acid sequence having at least 80% or more sequence identity to this polypeptide;
specific examples of the above-mentioned light chain FR2 include (LF2) a polypeptide consisting of amino acid residues 35 to 49 of the amino acid sequence shown in SEQ ID No: 8 and (LF2') a polypeptide consisting of an amino acid sequence having at least 80% or more sequence identity to this polypeptide;
specific examples of the above-mentioned light chain FR3 include (LF3) a polypeptide consisting of amino acid residues 57 to 88 of the amino acid sequence shown in SEQ ID No: 8 and (LF3') a polypeptide consisting of an amino acid sequence having at least 80% or more sequence identity to this polypeptide; and
specific examples of the above-mentioned light chain FR4 include (LF4) a polypeptide consisting of amino acid residues 98 to 107 of the amino acid sequence shown in SEQ ID No: 8 and (LF4') a polypeptide consisting of an amino acid sequence having at least 80% or more sequence identity to this polypeptide.

The human monoclonal antibody of the present invention and the human monoclonal antibody L1/L2 of the present invention are human antibodies. Examples of the "human antibodies" include a human chimeric antibody, a humanized antibody, and a complete human antibody, and preferred examples thereof are a humanized antibody and a complete human antibody.

As used herein, the "human chimeric antibody" has the variable region of an antibody derived from a non-human animal (for example, a non-human mammal such as a chicken, a mouse, a rat, or a bovine) and the constant region of a human-derived antibody linked to each other. The human chimeric antibody can be obtained by, for example, immunizing a non-human animal (preferably a non-human mammal) with an antigen, excising an antibody variable region that binds to the antigen from the gene of the mouse monoclonal antibody, binding it to the gene of the constant region of an antibody derived from the human bone marrow, and incorporating the gene into an expression vector, so that the expression vector is introduced into a host to produce the human chimeric antibody (for example, Japanese unexamined Patent Application Publication No. 8-280387; U.S. Patent No. 4816397; U.S. Patent No. 4816567; and U.S. Patent No. 5807715).

Examples of a human constant region of a human chimeric antibody include Cγ1, Cy2, Cy3, Cy4, Cµ, Cδ, Cα1, Cα2, and Cε in the heavy chain and Cκ and Cλ in the light chain. The amino acid sequences of these constant regions and the nucleotide sequences encoding them are known. Further, to improve the stability of an antibody itself or the stability of antibody production, one or more amino acids in the constant region derived from a human antibody can be substituted, deleted, added, and/or inserted.

As used herein, the "humanized antibody" is an antibody obtained by transplanting the gene sequence of the antigen-binding site (a CDR) of an antibody (that is, CDR grafting) derived from a non-human animal (for example, a non-human mammal such as a chicken, a mouse, a rat, or a bovine) into an antibody gene derived from a human, and the methods for preparing it, such as overlap extension PCR, are known (see, for example, European Patent Application No. 239400, European Patent Application No. 125023, International Publication No. WO 90/07861, and International Publication No. WO 96/02576). The variable region of an antibody is usually composed of three CDRs sandwiched by four framework regions (FRs). A CDR is a region that substantially determines the binding specificity of an antibody. While the amino acid sequence of a CDR is rich in diversity, amino acid sequences constituting an FR often show high homology among antibodies having different binding specificity. Thus, it is generally said that the binding specificity of an antibody can be transplanted to other antibodies by transplanting a CDR. Further, in transplantation of a non-human-derived CDR to a human FR, a human FR with high homology to the non-human animal-derived FR is selected from viewpoints of maintaining the function of the CDR. In other words, amino acids in a CDR not only recognize an antigen, but also coordinate with amino acids of an FR in the vicinity of the CDR, and are involved in maintenance of the loop structure of the CDR. Therefore, a human FR consisting of amino acid sequences with high homology to the amino acid sequences of an FR adjacent to a CDR to be transplanted is preferably used.

A known human FR with high homology to a non-human animal-derived FR can be searched by using, for example, a search system which is available on the Internet and specialized in antibodies (<http://www.bioinf.org.uk/abysis/>). A mutation can be introduced into the sequence of a non-human-derived antibody other than CDR so that it is consistent with the sequence of a thus-obtained human FR. Alternatively, when a gene (cDNA) encoding the amino acid sequence of a human FR obtained by search is available, a non-human-derived CDR may be introduced into the sequence. Introduction of a mutation and the like can be performed by using techniques known to the field, such as nucleic acid synthesis and site specific mutation induction.

By qualitatively or quantitatively measuring and assessing the affinity of a humanized antibody prepared in this manner for an antigen, an FR of a human-derived antibody in which the FR is linked via a CDR, and the CDR forms a favorable antigen-binding site can be suitably selected. Further, as necessary, an amino acid residue in the FR can also be substituted in accordance with the method described in "Cancer Res., 1993, 53, 851-856" or the like, so that a CDR of a humanized antibody forms an appropriate antigen-binding site. Further, a variant FR sequence having an intended property can be selected by measuring and assessing the antigen affinity of a variant antibody in which an amino acid has been substituted.

As used herein, the term "complete human antibody" means an antibody in which all sequences in the antibody are human-derived sequences. A complete human antibody can be prepared, for example, in a transgenic mouse which has been engineered so as to express the gene of an antibody having human heavy and light chains. A transgenic mouse producing a human antibody can be prepared in accordance with, for example, the methods described in International Publication No. WO 02/43478, U.S. Patent No. 6657103 (Abgenix), and the like. Further, a hybridoma cell line fused with a B cell derived from a transgenic mouse producing an intended antibody can be prepared in accordance with, for example, the methods described in U.S. Patent No. 5569825, U.S. Patent No. 5625126, U.S. Patent No. 5633425, U.S. Patent No. 5661016, U.S. Patent No. 5545806, "Jakobovits, Adv. Drug Del. Rev. 31:33-42 (1998)," and "Green, et al, J. Exp. Med. 188:483-95 (1998)."

As described above, the human monoclonal antibody of the present invention or the human monoclonal antibody L1/L2 of the present invention also includes one portion (a partial fragment) of an antibody which is a functional fragment specifically recognizing HMGB1 protein, in addition to an antibody consisting of a whole antibody. Examples of such a functional fragment include Fab, Fab', F(ab')₂, a variable region fragment (Fv), a disulfide-bond Fv, a single-chain Fv (scFv), sc(Fv)₂, a diabody, a polyspecific antibody, and polymers thereof.

The term "Fab" used herein means a monovalent antigen-binding fragment of an immunoglobulin consisting of one light chain and a portion of a heavy chain. A "Fab" can be obtained by papain digestion of an antibody or a genetic recombination method. The term "Fab'" is made different from Fab by adding a small number of residues to the carboxyl terminus of the heavy chain CH1 domain, including one or more cysteines in the hinge region of an antibody. The term "F(ab')₂" means a bivalent antigen-binding fragment of an immunoglobulin consisting of portions of two light chains and two heavy chains.

A "variable region fragment (Fv)" is the smallest antibody fragment that has a complete antigen recognizing and binding site. The Fv is a dimer in which a heavy chain variable region and a light chain variable region are strongly linked to each other by a noncovalent bond. The "single-chain Fv (scFv)" comprises a heavy chain variable region and a light chain variable region of an antibody, and these regions exist in a single polypeptide chain. The "sc(Fv)₂" is obtained as a single chain by linking two heavy chain variable regions and two light chain variable regions with linkers or the like. The "diabody" is a small antibody fragment having two antigen-binding sites, and this fragment comprises a heavy chain variable region bound to a light chain variable region in the same polypeptide chain, and each region is paired with a complementary region in another chain. The "polyspecific antibody" is a monoclonal antibody having binding specificity to at least two different antigens. For example, a polyspecific antibody can be prepared by simultaneously expressing two pairs of an immunoglobulin heavy chain and a light chain in which two heavy chains have different specificity.

The H chain CDR1 to CDR3 and the L chain CDR1 to CDR3 in the human monoclonal antibody of the present invention include modified CDRs in which one or more amino acids have been substituted, deleted, added, and/or inserted in the amino acid sequences shown in SEQ ID Nos: 1 to 6 without reducing a desirable activity (that is, affinity for HMGB1). The human monoclonal antibody of the present invention comprising the modified CDRs can be prepared by, for example, introducing a mutation into a DNA encoding the antibody chain of the human monoclonal antibody #129 or synthesizing a peptide. Modification of amino acids in regions other than CDRs in an antibody (for example, an FR, a constant region) is considered to have a relatively small effect on the affinity for an antigen. Currently, techniques for screening for an antibody with antigen affinity that has been enhanced by modifying an amino acid of a CDR are known (for example, "PNAS, 102:8466-8471 (2005)," "Protein Engineering, Design & Selection, 21:485-493 (2008)," International Publication No. WO 2002/051870, "J. Biol. Chem., 280:24880-24887 (2005)," "Protein Engineering, Design & Selection, 21:345-351 (2008)," or "MAbs. Mar-Apr; 6(2):437-45 (2014)"). Further, currently, an antibody with antigen affinity that has been enhanced by utilizing an integrated computational chemistry system or the like (for example, Molecular Operating Environment (MOE; manufactured by Chemical Computing Group [CCG], Canada)) can be modeled (for example, <http://www.rsi.co.jp/kagaku/cs/ccg/products/application/p rotein.htmL>).

As used herein, the expression "(one or) more amino acids" in "one or more amino acids have been substituted, deleted, added, and/or inserted" means preferably 10 or less amino acids, more preferably five or less amino acids, yet more preferably three or less amino acids (for example, two or less amino acids, one amino acid). A preferred example of the substitution, deletion, addition, and insertion of amino acids is conservative substitution. The "conservative substitution" means substitution with other amino acid residues having a chemically similar side chain. Groups of amino acid residues having a chemically similar amino acid side chain are well known in this technical field. For example, acidic amino acids (aspartic acid and glutamic acid), basic amino acids (lysine, arginine, and histidine), and neutral amino acids can be classified into amino acids having a hydrocarbon chain (glycine, alanine, valine, leucine, isoleucine, and proline), amino acids having a hydroxy group (serine and threonine), amino acids containing sulfur (cysteine and methionine), amino acids having an amide group (asparagine and glutamine), an amino acid having an imino group (proline), and amino acids having an aromatic group (phenylalanine, tyrosine, and tryptophan).

The H chain CDR1 in the human monoclonal antibody of the present invention is preferably an H chain CDR1 consisting of the amino acid sequence shown in SEQ ID No: 1 as its effect is demonstrated in the example described later, the H chain CDR2 in the human monoclonal antibody of the present invention is preferably an H chain CDR2 consisting of the amino acid sequence shown in SEQ ID No: 2 as its effect is demonstrated in the example described later, and the H chain CDR3 in the human monoclonal antibody of the present invention is preferably an H chain CDR3 consisting of the amino acid sequence shown in SEQ ID No: 3 as its effect is demonstrated in the example described later; and the L chain CDR1 in the human monoclonal antibody of the present invention is preferably an L chain CDR1 consisting of the amino acid sequence shown in SEQ ID No: 4 as its effect is demonstrated in the example described later, the L chain CDR2 in the human monoclonal antibody of the present invention is preferably an L chain CDR2 consisting of the amino acid sequence shown in SEQ ID No: 5 as its effect is demonstrated in the example described later, and the L chain CDR3 in the human monoclonal antibody of the present invention is preferably an L chain CDR3 consisting of the amino acid sequence shown in SEQ ID No: 6, 20, or 17 as its effect is demonstrated in the example described later.

As used herein, the expression "at least 80% or more sequence identity" to a predetermined amino acid sequence means preferably at least 85% or more sequence identity, more preferably at least 90% or more sequence identity, yet more preferably at least 95% or more (for example, at least 96% or more, at least 97% or more, at least 98% or more, at least 99% or more, 100%). Homology of amino acid sequences is determined by using a BLASTP (amino acid level) program (for example, "J. Mol. Biol., 215:403-410, 1990"). The program is based on a BLAST algorithm (for example, "Proc. Natl. Acad. Sci. USA, 87:2264-2268, 1990" and "Proc. Natl. Acad. Sci. USA, 90:5873-5877, 1993"). When amino acid sequences are analyzed using a BLASTP, parameters are set at, for example, a score of 50 and a word length of 3. Further, when amino acid sequences are analyzed using a gapped BLAST program, the analysis can be performed in accordance with the method described in "Nucleic Acids Res. 25:3389-3402, 1997." When a BLAST and a gapped BLAST program are used, default parameters for each program are used. Specific techniques of these analysis methods are known.

The human monoclonal antibody of the present invention or the human monoclonal antibody L1/L2 of the present invention also includes human monoclonal antibodies obtained by modifying a process following translation of an antibody, for example, changing the number or position of glycosylation sites. Such modified human monoclonal antibodies can improve the antibody-dependent cellular cytotoxicity (ADCC) activity of an antibody. The glycosylation of an antibody is typically a N-linked or O-linked glycosylation. Glycosylation of an antibody is largely dependent on a host cell used to express the antibody. The glycosylation pattern can be modified by a known method such as introduction or deletion of a specific enzyme involved in sugar production (Japanese unexamined Patent Application Publication No. 2008-113663, U.S. Patent No. 5047335, U.S. Patent No. 5510261, U.S. Patent No. 5278299, International Publication No. WO 99/54342). Further, the human monoclonal antibody of the present invention or the human monoclonal antibody L1/L2 of the present invention may be a human monoclonal antibody with deamidation inhibited by substituting a deamidated amino acid or an amino acid adjacent to the deamidated amino acid with another amino acid for the purpose of increasing antibody stability, or a human monoclonal antibody with antibody stability enhanced by substituting glutamic acid with another amino acid.

The human monoclonal antibody of the present invention or the human monoclonal antibody L1/L2 of the present invention can be prepared by a known hybridoma method or a known DNA recombination method. Representative examples of the hybridoma method include the method of Kohler and Milstein (see "Nature, 256:495 (1975**)").** Examples of the antibody-producing cell used in the cell fusion process in this method include a spleen cell, a lymph node cell, and a peripheral white blood cell of an animal (for example, a mammal such as a mouse, a rat, a hamster, a rabbit, a monkey, or a goat) immunized with an antigen (for example, HMGB1 protein, a partial peptide thereof, a protein obtained by fusing these proteins with Fc protein or the like, a cell expressing these proteins). An antibody-producing cell obtained by allowing an antigen to act on the above-mentioned cell or a lymphocyte isolated beforehand from an unimmunized animal in a medium can also be used. As a myeloma cell, various known cell lines can be used. An antibody-producing cell and a myeloma cell may be derived from different animal species as long as they can be fused, but are preferably derived from an identical animal species as a source. A hybridoma is produced by cell fusion of, for example, a spleen cell obtained from a mouse immunized with an antigen and a mouse myeloma cell, and a hybridoma producing a monoclonal antibody specific to HMGB1 protein can be obtained by subsequent screening. A human monoclonal antibody that specifically binds to HMGB1 protein can be obtained by culturing a hybridoma or from the ascites of a mammal to which a hybridoma has been administered.

The DNA recombination method is a technique in which an antibody gene encoding the human monoclonal antibody of the present invention or the human monoclonal antibody L1/L2 of the present invention is cloned from a hybridoma, a B cell, or the like and incorporated into a suitable vector, and this vector is introduced into a host cell (for example, a mammal cell line such as HEK cell, *Escherichia coli,* a yeast cell, an insect cell, a plant cell) to produce the human monoclonal antibody of the present invention or the human monoclonal antibody L1/L2 of the present invention as a recombinant antibody (for example, "Eur. J. Biochem. 192:767-775 (1990)"). When an antibody gene encoding the human monoclonal antibody of the present invention or the human monoclonal antibody L1/L2 of the present invention is expressed, a host cell may be transformed by incorporating antibody genes encoding a heavy chain and a light chain into separate expression vectors or by incorporating the antibody genes encoding a heavy chain and a light chain into a single expression vector (International Publication No. WO 94/11523). The human monoclonal antibody of the present invention or the human monoclonal antibody L1/L2 of the present invention can be obtained in a substantially pure and uniform form by culturing the above-mentioned host cell and isolating the antibody out of the host cell or a culture broth and purifying it. Isolation and purification of an antibody can be performed by using a method usually used in purification of a polypeptide. By preparing a transgenic animal (for example, a bovine, a goat, a sheep, a swine) into which an antibody gene has been incorporated by using a technique to prepare a transgenic animal, a large amount of a human monoclonal antibody derived from the human monoclonal antibody of the present invention gene or the human monoclonal antibody L1/L2 of the present invention gene can be obtained from milk of the transgenic animal.

Examples of the additive as used herein include pharmaceutically acceptable formulation ingredients such as a carrier, a binder, a stabilizer, an excipient, a diluent, a pH buffer, an isotonic agent, a dissolving aid, and a nutrient. Examples of the pharmaceutically acceptable carrier include mannitol, lactose, sucrose, and human albumin when the agent for preventing or treating of the present invention is a powder; and physiological saline, water for injection, a phosphate buffer solution, and aluminum hydroxide when the agent for preventing or treating of the present invention is a liquid.

A subject for administration of the agent 1 for preventing or treating of the present invention is sufficient as long as the subject (human) requires prevention or treatment of the diseases of the present invention and the like, and examples thereof include a subject diagnosed as having a high risk of onset or development of the diseases of the present invention and the like, and a patient who has the onset of or is developing the diseases of the present invention and the like. Further, a subject for administration of the agent 2 for preventing or treating of the present invention is sufficient as long as the subject (human) requires prevention or treatment of Alzheimer's disease or frontotemporal lobar degeneration, and/or the diseases of the present invention and the like, and examples thereof include a subject diagnosed as having a high risk of onset or development of Alzheimer's disease or frontotemporal lobar degeneration, and/or the diseases of the present invention and the like, and a patient who has the onset of or is developing Alzheimer's disease or frontotemporal lobar degeneration, and/or the diseases of the present invention and the like.

Examples of a method for administering the agent for preventing or treating of the present invention include administration by injection (for example, subcutaneous administration, intramuscular administration, intravenous administration, intraarterial administration), administration through an oral cavity mucous membrane, intrarectal administration, local administration into the brain (intracerebroventricular administration), percutaneous administration, and oral administration, and a preferred example is intravenous administration. The dose of the human monoclonal antibody of the present invention and the human monoclonal antibody L1/L2 of the present invention can vary depending on the age, body weight, sex, health condition, and severity of progression of symptoms of a subject for administration, but the dose for adults is usually 0.1 to 1000 mg, preferably 1 to 100 mg per kg of body weight daily. The agent 1 for preventing or treating of the present invention may be used in combination with a known medicine used in the prevention or treatment of the diseases of the present invention and the like. Further, the agent 2 for preventing or treating of the present invention may be used in combination with a known medicine used in the prevention or treatment of Alzheimer's disease or frontotemporal lobar degeneration, and/or the diseases of the present invention and the like.

The present invention will be specifically described using an example below, but the technical scope of the present invention is not limited to this example. Of note, this example was conducted strictly in compliance with the recommendations in the guidelines on management and use of laboratory animals of the Japanese government and the National Institute of Health Sciences. Further, all experiments were approved by the gene recombination experiment committee, the ethics committee, and the animal experiment committee of the Tokyo Medical and Dental University.

### Example 1

### 1. Materials and methods

### [Anti-human HMGB1 antibody]

In this example, a human monoclonal antibody (humanized IgG antibody) #129 prepared in the examples in an international patent application (PCT-JP2019-36926 [International Publication No. WO 2020/059847]) was used as an anti-human HMGB1 antibody. The amino acid sequences of the H chain and the L chain of the human monoclonal antibody #129 are shown in Tables 1 and 2. Of note, when the human monoclonal antibody #129 was subcutaneously administered to a mouse, and the antibody concentrations in the brain and the serum were measured by the ELISA method. The results demonstrated that 2% to 8% of the administered human monoclonal antibody #129 had passed the blood brain barrier (BBB) and had been transferred into the brain.

**[Table 1]**

| |
|---|
| Amino acid sequence of H chain of human monoclonal antibody #129 (SEQ ID No: 9) |
| EVQLLESGGGLVQPGGSLRLSCAASGFTFSSYAMSWVRQAPGKGLEWVSDISGS GGSTYYADSVKGRFTISRDNSKNTLYLQMNSLRAEDTAVYYCARGYGMDVWGQ GTMVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPA PIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKS LSLSPGK |

The underlined region in the table represents the H chain V region (SEQ ID No: 7). The three regions surrounded in a square in the table represent the H chain CDR1 (SEQ ID No: 1), the H chain CDR2 (SEQ ID No: 2), and the H chain CDR3 (SEQ ID No: 3) in order from the amino terminus side.

**[Table 2]**

| |
|---|
| Amino acid sequence of L chain of human monoclonal antibody #129 (SEQ ID No: 10) |
| DIQMTQSPSSLSASVGDRVTITCRASQSVTNYLAWYQQKPGKAPKLLIYGASIL ETGVPSRFSGSGSGTDFTLTINSLQPEDFATYYCLQHNSTPLTFGQGTKLEIKT VAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC |

The underlined region in the table represents the L chain V region (SEQ ID No: 8). The three regions surrounded in a square in the table represent the L chain CDR1 (SEQ ID No: 4), the L chain CDR2 (SEQ ID No: 5), and the L chain CDR3 (SEQ ID No: 6) in order from the amino terminus side.

### [Analysis of antibody affinity using surface plasmon resonance (SPR) method]

Interactions with human HMGB1 protein were analyzed using the human monoclonal antibody #129 of the present invention and a mouse monoclonal antibody 2C8C described in International Publication No. WO 2018/030405 as a comparative control. Specifically, interactions were measured over time with a surface plasmon resonance measuring device ("Biacore T100"; manufactured by GE Healthcare) using a sensor chip on which the human monoclonal antibody #129 of the present invention or the mouse monoclonal antibody 2C8C was immobilized and the human HMGB1 protein as an analyte, and the equilibrium dissociation constant (KD value), which is an indicator of antibody affinity, was calculated (Figure 1).

### [Preparation of four types of FTLD-ALS model mice]

Among four types of FTLD-ALS model mice (VCP^{T262A}-KI mice, PGRN^{R504X}-KI mice, CHMP2B^{Q165X}-KI mice, and TDP-43^{N267S}-KI mice) (herein sometimes referred to as "four types of FTLD-ALS model mice"), the PGRN^{R504X}-KI mouse was prepared in accordance with the method described in "Nat. Commun., 2018 9, 433."

Further, a targeting vector for preparing the VCP^{T262A}-KI mouse was constructed. Specifically, a PCR product of a 5.4-kb Not*I*-Xho*I* fragment amplified from a Bac clone (ID: RP23-111G9 or RP23-124L1) was subcloned at the PspOM*I*-Xho*I* site in pBS-DTA. Similarly, a 2.9-kb BamHI-NotI fragment amplified from the same Bac clone was subcloned in pBS-LNL(-) having a Neo cassette (loxP-Neo-loxP). A 4.5-kb fragment (the 1.6-kb Neo cassette + the 2.9-kb fragment) was excised by treatment with NotI, and a 0.8-kb Xho*I*-Sma*I* fragment having a T262A mutation was amplified from the same Bac clone by PCR and treated with Xho*I* and Sma*I*. These 0.8-kb and 4.5-kb fragments were subcloned at the XhoI-NotI site in the targeting vector. After the targeting vector was linearized by treatment with NotI, these fragments were electroporated into ES cells having a C57BL/6J background. VCP^{T262A}-KI mice were selected from non-transgenic littermates by PCR using a primer set (Primer 1 [5'-ATATGCTCTCACTGTATGGTATTGC-3'; SEQ ID No: 11] and Primer 2 [5'-TCCAGATGAGCTTAGAAGATTAGAA-3'; SEQ ID No: 12]) to amplify a DNA fragment containing a LoxP sequence.

Further, a targeting vector for preparing a CHMP2B^{Q165X}-KI mouse was constructed. Specifically, a PCR product of a 5.7-kb Cla*I*-Sal*I* fragment amplified from a Bac clone (ID: RP23-13H5 or RP23-273E18) was subcloned at the Clal*I*-Sal*I* site in pBS-DTA (manufactured by Unitech Inc.). A 3.0-kb SacII-NotI fragment was amplified from the Bac clone and was subcloned in pBS-LNL(+) having a Neo cassette (loxP-Neo-loxP). A 4.6-kb SacII-NotI fragment (1.6-kb Neo cassette + 3.0-kb fragment) was subcloned at the Sac*II*-Cla*I* site in the targeting vector. CHMP2B^{Q165X}-KI mice were selected from non-transgenic littermates by PCR using a primer set (Primer 3 [5'-TGGATTTTATTTATGTCTGAATGTG-3'; SEQ ID No: 13] and Primer 4 [5'-ATAAGCAACTTCACAAGGCATCTTA-3'; SEQ ID No: 14]) to amplify a DNA fragment containing a LoxP sequence.

Further, a targeting vector for preparing a TDP-43^{N2675}-KI mouse was constructed. Specifically, a 5.9-kb Cla*I*-Sal*I* fragment was amplified from a Bac clone (ID: RP23-364M1 or RP23-331P21) and subcloned at the Cla*I*-Sal*I* site in pBS-TK (manufactured by Unitech Inc.). A 2.7-kb SacII-NotI fragment was amplified from the Bac clone and subcloned in pA-LNL(+) having a Neo cassette (loxP-Neo-loxP). A 4.3-kb fragment (1.6-kb Neo cassette + 2.7-kb fragment) was subcloned at the Sac*II*-Cla*I* site in the targeting vector. TDP-43^{N267S}-KI mice were selected from non-transgenic littermates by PCR using a primer set (Primer 5 [5'-ACAGTTGGGTGTGATAGCAGGTACT-3'; SEQ ID No: 15] and Primer 6 [5'-TCGAGAATTACAGGAATGTATCATC-3'; SEQ ID No: 16]) to amplify a DNA fragment containing a LoxP sequence.

### [Immunohistochemical staining method]

The brain was enucleated from four types of FTLD-ALS model mice or C57BL/6J mice of various months of age and immobilized in PBS containing 4% paraformaldehyde for 12 hours, and a paraffin-embedded brain tissue section with a thickness of 5 µm was prepared using a microtome (manufactured by Yamato Kohki Industrial Co., Ltd.). The prepared paraffin-embedded brain tissue section was deparaffinized using xylene, immersed in a 0.01 M citric acid buffer solution (pH 6.0) for re-hydration, and then heated at 120°C for 15 minutes. Then, permeabilization was performed in PBS containing 0.5% triton-X100, and blocking was performed in PBS containing 10% FBS for 60 minutes. Then, antibody reactions were allowed to occur at 4°C for 12 hours using various primary antibodies to detect 11 types of proteins (pSer46-MARCKS, γH2AX, 53BP1, phosphorylated TDP-43 [Ser409/410] [pSer409/410-TDP-43], ubiquitin, p62, KDEL, MAP2, PSD95, VAMP2, and phosphorylated tau [Ser203 (mouse) /Ser214 (human)] [pSer203-tau]) (Table 3). Cells were washed three times with PBS, an antibody reaction was allowed to occur at room temperature for one hour using secondary antibodies against various primary antibodies (Table 3), cells were washed three times with PBS, the cell nuclei were stained in PBS containing 0.2 µg/mL 4',6-diamidino-2-phenylindole (DAPI), and a fluorescence image derived from the above-mentioned 11 types of proteins and a fluorescence image derived from the cell nuclei were captured using a confocal microscope (FV1200IX83; manufactured by Olympus). Then, the number of pSer46-MARCKS-positive cells per region of 143 µm × 143 µm was calculated on the basis of the DAPI fluorescence image and the pSer46-MARCKS fluorescence image (Figures 2-1 and 9). Further, a KDEL signaling region in MAP2-positive cells, wherein the MAP2 is a mature neuron marker, was measured on the basis of the DAPI fluorescence image, the KDEL fluorescence image, and the MAP2 fluorescence image (Figure 10). Further, the γH2AX signaling region in MAP2-positive cells was measured on the basis of the DAPI fluorescence image, the γH2AX fluorescence image, and the MAP2 fluorescence image (Figure 11). Further, the 53BP1 signaling region in MAP2-positive cells was measured on the basis of the DAPI fluorescence image, the 53BP1 fluorescence image, and the MAP2 fluorescence image (Figure 12). Further, the pSer409/410-TDP-43 signaling region in a ubiquitin-positive inclusion was measured on the basis of the DAPI fluorescence image, the pSer409/410-TDP-43 fluorescence image, and the ubiquitin fluorescence image (Figure 13). Further, the p62 signaling region in an inclusion was measured on the basis of the DAPI fluorescence image and the p62 fluorescence image (Figure 14). Further, the proportion of cells with a VAMP2 signal and a PSD95 signal colocalized therein was measured on the basis of the DAPI fluorescence image, the VAMP2 fluorescence image, and the PSD95 fluorescence image (Figure 15). Further, the proportion of cells with a pSer203-tau signal and a PSD95 signal colocalized therein was measured on the basis of the DAPI fluorescence image, the pSer203-tau fluorescence image, and the PSD95 fluorescence image (Figure 16).

**[Table 3]**

| Primary antibody | Secondary antibody (antibody against the primary antibody in the left column) |
|---|---|
| Rabbit anti-pSer46-MARCKS antibody (manufactured by GL Biochem; dilution factor, 1:2,000) | Cy3-labeled anti-rabbit IgG (manufactured by Jackson Laboratory; dilution factor, 1:500) |
| Mouse anti-yH2AX antibody (Ser139; #05-636; manufactured by Millipore; dilution factor, 1:200) | Alexa488-labeled anti-mouse IgG (A21202; manufactured by Molecular Probes; dilution factor, 1:1000) |
| Rabbit anti-53BP1 antibody (NB100-304; manufactured by Novus Biologicals; dilution factor, 1:5,000) | Alexa488-labeled anti-rabbit IgG, (A21206; manufactured by Molecular Probes; dilution factor, 1:1000) |
| Rabbit anti-phosphorylated TDP43 (Ser409/410) antibody (TIP-PTD-P02; manufactured by Cosmo Bio; dilution factor, 1:5,000) | Alexa488-labeled anti-rabbit IgG, (A21206; manufactured by Molecular Probes; dilution factor, 1:1000) |
| Mouse anti-ubiquitin antibody (P4D1; manufactured by Cell Signaling Technology; dilution factor, 1:10,000) | Cy3-labeled anti-mouse IgG (715-165-150; manufactured by Jackson Laboratory; dilution factor, 1:500) |
| Mouse anti-p62 antibody (#610497; manufactured by BD Bioscience; dilution factor, 1:200) | Alexa488-labeled anti-mouse IgG (A21202; manufactured by Molecular Probes; dilution factor, 1:1000) |
| Mouse anti-KDEL antibody (ADI-SPA-827; manufactured by Enzo Life Science; dilution factor, 1:200) | Alexa488-labeled anti-mouse IgG (A21202; manufactured by Molecular Probes; dilution factor, 1:1000) |
| Rabbit anti-MAP2 antibody (ab32454; manufactured by Abcam; dilution factor, 1:1,000) | Cy3-labeled anti-rabbit IgG (manufactured by Jackson Laboratory; dilution factor, 1:500) |
| Mouse anti-MAP2 antibody (sc-32791; manufactured by Santa Cruz Biotechnology; dilution factor, 1:50) | Cy3-labeled anti-mouse IgG, (715-165-150; manufactured by Jackson Laboratory; dilution factor, 1:500) |
| Rabbit anti-PSD95 antibody (D74D3; manufactured by Cell Signaling Technology; dilution factor, 1:200) | Alexa488-labeled anti-rabbit IgG, (A21206; manufactured by Molecular Probes; dilution factor, 1:1000) |
| Mouse anti-VAMP2 antibody (GTX634812; manufactured by GeneTex; dilution factor, 1:200) | Cy3-labeled anti-mouse IgG, (715-165-150; manufactured by Jackson Laboratory; dilution factor, 1:500) |
| Rabbit anti-phosphorylated tau (Ser203/Ser214) antibody (ab170892; manufactured by Abcam; dilution factor, 1:200) | Cy3-labeled anti-rabbit IgG (manufactured by Jackson Laboratory; dilution factor, 1:500) |
| Mouse anti-PSD95 antibody (MA1-045; manufactured by Thermo Fisher Scientific; dilution factor, 1:200) | Alexa488-labeled anti-mouse IgG (A21202; manufactured by Molecular Probes; dilution factor, 1:1000) |

### [Nissl's staining method]

The paraffin-embedded brain tissue sections subjected to the deparaffinization treatment and the rehydration treatment in the above-described section of [Immunohistochemical staining method] were washed with deionized water and immersed in a cresyl violet solution (a 300-mL solution containing 0.1% cresyl violet and five drops of 10% acetic acid solution) at 37°C for 15 minutes. Then, the tissue was dehydrated with an ethanol solution and made transparent with xylene, and then an image of a necrotized brain tissue was captured using an electron microscope (H-7100; manufactured by Hitachi High-Tech Corporation) (Figure 2-2).

### [Administration of anti-human HMGB1 antibody]

At a frequency of once per month during the treatment period shown in Table 4, 6 µg of the human monoclonal antibody #129 or a control human IgG (#12000C; manufactured by Thermo Fisher Scientific) per 30 g of body weight was administered into the caudal vein of four types of FTLD-ALS model mice and the background mouse (C57BL/6J) thereof (Figure 3). The timing of initiating the antibody administration for each FTLD-ALS model mouse was the month of age when development of the disease was confirmed by a Morris water maze test.

**[Table 4]**

| | VCP^{T262A}-KI | PGRN^{R504X}-KI | CHMP2B^{Q165X}-KI | TDP43^{N267S}-KI |
|---|---|---|---|---|
| Duration of treatment with antibody | 6 to 10 months of age | 3 to 7 months of age | 1 to 6 months of age | 6 to 10 months of age |
| Duration of Morris water maze test | 1 to 5 days from 10 months of age | 1 to 5 days from 7 months of age | 1 to 5 days from 6 months of age | 1 to 5 days from 10 months of age |
| Duration of Y-maze test | 7 to 10 months of age | 5 to 7 months of age | 4 to 6 months of age | 7 to 10 months of age |
| Date of fear conditioning test | 10 months of age | 7 months of age | 6 months of age | 10 months of age |

### [Cognitive function tests]

During each study period or on each study day shown in Table 4, three different cognitive function tests (a Morris water maze test, a Y-maze test, and a fear-conditioning test) were performed on the four types of FTLD-ALS model mice in accordance with the method described in "Hum. Mol. Genet. 24, 540-558 (2015)" or "Mol. Psychiatry 20, 459-471 (2015)." Specifically, in the Morris water maze test, mice underwent a Morris water maze test four times (60 seconds) once daily for five days, and the mean time to reach the platform (latency) (the vertical axis in Figure 3) was measured. Further, a Y-maze consisting of three identical arms whose angles therebetween are equal (manufactured by O'Hara & Co., Ltd.) was used in the Y-maze test. A mouse was placed at the end of one arm and allowed to behave freely in the maze for eight minutes, and the percentage of spontaneous alternation (expressed as "Alternation rate" on the vertical axis in Figures 4 to 7) was calculated by dividing the number of entries into a new arm different from the previous arm by the total number of movements from one arm to another arm. In the fear-conditioning test, freezing responses were measured at 24 hours after conditioning (65 dB white noise for 30 seconds + 0.4 mA food shock for 2 seconds) without receiving a foot shock in the same chamber (expressed as [total freeze percent] on the vertical axis in Figure 8).

### [Western blot method]

The cerebral cortex tissue from four types of FTLD-ALS model mice or C57BL/6J mice at various months of age was dissolved in the presence of a dissolution buffer (100 mM Tris-HCl [pH 7.5], 2% SDS) containing a protease inhibitor cocktail (#539134; manufactured by Calbiochem; dilution factor, 1:100) at 4°C for one hour. After centrifugation (12,000 *g* × 10 minutes), the supernatant was collected, an equal volume of a sample buffer (62.5 mM Tris-HCl [pH 6.8], 2% [w/v] SDS, 2.5% [v/v] 2-mercaptoethanol, 5% [v/v] glycerol, and 0.0025% [w/v] bromophenol blue) was added, then proteins in the sample were equalized using the BCA method (Pierce BCA protein assay kit; manufactured by Thermo Scientific). Then, a protein sample was isolated by SDS-PAGE, then transferred to a polyvinylidene difluoride (PVDF) membrane (Immobilon-P; manufactured by Millipore) by a semi-dry transfer method, and blocked in a TBST solution containing 5% skim milk (10 mM Tris-HCl [pH 8.0], 150 mM NaCl, 0.05% Tween 20). Using various primary antibodies (Table 5) to detect 13 types of proteins (phosphorylated PKCα [Ser319] [pSer319-PKCα], phosphorylated PKCγ [Thr655] [pThr655-PKCγ], phosphorylated PKCδ [Ser643] [pSer643-PKCδ], phosphorylated PKCε [Ser729] [pSer729-PKCε], phosphorylated tau [Ser203 (mouse)/Ser214 (human)] [pSer203-tau], PSD95, VAMP2, γH2AX, 53BP1, phosphorylated TDP-43 [Ser409/410] [pSer409/410-TDP-43], pSer46-MARCKS, phosphorylated Ku70 [Ser77/Ser78] [pSer77/78-Ku70], and β-actin), antibody reactions were allowed to occur in a TBST solution containing 0.1% skim milk or a Can Get Signal solution (manufactured by Toyobo Co., Ltd., Osaka, Japan) at 4°C for 12 hours. The membrane was washed three times with a TBST solution, and antibody reactions were allowed to occur at room temperature for one hour using secondary antibodies against various primary antibodies (Table 5). Then, the above-mentioned 13 types of proteins were detected using an ECL Prime Western blot detection reagent (RPN2232; manufactured by GE Healthcare) and a luminescence image analyzer (ImageQuant LAS 500; manufactured by GE Healthcare) (Figure 17).

**[Table 5]**

| Primary antibody | Secondary antibody (antibody against the primary antibody in the left column) |
|---|---|
| Rabbit anti-phosphorylated PKCα (Ser319) antibody (AP0560; manufactured by NeoScientific; dilution factor, 1:10,000) | HRP-binding anti-rabbit IgG (NA934; manufactured by GE Healthcare; dilution factor, 1:3,000) |
| Rabbit anti-phosphorylated PKCy (Thr655) antibody (ab5796; manufactured by Abcam; dilution factor, 1:10,000) | HRP-binding anti-rabbit IgG (NA934; manufactured by GE Healthcare; dilution factor, 1:3,000) |
| Rabbit anti-phosphorylated PKCδ (Ser643) antibody (#9376; manufactured by Cell Signaling Technology; dilution factor, 1:3,000) | HRP-binding anti-rabbit IgG (NA934; manufactured by GE Healthcare; dilution factor, 1:3,000) |
| Rabbit anti-phosphorylated PKCε (Ser729) antibody (ab63387; manufactured by Abcam; dilution factor, 1:3,000) | HRP-binding anti-rabbit IgG (NA934; manufactured by GE Healthcare; dilution factor, 1:3,000) |
| Rabbit anti-phosphorylated tau (Ser203/Ser214), (ab170892; manufactured by Abcam; dilution factor, 1:10,000) | HRP-binding anti-rabbit IgG (NA934; manufactured by GE Healthcare; dilution factor, 1:3000) |
| Rabbit anti-PSD95 antibody (D74D3; manufactured by Cell Signaling Technology; dilution factor, 1:5,000) | HRP-binding anti-rabbit IgG (NA934; manufactured by GE Healthcare; dilution factor, 1:3,000) |
| Mouse anti-VAMP2 antibody (GTX634812; manufactured by GeneTex; dilution factor, 1:5,000) | HRP-binding anti-mouse IgG (NA931; manufactured by GE Healthcare; dilution factor, 1:3,000) |
| Mouse anti-yH2AX antibody (Ser139; #05-636; manufactured by Millipore; dilution factor, 1:2,000) | HRP-binding anti-mouse IgG (NA931; manufactured by GE Healthcare; dilution factor, 1:3,000) |
| Rabbit anti-53BP1 antibody (NB100-304; manufactured by Novus Biologicals; dilution factor, 1:50,000) | HRP-binding anti-rabbit IgG (NA934; manufactured by GE Healthcare; dilution factor, 1:3,000) |
| Rabbit anti-phosphorylated TDP43 (Ser409/410) antibody (TIP-PTD-P02; manufactured by Cosmo Bio; dilution factor, 1:6,000) | HRP-binding anti-rabbit IgG (NA934; manufactured by GE Healthcare; dilution factor, 1:3,000) |
| Rabbit anti-pSer46-MARCKS (manufactured by GL Biochem; dilution factor, 1:100,000) | HRP-binding anti-rabbit IgG (NA934; manufactured by GE Healthcare; dilution factor, 1:3,000) |
| Rabbit anti-phosphorylated Ku70 (Ser77/Ser78) antibody (ordered from Cosmo Bio; dilution factor, 1:100,000) | HRP-binding anti-rabbit IgG (NA934; manufactured by GE Healthcare; dilution factor, 1:3,000) |
| Mouse anti-β-actin antibody (manufactured by Santa Cruz Biotechnology; dilution factor, 1:3,000) | HRP-binding anti-mouse IgG (NA931; manufactured by GE Healthcare; dilution factor, 1:3,000) |

### 2. Results

### [Analysis of antibody affinity using surface plasmon resonance (SPR) method]

While the KD value of the mouse monoclonal antibody 2C8C was 1.95 × 10⁻⁹ M, the KD value of the human monoclonal antibody #129 of the present invention was 3.79 × 10⁻¹¹ M. Given that the KD values of common antibody drugs, as well as the KD value of the mouse monoclonal antibody 2C8C, are within the range of 10⁻⁹ M to 10^{-1C} M, the human monoclonal antibody #129 of the present invention is shown to be an antibody with very high affinity for human HMGB1.

### [Immunohistochemical staining method]

pSer46-MARCKS occurs from an early stage before onset of Alzheimer's disease, and HMGB1 leaking out of a cell owing to necrosis of a neuron induces phosphorylation of MARCKS, inducing degeneration of a neurite ("SCIENTIFIC REPORT, 2016 Aug 25; 6:31895"). Accordingly, whether phosphorylation of MARCKS and necrosis of neurons also occurred at an early stage of development of FTLD was analyzed using four types of FTLD-ALS model mice one to 12 months of age.

In the results, cells showing a high level of a pSer46-MARCKS-derived signal and an attenuated genomic DNA-derived DAPI signal were observed in cerebral cortex in the four types of FTLD-ALS model mice (VCP^{T262A}-KI mice, PGRN^{R504X}-KI mice, CHMP2B^{Q165X}-KI mice, and TDP-43^{N267S}-KI mice) at one month, three months, six months, and 12 months of age (Figure 2-1). Further, expansion of the endoplasmic reticulum (ER), which is characteristic to necrosis, was observed in the cerebral cortex in two types of FTLD-ALS model mice (VCP^{T262A}-KI mice and PGRN^{R504X}-KI mice) at one month of age (Figure 2-2).

These results indicate that necrosis occurs from an early stage of development of FTLD-ALS.

### [Treatment effect of human monoclonal antibody #129 on symptoms of FTLD-ALS model mice: cognitive function tests]

Subsequently, whether FTLD-ALS symptoms improved when the human monoclonal antibody #129 of the present invention was administered to four types of FTLD-ALS model mice was analyzed using three types of cognitive function tests (a Morris water maze test, a Y-maze test, and a fear-conditioning test). First, the Morris water maze test demonstrated that the time to reach the platform was prolonged as the four types of FTLD-ALS model mice aged by month ("KI n.t." in Figure 3), as compared with C57BL/6J mice ("B6 n.t." in Figure 3), which are normal mice (Figure 3). In contrast, it was shown that, when the human monoclonal antibody #129 of the present invention was administered to the four types of FTLD-ALS model mice ("KI Ab" in Figure 3), the prolongation of time to reach the platform was significantly suppressed (the time was shortened) in all the FTLD-ALS model mice from an early stage of development of FTLD regardless of types of FTLD mutant genes to the same extent as in the C57BL/6J mice, whereas, when a control human IgG was administered to the four types of FTLD-ALS model mice ("KI IgG" in Figure 3), no change was observed in the prolongation of time to reach the platform (Figure 3).

These results indicate that the human monoclonal antibody #129 of the present invention or an anti-HMGB1 antibody having the CDRs common thereto has an effect of preventing and improving the decrease in cognitive function caused by FTLD-ALS. Effects similar to these results were also confirmed when a Y-maze test (Figures 4 to 7) and a fear-conditioning test (Figure 8) were performed.

### [Treatment effect of human monoclonal antibody #129 on symptoms in FTLD-ALS model mice: immunohistochemical staining method]

Subsequently, the effect of the human monoclonal antibody #129 of the present invention of improving cognitive function in FTLD-ALS was analyzed using an immunohistochemical staining method. First, in the four types of FTLD-ALS model mice ("KI n.t." in Figure 3), as compared with the C57BL/6J mice ("B6 n.t." in Figure 3), which are normal mice, the proportion of necrosis marker (pSer46-MARCKS)-positive cells in the cerebral cortex increased (Figure 9), and the ER marker (KDEL)-derived signaling region expanded (Figure 10). In contrast, it was shown that, when the human monoclonal antibody #129 of the present invention was administered to the four types of FTLD-ALS model mice ("KI **Ab"** in Figures 9 and 10), the increases in the proportion of pSer46-MARCKS-positive cells and the expansion of the KDEL-derived signaling region were significantly suppressed to the same extent as in the C57BL/6J mice, whereas, when a control human IgG was administered to the four types of FTLD-ALS model mice ("KI IgG" in Figures 9 and 10), no changes were observed in the increase in the proportion of the pSer46-MARCKS-positive cells or the expansion of the KDEL-derived signaling region (Figures 9 and 10).

These results indicate that the human monoclonal antibody #129 of the present invention and the anti-HMGB1 antibody having the CDRs common thereto have an effect of preventing and improving necrosis and ER expansion in the cerebral cortex caused by FTLD-ALS.

Further, in the four types of FTLD-ALS model mice, as compared with the C57BL/6J mice, the DNA damage marker (γH2AX and 53BP1)-derived signaling region in the cerebral cortex expanded. However, it was shown that, when the human monoclonal antibody #129 of the present invention was administered, the expansion of the γH2AX and 53BP1-derived signaling region was significantly suppressed to the same extent as in the C57BL/6J mice (Figures 11 and 12).

These results indicate that the human monoclonal antibody #129 of the present invention and the anti-HMGB1 antibody having the CDRs common thereto have an effect of preventing and improving DNA damage in the cerebral cortex caused by FTLD-ALS.

Further, in the four types of FTLD-ALS model mice, as compared with the C57BL/6J mice, the protein agglutination marker (pSer409/410-TDP-43 and p62)-derived signaling region expanded in the cerebral cortex. However, it was shown that, when the human monoclonal antibody #129 of the present invention was administered, the expansion of the pSer409/410-TDP-43 and p62-derived signaling region was significantly suppressed (Figures 13 and 14).

These results indicate that the human monoclonal antibody #129 of the present invention and the anti-HMGB1 antibody having the CDRs common thereto have an effect of preventing and improving protein agglutination in the cerebral cortex caused by FTLD-ALS.

Further, the present inventors investigated synapse damage by analysis of a phosphorylated proteome using colocalization of VAMP2 and PSD95 and colocalization of pSer203-tau and PSD95 as indicators. Thus, the results of the analysis of synapse damage using these colocalizations as indicators showed that colocalization of VAMP2 and PSD95 in the cerebral cortex had decreased in the four types of FTLD-ALS model mice, as compared with the C57BL/6J mice, but the decrease was significantly suppressed when the human monoclonal antibody #129 of the present invention was administered (Figure 15). Further, colocalization of pSer203-tau and PSD95 increased in the cerebral cortex of the four types of FTLD-ALS model mice as compared with the C57BL/6J mice. However, it was shown that, when the human monoclonal antibody #129 of the present invention was administered, the increase was significantly suppressed (Figure 16).

These results indicate that the human monoclonal antibody #129 of the present invention and the anti-HMGB1 antibody having the CDRs common thereto have an effect of preventing and improving synapse damage in the cerebral cortex caused by FTLD-ALS.

### [Treatment effect of human monoclonal antibody #129 on symptoms of FTLD-ALS model mice: Western blot method]

To support the results of the analysis using the immunohistochemical staining method, an analysis using Western blot method was performed. First, when the phosphorylation levels of four types of proteins associated with an AD-FTLD core signal (pSer319-PKCα, pThr655-PKCy, pSer643-PKCδ, and pSer729-PKCε) were analyzed, the results showed that the phosphorylation levels of two types of proteins (pSer319-PKCα and pSer643-PKCδ) predicted by a dynamic network analysis with a strict threshold for core node selection (centricity > 2SD) increased in the four types of FTLD-ALS model mice as compared with the C57BL/6J mice. However, when the human monoclonal antibody #129 of the present invention was administered to the four types of FTLD-ALS model mice, the increases in the pSer319-PKCα and pSer643-PKCδ levels were suppressed (Lanes (1) and (3) in Figure 17). In contrast, no difference was observed in the phosphorylation levels of two types of proteins (pThr655-PKCγ and pSer729-PKCε) predicted by a dynamic network analysis under a relatively loose condition for a core node selection (centricity > 0) between the above-mentioned four types of FTLD-ALS model mice and the C57BL/6J mice (Lanes (2) and (4) in Figure 17).

Further, the phosphorylation level of the synapse instability-associated protein (pSer203-tau) increased in the four types of FTLD-ALS model mice as compared with the C57BL/6J mice. However, it was shown that, when the human monoclonal antibody #129 of the present invention was administered to the four types of FTLD-ALS model mice, the increase in the pSer203-tau level was suppressed (Lane (5) in Figure 17). Further, the expression levels of synapse-associated proteins (PSD95 and VAMP2) decreased in the four types of FTLD-ALS model mice as compared with the C57BL/6J mice. However, it was shown that, when the human monoclonal antibody #129 of the present invention was administered to the four types of FTLD-ALS model mice, the decreases in the expression levels of PSD95 and VAMP2 were suppressed (Lanes (6) and (7) in Figure 17).

Further, the expression levels of DNA damage-associated proteins (γH2AX and 53BP1) increased in the four types of FTLD-ALS model mice as compared with the C57BL/6J mice. However, it was shown that, when the human monoclonal antibody #129 of the present invention was administered to the four types of FTLD-ALS model mice, the increases in the expression levels of γH2AX and 53BP1 were suppressed (Lanes (8) and (9) in Figure 17).

Further, the phosphorylation level of the cell death-associated protein (pSer46-MARCKS) increased in the four types of FTLD-ALS model mice as compared with the C57BL/6J mice. However, it was shown that, when the human monoclonal antibody #129 of the present invention was administered to the four types of FTLD-ALS model mice, the increase in the pSer46-MARCKS level was suppressed (Lane (10) in Figure 17).

Further, the phosphorylation level of the DNA repair-associated protein (pSer77/78-Ku70) increased in the four types of FTLD-ALS model mice as compared with the C57BL/6J mice. However, it was shown that, when the human monoclonal antibody #129 of the present invention was administered to the four types of FTLD-ALS model mice, the increase in the pSer77/78-Ku70 level was suppressed (Lane (11) in Figure 17).

Further, the protein agglutination marker level (pSer409/410-TDP-43) increased in the four types of FTLD-ALS model mice as compared with the C57BL/6J mice. However, it was shown that, when the human monoclonal antibody #129 of the present invention was administered to the four types of FTLD-ALS model mice, the increase in the pSer409/410-TDP-43 level was suppressed (Lane (12) in Figure 17).

The above results indicate that the human monoclonal antibody #129 of the present invention and the anti-HMGB1 antibody having the CDRs common thereto have an effect of preventing and improving secondary neuron damage and neurite damage induced by extracellular HMGB1 released from necrotized neurons by FTLD-ALS.

### [Adverse reactions of the human monoclonal antibody #129 of the present invention]

Further, adverse reactions of the human monoclonal antibody #129 of the present invention were investigated. After the cognitive function tests shown in Figures 3 to 8, the human monoclonal antibody #129 of the present invention was administered to the four types of FTLD-ALS model mice and the C57BL/6J mice, then the lung, liver, kidney, intestine, spleen, skeletal muscle, heart, and skin were removed therefrom by incision, and a pathological examination was performed.

The results showed no abnormal findings (for example, obvious inflammation, cell death, tumor) due to administration of the human monoclonal antibody #129 of the present invention in any tissue. The results indicate that the human monoclonal antibody #129 of the present invention and the anti-HMGB1 antibody having the CDRs common thereto can prevent and improve symptoms in FTLD-ALS model mice without causing noticeable adverse reactions.

### Example 2

### 1. Materials and methods

### [AD model mice]

5 × FAD mice, that is, transgenic mice that have overexpressed mutant human APP (770) having Swedish (KM670/671NL) mutation, Florida (I716V) mutation, and London (V717I) familial Alzheimer's disease (FAD) mutation, and human PS1 having two FAD mutations (M146L and L285V) were purchased from The Jackson Laboratory. Genes that encode the above-mentioned APP and PS1 in 5 × FAD mice are under the control of a mouse Thy1 promoter ("J Neurosci 26, 10129-10140 (2006)"). The background of 5 × FAD mice was C57BL/SJL, which had been created by mating C57BL/6J females and SJL/J males. Comprehensive proteomic analysis was performed as mentioned earlier, by using brain tissue samples from male 5 × FAD mice at 1, 3, 6, and 12 months of age ("Hum Mol Genet 24, 540-558 (2015)").

APP-KI (App^{NL-G-F/NL-G-F}) mice ("Nat. Neurosci. 17, 661-663 (2014)"), that is, mice having Swedish (KM670/671NL) mutation, Beyreuther/Iberian (I716F) mutation, and Arctic (E693G) mutation of humanized APP mouse genes were obtained from Riken BioResource Research Center.

### [Administration of human monoclonal antibody #129 of the present invention]

Into the back and neck region of 5 × FAD mice or B6/SJL mice, 0.1 µg/kg of a control IgG (human IgG isotype control, #12000C; manufactured by Thermo Fisher Scientific, Inc.) or the human monoclonal antibody #129 of the present invention was subcutaneously administered once a week from 6 months to 8 months of age (hereinafter simply referred to as "subcutaneously administered") (9 times in total). When the human monoclonal antibody #129 of the present invention was intravenously injected into each mouse, the human monoclonal antibody #129 of the present invention was diluted with physiological saline to a final volume of 50 µl and administered once a month into the caudal vein from 6 months to 8 months of age after birth (3 times in total).

### [Immunohistochemical staining method]

In order to perform immunohistochemical staining, mouse and human brain tissue samples were immobilized in 4% paraformaldehyde and paraffin-embedded. Sagittal sections or coronal sections with a thickness of 5 µm were obtained using a microtome (manufactured by Yamato Kohki Industrial **Co.,** Ltd.). The immunohistochemical staining was performed using the following primary antibodies: rabbit anti-phosphorylated Ser46-MARCKS antibody (1:2000 [manufactured by GL Biochem (Shanghai) Ltd.]); mouse anti-Aβ antibody (1:1000; clone 82E1; #10323; manufactured by IBL **Co.,** Ltd.); mouse anti-MAP2 antibody (1:200; sc-32791; manufactured by Santa Cruz Biotechnology, Inc.); rabbit anti-MAP2 antibody (1:2000; ab32454; manufactured by Abcam Limited); mouse anti-phosphorylated H2X (γH2X) antibody (1:300; JBW301; manufactured by Millipore); rabbit anti-53BP1 antibody (1:5000; NB100-304; manufactured by Novus Biologicals, LLC); rabbit anti-YAP antibody (1:50; GTX129151; manufactured by GeneTex, Inc.); rabbit anti-NFκB antibody (1:100; #8242; manufactured by Cell Signaling Technology, Inc.); mouse anti-IL6 antibody (1:50; ab208113; manufactured by Abcam Limited); rabbit anti-CCL2/MCP1 antibody (1:100; NBP1-07035; manufactured by Novus Biologicals, LLC); rabbit anti-Lamin B1 antibody (1:5000; ab16048; manufactured by Abcam Limited); goat anti-Iba1 antibody (1:500; #011-27991; manufactured by FUJIFILM Wako Pure Chemical Corporation), rabbit anti-Iba1 antibody (1:100; #019-19741; manufactured by FUJIFILM Wako Pure Chemical Corporation); mouse anti-GFAP antibody Cy3-conjugated (1:5000; #C9205; manufactured by Sigma Aldrich); mouse anti-NeuN antibody (1:1000; ab104224; manufactured by Abcam Limited); and the reaction products were visualized with Alexa Fluor 488-, 568-, 647-bound secondary antibodies (1:1000; manufactured by Molecular Probes, Inc.). Cell nuclei were stained with DAPI (0.2 µg/mL in PBS; #D523; DOJINDO LABORATORIES). All microscopic images were obtained using a confocal microscope (FV1200IX83; manufactured by Olympus Corporation).

### [Western blot method]

The entire cerebral cortex in various mice was subjected to Western blotting to detect ten types of proteins (yH2AX, 53BP1, γH2AX, 53BP1, pSer77/78-Ku70, β-actin, pThr638-PKCα, pThr641-PKCβI, pSer660-PKCβII/δ, and pan-PKC) using the antibodies in Tables 5 and 6, according to the method described in the above-described section of [Western blot method] in Example 1.

**[Table 6]**

| Primary antibody | Secondary antibody (antibody against the primary antibody in the left column) |
|---|---|
| Rabbit anti-phosphorylated PKCα (Thr638) antibody (ab32502; manufactured by Abcam; dilution factor, 1:20,000) | HRP-binding anti-rabbit IgG (NA934; manufactured by GE Healthcare; dilution factor, 1:3,000) |
| Rabbit anti-phosphorylated PKCβI (Thr641) antibody (sc-101776; manufactured by Santa Cruz Biotechnology; dilution factor, 1:1,000) | HRP-binding anti-rabbit IgG (NA934; manufactured by GE Healthcare; dilution factor, 1:3,000) |
| Goat anti-phosphorylated PKCβII/δ(Thr660) antibody (sc-11760; manufactured by Santa Cruz Biotechnology; dilution factor, 1:10,000) | HRP-binding anti-goat IgG (sc-2020; manufactured by Santa Cruz Biotechnology; dilution factor, 1:3,000) |
| Rabbit anti-pan-PKC antibody (GTX52352; manufactured by GeneTex; dilution factor, 1:1,500) | HRP-binding anti-rabbit IgG (NA934; manufactured by GE Healthcare; dilution factor, 1:3,000) |

### [Time-lapse Ku70 imaging]

Laser micro-irradiation and signal acquisition from damage sites were performed according to a known method ("Nat Commun 4, 1816 (2013)"; "Nat Cell Biol 5, 255-260 (2003)"). U2OS cells were seeded in a 35 mm glass bottom dish (D11140H, Matsunami Glass Ind., Ltd.) containing 2 mL culture solution (DMEM; D5796; Sigma Aldrich, St. Louis, MO, USA), and transfected with 2.5 g EGFP-C1-FLAG-Ku70 plasmid (#46957; Addgene, Inc., Watertown, MA, USA) using Lipofectamine LTX (Thermo Fisher Scientific, Inc. Waltham, MA, USA) next day. At 48 hours after the transfection and 3 hours before laser micro-irradiation and time-lapse imaging, ds-HMGB1 (6 ng/mL, 0.24 nM) and/or the human monoclonal antibody #129 of the present invention (6 ng/mL, 0.04 nM) were added to the medium. U2OS cells were treated with 2 µm Hoechst 33258 (DOJINDO LABORATORIES, Kumamoto, Japan) for 20 minutes and sensitized to DSB. A rectangular region on the cell nucleus was irradiated with laser from a 405 nm laser diode (40 × objective lens, 95%, 100 scans, 12.5 units/pixel), using a confocal microscope (FV1200IX83; Olympus Corporation, Tokyo, Japan) under live cell imaging conditions (37°C, 5% CO₂) maintained with Stage Top Incubator (Tokai Hit., Co., Ltd., Shizuoka, Japan). Time-lapse images were obtained every 30 seconds for 10 minutes.

### [Y-maze test]

In Figures 22d and 22e, a Y-maze test was performed according to the above-described section of [Cognitive function tests] in Example 1

### 2. Results

### [Blocking of HMGB1 signal allows repair function of DNA damage in nerve cells to be recovered]

The results of immunohistochemical staining and Western blot analyses of the expression levels of γH2AX and 53BP1 in the cerebral cortex showed that the expression level of γH2AX and 53BP1 in the 5 × FAD mice to which the control IgG was subcutaneously administered significantly increased as compared with the B6/SJL mice to which the human monoclonal antibody #129 of the present invention or the control IgG was subcutaneously administered (Figures 18a and b). Meanwhile, the expression level of γH2AX and 53BP1 in the 5 × FAD mice to which the human monoclonal antibody #129 of the present invention was subcutaneously administered significantly decreased as compared with the 5 × FAD mice to which the control IgG was subcutaneously administered (Figures 18a and b). Further, in the case where the human monoclonal antibody #129 of the present invention was intravenously administered, DNA damage in the cerebral cortical neurons of 5 × FAD mice was similarly significantly suppressed (Figures 18c and d).

These results indicate that administering to the 5 × FAD mice the human monoclonal antibody #129 of the present invention or the anti-HMGB1 antibody having the CDRs common thereto can suppress DNA damage in the cerebral cortical neurons. Meanwhile, since DNA damage in the MAP2-positive neuron in the parietal cortex of a human AD patient increased (Figure 18e), it can be fully expected that DNA damage in the cerebral cortical neurons can be similarly suppressed, when the human monoclonal antibody #129 of the present invention or the anti-HMGB1 antibody having the CDRs common thereto was administered to human AD patients.

Ku70 is known to assemble at DSB sites through nonhomologous end-joining (NHEJ) pathway and to be involved in DNA damage repair. Thus, time-lapse imaging analysis of EGFP-Ku70 expressing cells after laser micro-irradiation was performed to examine how trigger signals from extracellular HMGB1 affect Ku70 assembling at DSB sites, in human U2OS cells that endogenously express TLR4 and RAGE receptors. First, in order to select the HMGB1 concentration used for U2OS cell culture, the HMGB1 concentrations in the cerebrospinal fluid (CSF) of human AD patients (n = 56) were assessed by the ELISA method. The results indicate that the HMGB1 concentrations in CSF were 11 pg/mL to 13.7 ng/mL (mean = 936 pg/mL). Consequently, in this in vitro experiment, 6 ng/mL (0.24 nM) ds-HMGB1 was used as a model for HMGB1 concentration in the nerve papilla space after nerve cell injury.

Under these conditions, when adding ds-HMGB1 to the medium, the accumulation of EGFP-Ku70 at the DNA damage sites of U2OS cells significantly delayed (Figures 18f and g). What is worthy of note is that, after laser micro-irradiation, the reservoir region of Ku70 was depleted (Figure 18f, arrows), whereas the addition of ds-HMGB1 inhibited the recruitment of Ku70 from the reservoir region of Ku70 (Figure 18f). Meanwhile, when adding the human monoclonal antibody #129 of the present invention together with ds-HMGB1, the localization of EGFP-Ku70 to the DNA damage sites was almost completely restored (Figures 18f and g) .

These results, taken together, indicate that the administered human monoclonal antibody #129 of the present invention blocks the HMGB1 signals in 5 × FAD mice, and thereby allowing Ku70 to assemble at the DNA damage sites in nerve cells, as well as allowing the repair function of DNA damage in nerve cells to be recovered, and the amplification of TRIAD necrosis to be inhibited.

### [TRIAD is cell death phenotype of senescence]

Senescent glial cells and nerve cells have been identified in neurodegenerative and aging brains ("Aging Cell 17, (2018)"; "J. Clin. Invest. 128, 1208-1216 (2018)"; "Nat. Neurosci. 22, 719-728 (2019)"; "Nat. Neurosci. 22, 683-684 (2019)"; "Front. Cell. Neurosci. 14, 1-14 (2020)"; and "Nat. Rev. Neurosci. 21, 433-444 (2020) ") . Cellular senescence shares multiple features with TRIAD necrosis, such as DNA damage, SASP/DAMP secretion (especially secretion of HMGB1), transcriptional change, apoptosis resistance ("Cell 179, 813-827 (2019)") ("Nat. Commun. 11, 1-22 (2020)"; "Sci Rep 6, 31895 (2016)"; "J. Cell Biol. 172, 589-604 (2006)"; "Hum Mol Genet 25, 4749-4770 (2016)"; and "Cell Death Dis 7, e2207 (2016)").

Therefore, the identity between TRIAD and cellular senescence was verified. A nuclear membrane protein Lamin B1, as a senescence marker, was localized in a ring shape in nerve cells, astrocytes, and microglia of normal mice (C57BL/6J mice) (Figure 19a, arrows), whereas the localization of Lamin B1 in a ring shape became unclear or disappeared in a part of nerve cells and astrocytes of two types of AD model mice (5 × FAD mice and APP-KI mice) (Figure 19a, arrows). Further, although abnormal morphology of Lamin B1 ring was observed in the microglia of the two types of AD model mice, the Lamin B1 ring by itself mostly remained (Figure 19a, arrows).

An immunohistochemical staining analysis of the cerebral cortex in 5 × FAD mice, using three types of antibodies (anti-YAP antibody, anti-pSer46-MARCKS antibody, and anti-Lamin B1 antibody) revealed that senescent cells negative for Lamin B1 ring were likely to be negative for nuclear YAP, and thereby phenotypes of TRIAD and cellular senescence were overlapped (Figure 20a, narrow white solid arrows). TRIAD-senescent cells surrounded larger pSer46-MARCKS-positive foci of primary TRIAD necrosis (Figure 20a, bold white arrows). Meanwhile, the nuclear YAP-positive cells maintained the Lamin B1 ring (Figure 20a, arrows).

Further, the intravenous administration of the human monoclonal antibody #129 of the present invention to two types of AD model mice (5 × FAD mice and APP-KI mice) significantly reduced, in all mice, the number of senescent cells in the cerebral cortex (Figure 20b), as well as the number of senescent cells in the neurons, astrocytes and microglia (Figure 19b) as compared with the case of intravenous administration of the control IgG to the above-mentioned two types of AD model mice.

These results indicate that the human monoclonal antibody #129 of the present invention and the anti-HMGB1 antibody having common CDRs thereto have an effect of treating aging-related degenerative or neurological diseases, brain aging, and diseases associated with brain aging.

Further, in order to examine whether the human monoclonal antibody #129 of the present invention can treat cellular senescence-related inflammation around glial cells, analysis was performed using a transcription factor (NFκB) that regulates the inflammation and/or inflammatory cytokines (MCP1 and IL6) as an index. The nuclear translocation level of NFκB in microglia (detected with the anti-Iba1 antibody) of the two types of AD model mice (5 × FAD mice and APP-KI mice) significantly increased, as compared with the normal mice (B6/SJL mice or C57BL/6 mice) (Figure 21a). However, when the human monoclonal antibody #129 of the present invention was administered to the AD mice, the nuclear translocation level of NFκB in microglia significantly decreased (Figure 21a). Further, the MCP1 and IL6 expression levels around the astrocytes (detected with anti-GFAP antibody) and the microglia in the above-mentioned two types of AD model mice increased compared with the normal mice (B6/SJL mice or C57BL/6 mice) (Figure 21b). However, when the human monoclonal antibody #129 of the present invention was administered to the AD mice, the MCP1 and IL6 expression levels around the astrocytes and the microglia significantly decreased (Figure 21b).

These results indicate that the human monoclonal antibody #129 of the present invention and the anti-HMGB1 antibody having common CDRs thereto can treat inflammation around cellular senescence-related glial cells.

### [Blocking of HMGB1 signal suppresses DNA damage and Aβ pathology progression]

In order to verify in vivo whether the repair function of DNA damage in nerve cells is restored when inhibiting the activity of PKCα, a protein kinase of Ku70, PBS (control) or a PKCα inhibitor (Go6976) was subcutaneously administered to B6/SJL mice or 5 × FAD mice at 6 weeks (1.5 months) of age (left diagram of Figure 22a), and the expression levels of γH2AX and 53BP1 in the cerebral cortex were analyzed by immunohistochemical staining and Western blotting, as well as a cognitive function test (Y-maze test) was used to analyze whether the AD symptoms were improved.

The results of immunohistochemical staining and Western blotting revealed that the levels of three types of phosphorylation (pThr638-PKCα, pThr641-PKCβI, and pSer660-PKCβII/δ) as indicators of the activation of PKCα increased and the level of Ku70 phosphorylation (pSer77/78-Ku70) increased in 5 × FAD mice to which PBS was subcutaneously administered compared with B6/SJL mice to which Go6976 or PBS was subcutaneously administered, and the expression levels of γH2AX and 53BP1 significantly increased (Figures 22b and c). Meanwhile, 5 × FAD mice to which Go6976 was subcutaneously administered had a decreased level of the above-mentioned PKCα phosphorylation and a decreased level of the above-mentioned Ku70 phosphorylation compared with 5 × FAD mice to which PBS was subcutaneously administered, and the expression levels of γH2AX and 53BP1 significantly decreased (Figures 22b and c).

Further, the results of a Y-maze test revealed that the cognitive function of 5 × FAD mice to which PBS was subcutaneously administered significantly decreased compared with B6/SJL mice to which Go6976 or PBS was subcutaneously administered (Figure 22d). Meanwhile, 5 × FAD mice to which Go6976 was subcutaneously administered had a significantly improved cognitive function compared with 5 × FAD mice to which PBS was subcutaneously administered (Figure 22d).

These results indicate that inhibiting the activity of PKCα, a protein kinase of Ku70, in the 5 × FAD mice suppresses DNA damage in the cerebral cortical neurons, resulting in an improved cognitive function.

Subsequently, in order to verify whether the cognitive function of 5 × FAD mice improved when the human monoclonal antibody #129 of the present invention in place of the PKCα inhibitor (Go6976) was administered, the control IgG or the human monoclonal antibody #129 of the present invention was subcutaneously administered to B6/SJL mice or 5 × FAD mice at 6 months of age (center diagram of Figure 22a) to analyze using a cognitive function test (Y-maze test).

The results revealed that the cognitive function of 5 × FAD mice to which the control IgG was subcutaneously administered significantly decreased compared with B6/SJL mice to which the control IgG was subcutaneously administered (Figure 22e). Meanwhile, the cognitive function of the 5 × FAD mice to which the human monoclonal antibody #129 of the present invention was subcutaneously administered significantly improved as compared with the 5 × FAD mice to which the control IgG was subcutaneously administered (Figure 22e).

These results indicate that administering to the 5 × FAD mice the human monoclonal antibody #129 of the present invention or the anti-HMGB1 antibody having the CDRs common thereto suppresses DNA damage in the cerebral cortical neurons, resulting in an improved cognitive function.

The present inventors have recently discovered that YAP-dependent primary necrosis of the cerebral cortical nerve cells at an ultra-early stage of AD ("Nat. Commun. 11, 1-22 (2020)") is identical to TRIAD ("J. Cell Biol. 172, 589-604 (2006)"), and then, in neighboring nerve cells, secondary necrosis with unknown characteristics and associated with extracellular Aβ aggregates as ghost of cell death arises ("Nat. Commun. 11, 1-22 (2020)"). Therefore, in order to verify how the PKCα inhibitor (Go6976) and the human monoclonal antibody #129 of the present invention affect the two types of necrosis and extracellular Aβ aggregates in 5 × FAD mice, PBS or PKCα inhibitor (Go6976) was subcutaneously administered to B6/SJL mice or 5 × FAD mice at 6 weeks (1.5 months) of age (left diagram of Figure 22a), as well as the control IgG or the human monoclonal antibody #129 of the present invention was subcutaneously administered (center diagram of Figure 22a) or intravenously administered (right diagram of Figure 22a) to B6/SJL mice or 5 × FAD mice at 6 months of age to analyze by immunohistochemical staining using anti-pSer46-MARCKS antibody and anti-Aβ antibody

As a result, when either Go6976 or the human monoclonal antibody #129 of the present invention was administered to 5 × FAD mice, secondary necrosis, a cluster of necrotic nerve cell surrounding the central deposition of Aβ, and extracellular Aβ aggregates was significantly reduced as compared with the administration of control PBS or control IgG (Figure 22f).

These results indicate that the human monoclonal antibody #129 of the present invention and the anti-HMGB1 antibody having the CDRs common thereto have an effect of preventing necrosis and also inhibiting reciprocal amplification of TRIAD necrosis that is based on HMGB1-induced DNA damage.

### [Discussion]

Patent Document 3 indicates that the human monoclonal antibody #129 of the present invention, which is an anti-ds HMGB1 human monoclonal antibody, has an effect of inhibiting necrosis associated with decreased YAP in neurons (TRIAD). Further, Example 1 described above indicates that the human monoclonal antibody #129 of the present invention has an effect of preventing and improving neurite damage associated with MARCKS phosphorylation. Examples of pathological condition underlying these treatment effects include the amplification (or propagation) of TRIAD necrosis. When neurons undergo TRIAD necrosis, the neurons release HMGB1 including dsHMGB1. It was revealed that dsHMGB1 activates a PKC-ERK system through binding with TLR4 and RAGE and phosphorylates MARCKS ("2016 Aug 25;6:31895. doi:10.1038/srep31895"), and also revealed this time that the activated PKC phosphorylated Ku70 to decrease the repair function of DNA damage (Figure 18 of Example 2). Further, it was revealed this time that the human monoclonal antibody #129 of the present invention suppresses TRIAD necrosis in nerve cells and also inhibits reciprocal amplification of TRIAD necrosis that is based on HMGB1-induced DNA damage (Figures 18 and 22 of Example 2).

The present Example 1 indicated that the human monoclonal antibody #129 of the present invention and the anti-HMGB1 antibody having the CDRs common thereto exhibit a treatment effect on FTLD-ALS model mice. The VCP^{T262A} mutation, PGRN^{R504X} mutation, CHMP2B^{Q165}X mutation, and TDP43^{N267S} mutation that the FTLD-ALS model mice carry are known to cause not only FTLD but also ALS. Also, FTLD and ALS are understood to be the same disease spectrum in this field (for example, Patent Documents 1 to 13 below). Further, a large number of patients of neurodegenerative disease including FTLD and ALS (sporadic case) are a heterogenous population that does not have a single causative genetic mutation (or have a plurality of risk genes), and the efficacy of existing therapeutic agents differs depending on each patient, which are problematic. The human monoclonal antibody on HMGB1 in the present invention shows equal effects on pathological conditions that may arise due to various causes as shown in the present Example, and hence, it can be developed as a therapeutic agent for more patients with not only familial neurodegenerative disease but also sporadic neurodegenerative disease.
Document 1: Janssens J and Broeckhoven CV, Pathological mechanisms underlying TDP-43 driven neurodegeneration in FTLD-ALS spectrum disorders, Human Molecular Genetics, Volume 22, Issue R1, 15 October 2013, Pages R77-R87;
Document 2: Schymick JC, Yang Y, Andersen PM, et al. Progranulin mutations and amyotrophic lateral sclerosis or amyotrophic lateral sclerosis-frontotemporal dementia phenotypes. J Neurol Neurosurg Psychiatry. 2007; 78(7):754-756.doi:10.1136/jnnp.2006.109553;
Document 3: Sleegers K et al, Progranulin genetic variability contributes to amyotrophic lateral sclerosis. Neurology 2008, 71 (4) 253-259; DOI: 10.1212/01.wnl.0000289191.54852.75;
Document 4: Scarian E et al, The Role of VCP Mutations in the Spectrum of Amyotrophic Lateral Sclerosis-Frontotemporal Dementia. 2022 Front. Neurol. doi: 10.3389/fneur.2022.841394;
Document 5: Sreedharan J et al, TDP-43 Mutations in Familial and Sporadic Amyotrophic Lateral Sclerosis Science 2008 Vol 319, Issue 5870 pp. 1668-1672;
Document 6: Kabashi E et al, TARDBP mutations in individuals with sporadic and familial amyotrophic lateral sclerosis. Nat Genet. 2008 May; 40(5):572-4.;
Document 7: Williams K L et al, A novel TARDBP mutation in an Australian amyotrophic lateral sclerosis kindred. J Neurol Neurosurg Psychiatry. 2009 Nov; 80(11):1286-8.; Document 8: Parkinson Net al, ALS phenotypes with mutations in CHMP2B (charged multivesicular body protein 2B) Neurology 2006 Sep 26;67(6):1074-7.;
Document 9: Cox LE et al.(2010) Mutations in CHMP2B in Lower Motor Neuron Predominant Amyotrophic Lateral Sclerosis (ALS). PLoS ONE5(3): e9872. https://doi.org/10.1371/journal.pone.0009872;
Document 10: Waegaert R et al. Longitudinal transcriptomic analysis of altered pathways in a CHMP2Bintron5-based model of ALS-FTD. Neurobiol Dis. 2020 Mar; 136:104710. doi: 10.1016/j.nbd.2019.104710.Epub 2019 Dec 16.PMID: 31837425;
Document 11: Vernay A et al, A transgenic mouse expressing CHMP2B intron 5 mutant in neurons develops histological and behavioural features of amyotrophic lateral sclerosis and frontotemporal dementia. Hum Mol Genet. 2016 Aug 1; 25(15): 3341-3360. doi: 10.1093/hmg/ddw182. Epub 2016 Jun21.PMID: 27329763;
Document 12: Ugbode C, West RJH. Lessons learned from CHMP2B, implications for frontotemporal dementia and amyotrophic lateral sclerosis. Neurobiol Dis. 2021 Jan; 147:105144. doi: 10.1016/j.nbd.2020.105144. Epub 2020Nov 2.PMID: 33144171 Review. ;
Document 13: Blair IP et al. FUS mutations in amyotrophic lateral sclerosis: clinical, pathological, neurophysiological and genetic analysis. J Neurol Neurosurg Psychiatry. 2010 Jun; 81(6) :639-45. doi:10.1136/jnnp.2009.194399. Epub 2009 Dec 3.PMID: 19965854.

On the other hand, necrosis associated with decreased YAP (TRIAD) has been confirmed in Huntington's disease (HD) mice ("Mao et al, Hum Mol Genet 2016") and human HD patients ("Yamanishi et al, Acta Neuropathol Commun 2017") in addition to AD and FTLD, and in mutant SOD1 transgenic mice, which are model mice for amyotrophic lateral sclerosis (ALS), decreased motoneurons have been confirmed ("Morimoto et al, J Neurosci Res 2009"). Also, regarding the increase in phosphorylated MARCKS, which is another characteristic of TRIAD, it has been indicated that the amount of the phosphorylated MARCKS is increased in spinal fluid and/or serum of human ALS patients ("Homma et al, Life Sci Alliance 2021"). Further, enhancement of MARCKS phosphorylation has been confirmed in Parkinson's disease (PD) model mice (A53T Bac Tg mice) ("Fujita et al, eNeuro 2018").

Furthermore, the human monoclonal antibody #129 of the present invention has an effect of reducing TDP43 aggregates of cerebral cortical neurons in four types of FTLD-ALS model mice, as shown in Example 1. Therefore, the human monoclonal antibody #129 of the present invention is fully expected to have a suppressing effect on degenerative diseases such as ALS associated with TDP43 aggregation.

Moreover, the decrease in repair function of DNA damage is now recognized as a common pathological condition of degenerative diseases ("McKinnon Nature Neurosci 2013"; and "Madabhushi et al, Neuron 2014").

From the above, the human monoclonal antibody #129 of the present invention and the anti-HMGB1 antibody having the CDRs common thereto are fully expected to exhibit a treatment effect on general degenerative diseases, including ALS, PD, and HD.

Further, since DNA damage is known to be a cause of aging, and the human monoclonal antibody #129 of the present invention reduces the number of senescent cells in the cerebral cortex of two types of AD model mice (5 × FAD mice and APP-KI mice), and also suppresses the cellular senescence of neurons, astrocytes and microglia (Figures 19 and 20 of Example 2), the human monoclonal antibody #129 of the present invention and the anti-HMGB1 antibody having the CDRs common thereto have been confirmed to have effects of treating aging-related degenerative or neurological disease, brain aging, and diseases associated with brain aging.

### Example 3

### 1. Materials and methods

### [Model mice of various diseases]

As Huntington's disease model mice, R6/2 mice (Document 1) were used. As Parkinson's disease model mice, α-synuclein A53T mice (Document 2) were used. As amyotrophic lateral sclerosis model mice, SOD1-Tg mice (Document 3) were used. As spinocerebellar ataxia model mice, mutant ataxin-1 knock-in mice (Document 4) were used. As aging model mice, C57BL/6 mice at 12 months of age were used.

### [Antibody administration]

At a frequency of once per 2 weeks during the treatment period shown in Table 7, the human monoclonal antibody #129 adjusted with physiological saline to 6 µg per 30 g of body weight (equivalent to 0.2 mg/kg BW) or the same amount of physiological saline without antibody was administered into the caudal vein of model mice of various diseases. Also, as a normal control group, the same amount of physiological saline was administered into the caudal vein of normal genotype sibling mice.

For the Huntington's disease model mice and amyotrophic lateral sclerosis model mice in which improved motor function had not been clarified after the second administration, the amount to be administered was increased to 60 µg per 30 g of body weight (equivalent to 2 mg/kg BW) from the third administration.

Further, in experiments on the aging model mice, 6 µg per 30 g of body weight (equivalent to 0.2 mg/kg BW) was intravenously administered once per 4 weeks.

**[Table 7]**

| | R6/2 mouse | α-synuclein A53T mouse | SOD1-Tg mouse | Mutant ataxin-1 knock-in mouse | Aged mouse |
|---|---|---|---|---|---|
| Duration of treatment with antibody | 6 to 12 weeks of age | 8 to 18 weeks of age | 6 to 20 weeks of age | 8 to 18 weeks of age | 12 to 19 months of age |
| Duration of rotarod test | 6 to 14 weeks of age | 8 to 20 weeks of age | 6 to 22 weeks of age | 8 to 20 weeks of age | - |
| Duration of open field test | 6 to 14 weeks of age | 8 to 20 weeks of age | 6 to 22 weeks of age | - | - |
| Duration of footprint test | 6 to 14 weeks of age | 8 to 20 weeks of age | 6 to 22 weeks of age | 8 to 20 weeks of age | - |
| Y-maze test | - | - | - | - | 12 to 20 months of age |

### [Modified antibody]

Assuming that the light chain CDR3 of the human monoclonal antibody #129 had an amino acid sequence that is likely to undergo glycosylation, a human monoclonal antibody #129-L2 that is an amino acid sequence variant of the light chain CDR3 used in Example 4, which is described later, was administered in place of the human monoclonal antibody #129, in antibody administration experiments on the amyotrophic lateral sclerosis model mice (SOD1-Tg mice) and aging model mice. The alignment of the light chain variable region amino acid sequence of the human monoclonal antibody #129 and the human monoclonal antibody #129-L2 is as shown in Figure 25. The human monoclonal antibody #129 antibody and the human monoclonal antibody #129-L2 antibody were used to study the binding to human dsHMGB1 by surface plasmon resonance (SPR), and as a result, the dissociation constants (K_{D}) of the human monoclonal antibody #129 antibody and the human monoclonal antibody #129-L2 antibody for the human dsHMGB1 were 1.47 × 10⁻¹¹ and 1.44 × 10⁻¹¹, respectively, confirming that the two have almost equivalent binding strength.

### [Motor function tests]

Among the four types of motor function tests (rotarod test, open field test, footprint test, and Y-maze test), three types of the motor function tests (rotarod test, open field test, and footprint test) were performed on the model mice of various diseases and the normal genotype sibling mice during each test period shown in Table 7, according to the methods described in Documents 5 to 7 below. Specifically, in the rotarod test, the mice were placed on a rotating rod (3.5 rpm), and the rotation speed was linearly increased to 35 rpm for 300 seconds and kept at 35 rpm until 360 seconds. The mice underwent 12 trials (4 trials per day for 3 consecutive days) with a rest interval of 30 to 60 minutes between trials. The time taken for the mouse to fall from the rod was recorded on each trial. The average time taken to fall from the rotarod was calculated and used for subsequent analysis. Also, in the open field test, the mice were placed in the center of an open field space (50 cm × 50 cm × 40 cm[height]) and allowed to explore for 10 minutes. The light intensity was 70 lux at the center of the field. The traveling distance (cm) was used as an index. Further, in the footprint test, footprints during walking were recorded to compare walking patterns. The hind paws were covered with blue ink, the forepaws were covered with red ink, and the mice were placed on a passage of a length of 40 cm and a width of 7.5 cm, which served as a runway to a sealed box. White paper on the passage floor was replaced after each run. Footprint patterns were measured using the footprint pattern parameter: stride described in Document 8 below. Further, the Y-maze test was performed according to the method described in Example 1.

### [Immunohistochemical staining method]

The brain was enucleated from disease model mice or normal genotype sibling mice at various months of age and immobilized in PBS containing 4% paraformaldehyde for 12 hours, and a paraffin-embedded brain tissue section with a thickness of 5 µm was prepared using a microtome (manufactured by Yamato Kohki Industrial Co., Ltd.). The prepared paraffin-embedded brain tissue section was deparaffinized using xylene, immersed in a 0.01 M citric acid buffer solution (pH 6.0) for re-hydration, and then heated at 120°C for 15 minutes. Then, permeabilization was performed in PBS containing 0.5% triton-X100, and blocking was performed in PBS containing 10% FBS for 60 minutes. Then, antibody reactions were allowed to occur at 4°C for 12 hours using various primary antibodies to detect 17 types of proteins (pSer46-MARCKS, γH2AX, 53BP1, phosphorylated TDP-43 [Ser409/410] [pSer409/410-TDP-43], ubiquitin, KDEL, MAP2, phosphorylated tau [Ser396]), Huntingtin, Calbindin, α-synuclein, GFAP, Iba1, Lamin B1, Ataxin-1, YAP, and SOD1 (Table 8). Cells were washed three times with PBS, an antibody reaction was allowed to occur at room temperature for one hour using secondary antibodies against various primary antibodies (Table 8), cells were washed three times with PBS, the cell nuclei were stained in PBS containing 0.2 µg/mL 4',6-diamidino-2-phenylindole (DAPI), and a fluorescence image derived from the above-mentioned 17 types of proteins and a fluorescence image derived from the cell nuclei were captured using a confocal microscope (FV1200IX83; manufactured by Olympus Corporation). Then, the number of the cells corresponding to the conditions described in the description of respective figures, per region of 143 µm × 143 µm for the cerebral cortex was calculated on the basis of the DAPI fluorescence image. Further, signal values of the regions of interest were measured on the basis of the pSer46-MARCKS fluorescence image, the γH2AX fluorescence image, the 53BP1 fluorescence image, and the KDEL fluorescence image.

**[Table 8]**

| Primary antibody | Secondary antibody (antibody against the primary antibody in the left column) |
|---|---|
| Rabbit anti-pSer46-MARCKS antibody (manufactured by GL Biochem; dilution factor, 1:300) | Zenon 568 Rabbit IgG Labeling Kits (Z25306; manufactured by Invitrogen) |
| Mouse anti-yH2AX antibody (Ser139; #05-636; manufactured by Millipore; dilution factor, 1:500) | Cy3-labeled anti-mouse IgG (715-165-150, manufactured by Jackson Laboratory; dilution factor, 1:500) |
| Rabbit anti-53BP1 antibody (NB100-304; manufactured by Novus Biologicals; dilution factor, 1:20,000) | Alexa647-labeled anti-rabbit IgG, (A31573.; manufactured by Molecular Probes; dilution factor, 1:1000) |
| Rabbit anti-phosphorylated TDP43 (Ser409/410) antibody (TIP-PTD-P02; manufactured by Cosmo Bio; dilution factor, 1:500) | Alexa568-labeled anti-rabbit IgG, (A10042; manufactured by Molecular Probes; dilution factor, 1:1000) |
| Mouse anti-ubiquitin (Ub) antibody 488-conjugated (CL488-10201; manufactured by Proteintech; dilution factor, 1:100) | n/a |
| Mouse anti-huntingtin (Htt) antibody (MAB5374; manufactured by Sigma Aldrich; dilution factor, 1:100) | Cy3-labeled anti-mouse IgG (715-165-150; manufactured by Jackson Laboratory; dilution factor, 1:500) |
| Mouse anti-KDEL antibody (ADI-SPA-827; manufactured by Enzo Life Science; dilution factor, 1:200) | Cy3-labeled anti-mouse IgG (715-165-150; manufactured by Jackson Laboratory; dilution factor, 1:500) |
| Rabbit anti-MAP2 antibody 647-conjugated (ab225315; manufactured by Abcam; dilution factor, 1:100) | n/a |
| Mouse anti-MAP2 antibody 488-conjugated (ab225316; manufactured by Abcam; dilution factor, 1:300) | n/a |
| Rabbit anti-calbindin antibody (ab108404; manufactured by Abcam; dilution factor, 1:1000) | Alexa488-labeled anti-rabbit IgG (A21206; manufactured by Molecular Probes; dilution factor, 1:1000) |
| Mouse anti-phosphorylated α-synuclein antibody (Ser129, 015-25191; manufactured by FUJIFILM Wako Pure Chemical Corporation; dilution factor, 1:2,000) | Cy3-labeled anti-mouse IgG (715-165-150; manufactured by Jackson Laboratory; dilution factor, 1:500) |
| Rabbit anti-phosphorylated tau (Ser396) antibody, (ab109390; manufactured by Abcam; dilution factor, 1:200) | Alexa568-labeled anti-rabbit IgG, (A10042; manufactured by Molecular Probes; dilution factor, 1:1000) |
| Mouse anti-GFAP antibody Cy3-conjugated (C9205; manufactured by Sigma; dilution factor, 1:100) | n/a |

### [Western blot method]

The cerebral cortex tissues from disease model mice and normal genotype sibling mice, and cerebellar tissues from spinocerebellar ataxia model mice and their background mice at various months of age were dissolved in the presence of a dissolution buffer (100 mM Tris-HCl[pH 7.5], 2% SDS) containing a protease inhibitor cocktail (#539134; manufactured by Calbiochem; dilution factor, 1:100) at 4°C for one hour. After centrifugation (12,000 g × 10 minutes), the supernatant was collected, an equal volume of a sample buffer (125 mM Tris-HCl, pH 6.8, 4% [w/v] SDS, 12% [v/v] 2-mercaptoethanol, 20% [v/v] glycerol, and 0.005% [w/v] bromophenol blue) was added. Then, a protein sample was isolated by SDS-PAGE, then transferred to a polyvinylidene difluoride (PVDF) membrane (Immobilon-P; manufactured by Millipore) by a semi-dry transfer method, and blocked in a TBST solution containing 5% skim milk (10 mM Tris-HCl [pH 8.0], 150 mM NaCl, 0.05% Tween 20). Using various primary antibodies (Table 9) to detect 9 types of proteins (phosphorylated PKCα [Ser319] [pSer319-PKCα], phosphorylated tau [Ser203 (mouse)/Ser214 (human)] [pSer203-tau], phosphorylated tau [Ser384 (mouse)/Ser396 (human)] [pSer384-tau], phosphorylated γH2AX [Ser139] [pSer139-γH2AX], 53BP1, phosphorylated TDP-43 [Ser409/410] [pSer409/410-TDP-43], pSer46-MARCKS, ubiquitin, and β-actin), antibody reactions were allowed to occur in a TBST solution containing 0.1% skim milk or a Can Get Signal solution (manufactured by Toyobo Co., Ltd., Osaka, Japan) at 4°C for 12 hours. The membrane was washed three times with a TBST solution, and antibody reactions were allowed to occur at room temperature for one hour using secondary antibodies against various primary antibodies (Table 9). Then, the above-mentioned 9 types of proteins were detected using an ECL Prime Western blot detection reagent (RPN2235; manufactured by Cytiva) and a luminescence image analyzer (ImageQuant LAS 500; manufactured by GE Healthcare).

**[Table 9]**

| Primary antibody | Secondary antibody (antibody against the primary antibody in the left column) |
|---|---|
| Rabbit anti-phosphorylated PKCα (Thr638) antibody (ab32502; manufactured by Abcam; dilution factor, 1:5,000) | HRP-binding anti-rabbit IgG (NA934; manufactured by Cytiva; dilution factor, 1:3,000) |
| Rabbit anti-phosphorylated tau (Ser203/Ser214), (ab170892; manufactured by Abcam; dilution factor, 1:20,000) | HRP-binding anti-rabbit IgG (NA934; manufactured by Cytiva; dilution factor, 1:3,000) |
| Rabbit anti-phosphorylated tau (S384/S396), (ab109390; manufactured by Abcam; dilution factor, 1:5,000) | HRP-binding anti-rabbit IgG (NA934; manufactured by Cytiva; dilution factor, 1:3,000) |
| Mouse anti- phosphorylated γH2AX antibody (Ser139; #05-636; manufactured by Millipore; dilution factor, 1:2,000) | HRP-binding anti-mouse IgG (NA931; manufactured by Cytiva; dilution factor, 1:3,000) |
| Rabbit anti-53BP1 antibody (NB100-304; manufactured by Novus Biologicals; dilution factor, 1:20,000) | HRP-binding anti-rabbit IgG (NA934; manufactured by Cytiva; dilution factor, 1:3,000) |
| Rabbit anti-phosphorylated TDP43 (Ser409/410) antibody (TIP-PTD-P02; manufactured by Cosmo Bio; dilution factor, 1:3,000) | HRP-binding anti-rabbit IgG (NA934; manufactured by Cytiva; dilution factor, 1:3,000) |
| Rabbit anti-pSer46-MARCKS (manufactured by GL Biochem; dilution factor, 1:20,000) | HRP-binding anti-rabbit IgG (NA934; manufactured by Cytiva; dilution factor, 1:3,000) |
| Mouse anti-ubiquitin antibody (3936; manufactured by Cell Signaling Technology; dilution factor, 1:50,000) | HRP-binding anti-mouse IgG (NA931; manufactured by Cytiva; dilution factor, 1:3,000) |
| Mouse anti-β-actin antibody (sc-47778; manufactured by Santa Cruz Biotechnology; dilution factor, 1:1,000) | HRP-binding anti-mouse IgG (NA931; manufactured by Cytiva; dilution factor, 1:1,000) |

### 2. Results

### [Huntington's disease model mice]

Huntington's disease model mice underwent experiments using post-symptomatic R6/2 mice (Document 1) as shown in "1. Materials and methods" described above, and intravenous administration was performed once per 2 weeks (Figure 23A). At 6 weeks of age, which was the timing of initiating administration, the rotarod test indicated decreased motor function in R6/2 mice. As shown in "1. Materials and methods" described above, in the rotarod test after administering 6 µg of the human monoclonal antibody #129 per 30 g of body weight (equivalent to 0.2 mg/kg BW) twice, improved motor function had not been clarified, and therefore, the amount to be administered was increased to 60 µg per 30 g of body weight (equivalent to 2 mg/kg BW) from the third administration. As a result, at 14 weeks of age after a total of 4 intravenous administration of 6 µg intravenous administration twice and 60 µg intravenous administration twice, there was significant difference in stay duration between the normal mice and physiological saline-administered R6/2 mice in the rotarod test; whereas the significant difference between the normal mice and the human monoclonal antibody #129-administered R6/2 mice disappeared (Figure 23B). However, no significant difference between the physiological saline-administered R6/2 mice and the human monoclonal antibody #129-administered R6/2 mice was observed (Figure 23B). Meanwhile, at 14 weeks of age, in addition to comparison between the normal mice and the physiological saline-administered R6/2 mice, comparison between the physiological saline-administered R6/2 mice and the human monoclonal antibody #129-administered R6/2 mice revealed remarkably significant differences in total walking distance, in the open field test (Figure 23C). Further, at 14 weeks of age, in addition to comparison between the normal mice and the physiological saline-administered R6/2 mice, comparison between the physiological saline-administered R6/2 mice and the human monoclonal antibody #129-administered R6/2 mice revealed remarkably significant differences in stride, in the footprint test (Figure 23D).

Next, at 14 weeks of age, brain tissues were sampled from mice of three groups of physiological saline-administered normal mice, physiological saline-administered R6/2 mice, and human monoclonal antibody #129-administered R6/2 mice (that is, "three groups for analyzing Huntington's disease model mice") after motor function analysis to pathologically analyze. In immunohistochemical staining, triple staining of Htt, Ub and MAP2 was performed, and in the physiological saline-administered R6/2 mice, Htt intranuclear inclusions positive for Ub were observed in the MAP2-positive cerebral nerve cells (Figure 24A). Similar Htt intranuclear inclusions are findings that have been widely recognized in R6/2 mice regardless of the presence or absence of physiological saline administration (Document 1). On the other hand, in the human monoclonal antibody #129-administered R6/2 mice, it was confirmed that Htt intranuclear inclusions positive for Ub were remarkably reduced (Figure 24A). Further, when counting the number of MAP2-positive neurons in the cerebral cortex, the number in the physiological saline-administered R6/2 mice was reduced compared with the normal mice, whereas the number in the human monoclonal antibody #129-administered R6/2 mice was recovered from the physiological saline-administered R6/2 mice (Figure 24B). Meanwhile, the number of cells of microglia and astrocytes were examined using immunostaining with Iba1 and GFAP, and at 14 weeks, there was no significant difference between normal and R6/2 mice, and no change was observed in the number of microglia and astrocytes in R6/2 mice, either, by administration of the human monoclonal antibody #129 (Figures 24C and D).

When performing similar examination on the corpus striatum, Htt intranuclear inclusions positive for Ub were remarkably suppressed in the corpus striatum of R6/2 mice by administration of the human monoclonal antibody #129 (Figure 25A). Further, when counting the number of MAP2-positive neurons in the corpus striatum, the number in the physiological saline-administered R6/2 mice was reduced compared with the normal mice, whereas the number in the human monoclonal antibody #129-administered R6/2 mice was recovered from the physiological saline-administered R6/2 mice (Figure 25B). Meanwhile, the number of cells of microglia and astrocytes were examined using immunostaining with Iba1 and GFAP, and at 14 weeks, there was no significant difference between normal and R6/2 mice, and no change was observed in the number of microglia and astrocytes in R6/2 mice, either, by administration of the human monoclonal antibody #129 (Figures 25C and D).

It is known that neuronal necrosis that is referred to as TRIAD releases HMGB1 (Documents 9 and 10), and that the released HMGB1 induces TRIAD necrosis in surrounding nerve cells (Document 11). Meanwhile, as a TRIAD necrosis marker, positivity of cell body pSer46-MARCKS (Documents 9 to 11) and decreased nuclear YAP (Documents 10 to 13) are known. Accordingly, triple staining of YAP, pSer46-MARCKS and DAPI was performed, and in the physiological saline-administered R6/2 mice, TRIAD necrosis that shows pSer46-MARCKS positivity and decreased nuclear YAP was increased (Figure 26A). Contrary to this, the number of TRIAD necrosis nerve cells was recovered in the human monoclonal antibody #129-administered R6/2 mice from the physiological saline-administered R6/2 mice (Figure 26A). Also, abnormal expansion of endoplasmic reticulum is known to occur in the case of TRIAD (Documents 10 to 13). Accordingly, immunostaining on KDEL, which is an endoplasmic reticulum marker, was performed, and in the physiological saline-administered R6/2 mice, the number of nerve cells that indicate abnormal KDEL staining images was increased (Figure 26B). Meanwhile, in the human monoclonal antibody #129-administered R6/2 mice, the number of nerve cells that indicate abnormal KDEL staining images was decreased (Figure 26B).

Also, DNA damage in nerve cells is known to increase in the pathological conditions of neurodegenerative diseases including Huntington's disease (Documents 14 and 15). Therefore, DNA damage in nerve cells in the cerebral cortex was measured by quadruple staining with MAP2 and DAPI, using DNA damage markers 53BP1 and γH2AX. The results indicated that DNA damage in nerve cells increased in R6/2 mice compared with normal mice, and that the number of nerve cells having DNA damage was suppressed by administration of the human monoclonal antibody #129 (Figure 26C).

Similar immunohistochemistry examination was also performed on the corpus striatum, and the results confirmed that the administration of the human monoclonal antibody #129 remarkably suppressed the increase in TRIAD necrosis in R6/2 mice (Figure 27A), remarkably suppressed the increase in abnormal KDEL staining images (Figure 27B), and remarkably suppressed the increase in nerve cells containing DNA damage (Figure 27C) in the corpus striatum as well.

Next, in order to reconfirm the results of pathological analysis, biochemical analysis was performed on mouse brain tissue samples from three groups for analyzing Huntington's disease model mice at 14 weeks of age. Specifically, when comparing the amounts of proteins of pSer46-MARCKS, 53BP1, γH2AX, Ub, and pSer203-Tau by Western blotting, it was indicated that all of these degeneration pathology marker molecules increased in R6/2 mice compared with normal mice, and that these degeneration pathology marker molecules increased in R6/2 mice decreased to the normal level by administration of the human monoclonal antibody #129 (Figure 28).

The above results indicate that the human monoclonal antibody #129 has an effect of suppressing pathological change and cell death in the pathological conditions of Huntington's disease (HD), and suppressing the decrease in motor function.

### [Parkinson's disease model mice]

Parkinson's disease model mice underwent experiments using post-symptomatic α-synuclein A53T mice (Document 2) as shown in "1. Materials and methods" described above, and intravenous administration was performed once per 2 weeks (Figure 29A). Once per 2 weeks from 8 weeks to 18 weeks of age, 6 µg of the human monoclonal antibody #129 per 30 g of body weight (equivalent to 0.2 mg/kg BW) was administered six times in total (Figure 29A). At 8 weeks of age, which was the timing of initiating administration, the rotarod test indicated that α-synuclein A53T mice were hyperactive (Figure 29B), indicating that Parkinson's disease had already been developed. At 12 weeks of age after a total of three administrations and at 20 weeks of age after a total of six administrations, hyperactivity of α-synuclein A53T mice was suppressed with significant difference at Day 2 (Figure 29B). Similarly, in the open field test at 20 weeks of age, the total traveling distance of physiological saline-administered α-synuclein A53T mice increased compared with normal mice, indicating that α-synuclein A53T mice were hyperactive; however, the total traveling distance was suppressed by administration of the human monoclonal antibody #129 (Figure 29C), indicating improvement in symptoms. Meanwhile, in the footprint test at 20 weeks of age, comparison between three groups of the physiological saline-administered normal mice, the physiological saline-administered α-synuclein A53T mice, and the human monoclonal antibody #129-administered α-synuclein A53T mice (that is, "three groups for analyzing Parkinson's disease model mice") indicated that there was no significant difference in stride, meaning that symptoms of gait disorder did not appear (Figure 29D). The fact indicates that the Parkinson's disease model mice used in the example correspond to at an early stage after onset.

Next, at 20 weeks of age, brain tissues were sampled from mice of the three groups for analyzing Parkinson's disease model mice after motor function analysis to pathologically analyze. In immunohistochemical staining, quadruple staining of pSer129-Syn, Ub, MAP2, and DAPI was performed, and in the physiological saline-administered α-synuclein A53T mice, increase in Ub-positive proteins and increase in pSer129-Syn in cell nuclei were observed in the cytoplasm of MAP2-positive cerebral nerve cells (Figure 30A). Meanwhile, the findings of pSer129-Syn positive and Ub-positive were normalized by administration of the human monoclonal antibody #129. In MAP2 immunohistochemical staining, the number of nerve cells (neurons) in the cerebral cortex was decreased in α-synuclein A53T mice; however, the number in the human monoclonal antibody #129-administered α-synuclein A53T mice was normalized (Figure 30B). Meanwhile, there was no change in the number of cells of Iba1-positive microglia and GFAP-positive astrocytes in α-synuclein A53T mice, and there was no change in the number of cells even by administration of the human monoclonal antibody #129 (Figures 30C and D).

Next, neuronal necrosis that is referred to as TRIAD was examined (Document 12). As described above, it is known that TRIAD releases HMGB1 (Documents 9 and 10), and that the released HMGB1 induces TRIAD necrosis in surrounding nerve cells (Document 11). Further, in another immunohistochemical staining for Parkinson's disease model mice, findings have been reported that pSer46-MARCKS is stained in nerve cells (Document 16). Accordingly, triple staining of YAP, pSer46-MARCKS and DAPI was performed, and in the cerebral cortex of the physiological saline-administered α-synuclein A53T mice, TRIAD necrosis that shows pSer46-MARCKS positivity and decreased nuclear YAP was increased compared with physiological saline-administered normal mice (Figure 31A). Contrary to this, the number of TRIAD necrosis nerve cells was significantly decreased in the human monoclonal antibody #129-administered α-synuclein A53T mice from the physiological saline-administered α-synuclein A53T mice (Figure 31A). Further, since TRIAD is known to accompanied by abnormal expansion of endoplasmic reticulum (Documents 12 and 13), when immunostaining on KDEL, which is an endoplasmic reticulum marker, was performed, the number of nerve cells that show abnormal KDEL staining images was increased in the physiological saline-administered α-synuclein A53T mice, (Figure 30B). Meanwhile, in the human monoclonal antibody #129-administered α-synuclein A53T mice, the nerve cells that indicate abnormal KDEL staining images was normalized (Figure 30B).

Further, DNA damage in nerve cells was measured in the cerebral cortex by quadruple staining with MAP2 and DAPI, using DNA damage markers 53BP1 and γH2AX. The results indicated that DNA damage in nerve cells increased in α-synuclein A53T mice compared with normal mice, and that DNA damage was suppressed by administration of the human monoclonal antibody #129 (Figure 30C).

Next, in order to reconfirm the results of pathological analysis, biochemical analysis was performed on mouse brain tissue samples from three groups for analyzing Parkinson's disease model mice at 20 weeks of age. When comparing five types of proteins (pSer46-MARCKS, 53BP1, γH2AX, Ub, and pSer203-Tau) by Western blotting, it was indicated that all of the five types of proteins increased in α-synuclein A53T mice compared with normal mice, and that the five types of proteins increased in α-synuclein A53T mice decreased to the normal level by administration of the human monoclonal antibody #129 (Figure 32).

The above results indicate that the human monoclonal antibody #129 has an effect of suppressing pathological change and cell death in the pathological conditions of Parkinson's disease (PD), and suppressing the decrease in motor function.

### [Amyotrophic lateral sclerosis model mice]

Amyotrophic lateral sclerosis model mice underwent experiments using post-symptomatic SOD1-Tg mice (Document 3) as shown in "1. Materials and methods" described above, and intravenous administration was performed once per 2 weeks (Figure 33A). Once per 2 weeks from 6 weeks to 9 weeks of age, 6 µg of the human monoclonal antibody #129-L2 per 30 g of body weight (equivalent to 0.2 mg/kg BW) was administered twice in total (Figure 33A). The rotarod test immediately before initiating administration indicated decreased motor function in SOD1-Tg mice at 6 weeks of age (Figure 33B), indicating that SOD1-Tg mice had already developed amyotrophic lateral sclerosis. Since no improvement showing significant difference was observed in the rotarod test by 10 weeks of age, the amount to be administered was increased to 60 µg per 30 g of body weight (equivalent to 2 mg/kg BW) from the third administration at 10 weeks of age to the eighth administration at 20 weeks of age.

As a result of administrating the human monoclonal antibody #129-L2, rotarod stay duration of SOD1-Tg mice at 22 weeks of age significantly decreased compared with normal mice, but suppressed with significant difference by administration of the human monoclonal antibody #129-L2 (Figure 33B).

Similarly, in the open field test at 22 weeks of age, the total traveling distance of physiological saline-administered SOD1-Tg mice decreased compared with normal mice, indicating decreased motor function; however, the total traveling distance was increased by administration of the human monoclonal antibody #129-L2 (Figure 33C), indicating improvement in symptoms. Further, in the footprint test at 22 weeks of age, in comparison between the normal mice and the physiological saline-administered SOD1-Tg mice, there was a decrease in stride; however, the stride was increased (Figure 33C) by administration of the human monoclonal antibody #129-L2, indicating that symptoms of decreased motor function were improved (Figure 33D).

Next, at 22 weeks of age, brain tissues were sampled from mice of three groups of physiological saline-administered normal mice, physiological saline-administered SOD1-Tg mice, and human monoclonal antibody #129-L2-administered SOD1-Tg mice (that is, "three groups for analyzing amyotrophic lateral sclerosis model mice") after motor function analysis to pathologically analyze. In immunohistochemical staining, when quadruple staining of Ub, SOD1, MAP2, and DAPI was performed, there was an increase in the staining signals of SOD1 and Ub in large neurons in spinal cord anterior horn of SOD1-Tg mice, and some of them had inclusion-like morphology. Such pathological morphology almost disappeared by administration of the human monoclonal antibody #129-L2 (Figure 34A). Further, in quadruple staining of Ub, pSer396-Tau, MAP2, and DAPI, colocalization of Ub and pSer396-Tau in cytoplasm was observed in large neurons in spinal cord anterior horn of SOD1-Tg mice, which also almost disappeared by administration of the human monoclonal antibody #129-L2 (Figure 34B). In addition, in quadruple staining of Ub, pTDP43, MAP2, and DAPI, pTDP43 transfer into cytoplasm was barely observed; however, slight staining property of pTDP43 was observed in the cytoplasmic inclusions of Ub (Figure 34C). When counting the number of MAP2-positive neurons having such ubiquitin-positive cytoplasmic inclusions in the lumbar cord, the number in SOD1-Tg mice remarkably increased compared with normal mice in all of quadruple staining; however, the number remarkably suppressed by administration of the human monoclonal antibody #129-L2 (Figures 34A to C). There has been little examination on TDP43 aggregates in SOD1-Tg mice, and there is no established theory. However, clear TDP43-positive inclusions have barely been recognized even in the patient pathology of human amyotrophic lateral sclerosis (ALS) accompanied with SOD mutation (Document 17), which is consistent with the above-mentioned findings.

Next, neuronal necrosis that is referred to as TRIAD was examined (Document 12). As described above, it is known that TRIAD releases HMGB1 (Documents 9 and 10), and that the released HMGB1 induces TRIAD necrosis in surrounding nerve cells (Document 11). Further, in another immunohistochemical staining for SOD1-Tg mice, YAP fluctuations have been reported (Document 18). Meanwhile, as a TRIAD necrosis marker, positivity of cell body pSer46-MARCKS (Document 9) and decreased nuclear YAP (Document 10) are known. Accordingly, triple staining of YAP, pSer46-MARCKS and DAPI was performed, and TRIAD necrosis that shows pSer46-MARCKS positivity and decreased nuclear YAP was increased in large neurons in spinal cord anterior horn (motoneurons) of the physiological saline-administered SOD1-Tg mice (Figure 35A). Contrary to this, the number of nerve cells causing TRIAD necrosis was confirmed to be suppressed in the human monoclonal antibody #129-L2-administered SOD1-Tg mice (Figure 35A).

Further, when counting the number of MAP2-positive neurons in the lumbar cord, the number in the physiological saline-administered SOD1-Tg mice was reduced compared with the normal mice, whereas the number in the human monoclonal antibody #129-L2-administered SOD1-Tg mice was recovered from the physiological saline-administered SOD1-Tg mice (Figure 35B). Also, there was tendency for MAP2 staining property of neuropil to decrease in SOD1-Tg mice, which was also recovered in the human monoclonal antibody #129-L2-administered SOD1-Tg mice (Figure 35B). There was no significant difference in the number of microglia in spinal cord between normal and SOD1-Tg mice in Iba1 staining, and no change in the number of microglia was observed in SOD1-Tg mice, either, by administration of the human monoclonal antibody #129-L2 (Figure 35C). Meanwhile, in GFAP staining, the number of astrocytes in SOD1-Tg mice significantly increased; however, the number remarkably suppressed by administration of the human monoclonal antibody #129-L2 (Figure 35D).

Also, DNA damage in nerve cells is known to increase in the pathological conditions of neurodegenerative diseases including Huntington's disease as described above (Documents 14 and 15). Accordingly, DNA damage in nerve cells was measured in the lumbar anterior horn by quadruple staining with MAP2 and DAPI, using DNA damage markers 53BP1 and γH2AX. The results indicated that DNA damage in nerve cells increased in SOD1-Tg mice compared with normal mice, and that the DNA damage in nerve cells was suppressed by administration of the human monoclonal antibody #129-L2 (Figure 36).

Next, in order to reconfirm the results of pathological analysis, biochemical analysis was performed on mouse brain tissue samples from three groups for analyzing amyotrophic lateral sclerosis model mice at 22 weeks of age. Specifically, when comparing the amounts of proteins of pSer46-MARCKS, 53BP1, γH2AX, Ub, and pSer396-Tau by Western blotting, it was indicated that all of these degeneration pathology marker molecules increased in SOD1-Tg mice compared with normal mice, and that these degeneration pathology marker molecules increased in SOD1-Tg mice decreased to the normal level by administration of the human monoclonal antibody #129-L2 (Figure 37).

The above results indicate that the human monoclonal antibody #129-L2 has an effect of suppressing pathological change and cell death in the pathological conditions of amyotrophic lateral sclerosis (ALS), and suppressing the decrease in motor function.

### [Spinocerebellar ataxia model mice]

Spinocerebellar ataxia model mice underwent experiments using post-symptomatic mutant ataxin-1 knock-in mice (Document 4) as shown in "1. Materials and methods" described above, and intravenous administration was performed once per 2 weeks (Figure 38A). Once per 2 weeks from 8 weeks to 18 weeks of age, 6 µg of the human monoclonal antibody #129 per 30 g of body weight (equivalent to 0.2 mg/kg BW) was administered six times in total (Figure 38A). At 8 weeks of age, which was the timing of initiating administration, the rotarod test indicated that decrease in motor function was obvious in mutant ataxin-1 knock-in mice (Figure 38B), indicating that spinocerebellar ataxia had already been developed. At 20 weeks of age after a total of six administrations, rotarod stay duration of mutant ataxin-1 knock-in mice significantly decreased compared with normal mice, but improved with significant difference by administration of the human monoclonal antibody #129 (Figure 38B). Similarly, in the footprint test at 20 weeks of age, in comparison between the normal mice and the physiological saline-administered mutant ataxin-1 knock-in mice, there was a decrease in stride; however, the stride of mutant ataxin-1 knock-in mice was increased with significant difference by administration of the human monoclonal antibody #129 (Figure 38C), indicating improved symptoms.

Next, at 20 weeks of age, brain tissues were sampled from mice of three groups of physiological saline-administered normal mice, physiological saline-administered mutant ataxin-1 knock-in mice, and human monoclonal antibody #129-administered mutant ataxin-1 knock-in mice (that is, "three groups for analyzing spinocerebellar ataxia model mice") after motor function analysis to pathologically analyze. First, TRIAD, which is cell death targeted by the human monoclonal antibody #129, was investigated. It is known that neuronal necrosis that is referred to as TRIAD (Document 12) releases HMGB1 as described above (Documents 9 and 10), and that the released HMGB1 induces TRIAD necrosis (Document 11) or neurite degeneration (Document 9) in surrounding nerve cells. Meanwhile, as a TRIAD necrosis marker, positivity of cell body or neurite pSer46-MARCKS (Documents 9 to 11) and decreased nuclear YAP (Documents 10 to 13) are known. Also, it is known that the expression level of YAP affects the prognosis of mutant ataxin-1 knock-in mice even in the pathological conditions of spinocerebellar ataxia type 1 (SCA1) (Document 19). Accordingly, triple staining of YAP, pSer46-MARCKS and DAPI was performed, and in the physiological saline-administered mutant ataxin-1 knock-in mice, pSer46-MARCKS staining property of granule cells and their nerve fibers in the granule cell layer of the cerebellar cortex was remarkably increased compared with physiological saline-administered normal mice (Figure 39A). This is similar to the increase in pSer46-MARCKS staining property found in neurite degeneration due to HMGB1 release, which has been observed in Alzheimer's disease pathology (Document 9), indicating neurite degeneration in the granule cell layer. Meanwhile, in the human monoclonal antibody #129-administered mutant ataxin-1 knock-in mice, such an increase in pSer46-MARCKS staining property of granule cells and their nerve fibers was not observed but normalized (Figure 39A).

Regarding inclusions, as described in a previous report (Document 4), no significant intranuclear inclusion was observed in Purkinje cells of the mutant ataxin-1 knock-in mice; however, colocalization of high-signal Ub and Atxn1 was confirmed (Figure 39B). Such findings were not observed in normal mice, and disappeared even in mutant ataxin-1 knock-in mice by administration of the human monoclonal antibody #129 (Figure 39B).

Further, DNA damage in nerve cells is known to increase in the pathological conditions of neurodegenerative diseases including SCA1 (Documents 14 and 15). Therefore, DNA damage in Purkinje cells was measured by quadruple staining with MAP2 and DAPI, using DNA damage markers 53BP1 and γH2AX. The results indicated that DNA damage in Purkinje cells increased in mutant ataxin-1 knock-in mice compared with normal mice, and that DNA damage in Purkinje cells was suppressed by administration of the human monoclonal antibody #129 (Figure 39C).

In addition, in KDEL staining performed to detect abnormal endoplasmic reticulum, which is a characteristic of TRIAD, KDEL signal values of the Purkinje cell layer and granule cell layer were increased in mutant ataxin-1 knock-in mice compared with normal mice, confirming endoplasmic reticulum instability, which is observed in TRIAD (Figure 40A). Whether such findings eventually affect the number of Purkinje cells or the number of microglia and astrocytes in cerebellar cortex was confirmed by MAP2, Iba1, and GFAP staining (Figure 40B). As a result, the number of Purkinje cells decreased in mutant ataxin-1 knock-in mice compared with normal mice; however, cell shedding was suppressed by administration of the human monoclonal antibody #129 (Figure 40B). Meanwhile, there were no changes in microglia and astrocytes (Figure 40B).

Next, in order to reconfirm the results of pathological analysis, biochemical analysis was performed on mouse brain tissue samples from three groups for analyzing spinocerebellar ataxia model mice at 20 weeks of age. Specifically, when comparing the amounts of proteins of pSer46-MARCKS, 53BP1, γH2AX, Ub, and pSer203-Tau by Western blotting, it was indicated that all of these degeneration pathology marker molecules increased in mutant ataxin-1 knock-in mice compared with normal mice, and that these degeneration pathology marker molecules increased in mutant ataxin-1 knock-in mice decreased to the normal level by administration of the human monoclonal antibody #129 (Figure 41).

The above results indicate that the human monoclonal antibody #129 has an effect of suppressing pathological change and cell death in spinocerebellar ataxia, and suppressing the decrease in motor function.

### [Aging model mice]

Aging model mice underwent experiments using C57BL/6 mice at 12 months of age as shown in "1. Materials and methods" described above, and intravenous administration was performed eight times in total, once per 4 weeks from 12 months to 19 months of age (Figure 42A). In a Y-maze test on mice from 12 months to 18 months of age, there was no significant difference between physiological saline-administered C57BL/6 mice at 12 months of age and human monoclonal antibody #129-L2-administered C57BL/6 mice at 12 months of age; however, for the physiological saline-administered C57BL/6 mice at 12 months of age, a significant difference between the two became observed after 19 months of age in the Y-maze test, where a decreased score due to aging was observed (Figure 42B).

Next, brain tissues were sampled from mice of three groups of young mice (C57BL/6 mice at 3 months of age), physiological saline-administered C57BL/6 mice at 20 months of age, and human monoclonal antibody #129-L2-administered C57BL/6 mice at 20 months of age (that is, "three groups for analyzing aging model mice at 20 months of age") after motor function analysis to pathologically analyze. Lamin B1 is an inner nuclear membrane protein and shows a ring-shaped nuclear stain pattern in immunohistochemical staining; however, it is said that the ring-shaped nuclear localization disappears in cellular senescence (Document 20). Further, since the ring-shaped nuclear localization of Lamin B1 similarly disappears in nerve cells where TRIAD necrosis occurred (Document 11), there is an assumption that TRIAD is a cell death in normal aging (Document 11). Therefore, triple immunostaining of two types of TRIAD markers (YAP and pSer46-MARCKS) and Lamin B1 was performed on brain tissues from mice of three groups for analyzing aging model mice at 20 months of age, and the presence or absence of increase in TRIAD necrosis in normal aging and the effects of the human monoclonal antibody #129-L2 on nerve cell death in aging were investigated.

The results revealed that TRIAD necrosis that shows loss of nuclear YAP staining and pSer46-MARCKS cytoplasmic staining was remarkably increased in the cerebral cortex of the physiological saline-administered C57BL/6 mice at 20 months of age compared with the C57BL/6 mice at 3 months of age (Figure 43), and at the same time, senescent nerve cells in which nuclear ring staining of Lamin B1 had disappeared remarkably increased (Figure 43). Further, senescent nerve cells that have caused TRIAD necrosis showing three characteristics of loss of nuclear YAP staining, pSer46-MARCKS cytoplasmic staining, and loss of nuclear ring staining of Lamin B1 were significantly increased (Figure 43). Meanwhile, in the human monoclonal antibody #129-L2-administered aged mice (20 months of age), all of the percentage of the cells showing such three characteristics were remarkably suppressed (Figure 43).

DNA damage in nerve cells in the cerebral cortex was measured by quadruple staining with MAP2 and DAPI, using DNA damage markers 53BP1 and γH2AX. The results indicated that DNA damage in nerve cells increased in aged mice compared with young mice, and that the DNA damage in nerve cells in the cerebral cortex of the aged mice was suppressed by administration of the human monoclonal antibody #129-L2 (Figure 44A). Whether such findings eventually affect the number of nerve cells, the number of microglia and the number of astrocytes in cerebral cortex was confirmed by MAP2, Iba1, and GFAP staining (Figure 44B). As a result, the number of nerve cells decreased in the physiological saline-administered C57BL/6 mice at 20 months of age compared with young mice (C57BL/6 mice at 3 months of age); however, cell shedding was suppressed by administration of the human monoclonal antibody #129-L2 (Figure 44B). Meanwhile, there were no changes in the number of microglia and astrocytes (Figures 44C and D).

Next, in order to reconfirm the results of pathological analysis, biochemical analysis was performed on mouse brain tissue samples from three groups for analyzing aging model mice at 20 months of age. Specifically, when comparing the amounts of proteins of pSer46-MARCKS, 53BP1, γH2AX, Ub, and pSer203-Tau by Western blotting, it was indicated that all of these degeneration pathology marker molecules increased in aged mice (20 months of age) compared with young mice (3 months of age), and that these degeneration pathology marker molecules increased in aged mice decreased to the normal level by administration of the human monoclonal antibody #129-L2 (Figure 45).

The above results indicate that the human monoclonal antibody #129-L2 has an effect of suppressing aging-associated cellular changes and cell death, and suppressing the decrease in cognitive function.

### 3. Conclusion

The human monoclonal antibody of the present invention shows the fact of exhibiting treatment effects on Huntington's disease, amyotrophic lateral sclerosis, Parkinson's disease and spinocerebellar ataxia, as well as aging-associated cellular changes, cell death, cognitive function (memory), and motor function.
Document 1: Mangiarini, L., K. Sathasivam, M. Seller, B. Cozens, A. Harper, C. Hetherington, M. Lawton, Y. Trottier, H. Lehrach, S. Davies, and G. Bates. 1996. Exon 1 of the HD gene with an expanded CAG repeat is sufficient to cause a progressive neurological phenotype in transgenic mice. Cell. 87:493-506.;
Document 2: Giasson BI, Duda JE, Quinn SM, Zhang B, Trojanowski JQ, Lee VM. Neuronal alpha-synucleinopathy with severe movement disorder in mice expressing A53T human alpha-synuclein. Neuron. 2002 May 16; 34(4):521-33.;
Document 3: Gurney ME, Pu H, Chiu AY, Dal Canto MC, Polchow CY, Alexander DD, Caliendo J, Hentati A, Kwon YW, Deng HX, et al. Motor neuron degeneration in mice that express a human Cu, Zn superoxide dismutase mutation. Science. 1994 264(5166): 1772-1775. Erratum in: Science 1995 269(5221): 149.;
Document 4: Watase K, Weeber EJ, Xu B, Antalffy B, Yuva-Paylor L, Hashimoto K, Kano M, Atkinson R, Sun Y, Armstrong DL, Sweatt JD, Orr HT, Paylor R, Zoghbi HY. A long CAG repeat in the mouse Sca1 locus replicates SCA1 features and reveals the impact of protein solubility on selective neurodegeneration. Neuron. 2002 Jun 13; 34(6):905-19.;
Document 5: Ito H, Yoshimura N, Kurosawa M, Ishii S, Nukina N, Okazawa H. Knock-down of PQBP1 impairs anxiety-related cognition in mouse. Hum Mol Genet. 2009 Nov 15; 18(22):4239-54.;
Document 6: Ito H, Shiwaku H, Yoshida C, Homma H, Luo H, Chen X, Fujita K, Musante L, Fischer U, Frints SG, Romano C, Ikeuchi Y, Shimamura T, Imoto S, Miyano S, Muramatsu SI, Kawauchi T, Hoshino M, Sudol M, Arumughan A, Wanker EE, Rich T, Schwartz C, Matsuzaki F, Bonni A, Kalscheuer VM, Okazawa H. In utero gene therapy rescues microcephaly caused by Pqbp1-hypofunction in neural stem progenitor cells. Mol Psychiatry. 2015 Apr; 20(4):459-71.;
Document 7: Taniguchi JB, Kondo K, Fujita K, Chen X, Homma H, Sudo T, Mao Y, Watase K, Tanaka T, Tagawa K, Tamura T, Muramatsu SI, Okazawa H. RpA1 ameliorates symptoms of mutant ataxin-1 knock-in mice and enhances DNA damage repair. Hum Mol Genet. 2016 Oct 15; 25(20):4432-4447.;
Document 8: Keiser, M.S., Boudreau, R. L. and Davidson, B.L. (2014) Broad Therapeutic Benefit After RNAi Expression Vector Delivery to Deep Cerebellar Nuclei: Implications for Spinocerebellar Ataxia Type 1 Therapy. Mol. Ther., 22, 588-595.;
Document 9: Fujita K, Motoki K, Tagawa K, Chen X, Hama H, Nakajima K, Homma H, Tamura T, Watanabe H, Katsuno M, Matsumi C, Kajikawa M, Saito T, Saido T, Sobue G, Miyawaki A, Okazawa H. HMGB1, a pathogenic molecule that induces neurite degeneration via TLR4-MARCKS, is a potential therapeutic target for Alzheimer's disease. Sci Rep. 2016 Aug 25; 6:31895.;
Document 10: Tanaka H, Homma H, Fujita K, Kondo K, Yamada S, Jin X, Waragai M, Ohtomo G, Iwata A, Tagawa K, Atsuta N, Katsuno M, Tomita N, Furukawa K, Saito Y, Saito T, Ichise A, Shibata S, Arai H, Saido T, Sudol M, Muramatsu SI, Okano H, Mufson EJ, Sobue G, Murayama S, Okazawa H. YAP-dependent necrosis occurs in early stages of Alzheimer's disease and regulates mouse model pathology. Nat Commun. 2020 Jan 24; 11(1):507.;
Document 11: Tanaka H, Kondo K, Fujita K, Homma H, Tagawa K, Jin X, Jin M, Yoshioka Y, Takayama S, Masuda H, Tokuyama R, Nakazaki Y, Saito T, Saido T, Murayama S, Ikura T, Ito N, Yamamori Y, Tomii K, Bianchi ME, Okazawa H. HMGB1 signaling phosphorylates Ku70 and impairs DNA damage repair in Alzheimer's disease pathology. Commun Biol. 2021 Oct 11; 4(1):1175.;
Document 12: Hoshino M, Qi ML, Yoshimura N, Miyashita T, Tagawa K, Wada Y, Enokido Y, Marubuchi S, Harjes P, Arai N, Oyanagi K, Blandino G, Sudol M, Rich T, Kanazawa I, Wanker EE, Saitoe M, Okazawa H. Transcriptional repression induces a slowly progressive atypical neuronal death associated with changes of YAP isoforms and p73. J Cell Biol. 2006 Feb 13; 172(4):589-604.;
Document 13: Mao Y, Chen X, Xu M, Fujita K, Motoki K, Sasabe T, Homma H, Murata M, Tagawa K, Tamura T, Kaye J, Finkbeiner S, Blandino G, Sudol M, Okazawa H. Targeting TEAD/YAP-transcription-dependent necrosis, TRIAD, ameliorates Huntington's disease pathology. Hum Mol Genet. 2016 Nov 1; 25(21):4749-4770.;
Document 14: Qi ML, Tagawa K, Enokido Y, Yoshimura N, Wada Y, Watase K, Ishiura S, Kanazawa I, Botas J, Saitoe M, Wanker EE, Okazawa H. Proteome analysis of soluble nuclear proteins reveals that HMGB1/2 suppress genotoxic stress in polyglutamine diseases. Nat Cell Biol. 9(4):402-14, 2007.;
Document 15: Enokido Y, Tamura T, Ito H, Arumughan A, Komuro A, Shiwaku H, Sone M, Foulle R, Sawada H, Ishiguro H, Ono T, Murata M, Kanazawa I, Tomilin N, Tagawa K, Wanker EE, Okazawa H. Mutant huntingtin impairs Ku70-mediated DNA repair. J Cell Biol. 2010 189(3):425-43, 2010.;
Document 16: Fujita K, Homma H, Kondo K, Ikuno M, Yamakado H, Tagawa K, Murayama S, Takahashi R, Okazawa H. Ser46-Phosphorylated MARCKS Is a Marker of Neurite Degeneration at the Pre-aggregation Stage in PD/DLB Pathology. eNeuro. 2018 Sep 4; 5(4):ENEURO.0217-18.2018.;
Document 17: Forsberg KM, Graffmo KS, Stenvall E, Tabikh N, Marklund SL, Brannstrom T, Andersen PM. Widespread CNS pathology in amyotrophic lateral sclerosis homozygous for the D90A SOD1 mutation. Acta Neuropathol. 2023 Jan; 145(1):13-28.;
Document 18: Morimoto N, Nagai M, Miyazaki K, Kurata T, Takehisa Y, Ikeda Y, Kamiya T, Okazawa H, Abe K. Progressive decrease in the level of YAPdeltaCs, prosurvival isoforms of YAP, in the spinal cord of transgenic mouse carrying a mutant SOD1 gene. J Neurosci Res. 2009 Mar; 87(4):928-36.;
Document 19: Fujita K, Mao Y, Uchida S, Chen X, Shiwaku H, Tamura T, Ito H, Watase K, Homma H, Tagawa K, Sudol M, Okazawa H. Developmental YAPdeltaC determines adult pathology in a model of spinocerebellar ataxia type 1. Nat Commun. 2017 Nov 30; 8(1):1864.;
Document 20: Freund A, Laberge RM, Demaria M, Campisi J. Lamin B1 loss is a senescence-associated biomarker. Mol Biol Cell. 2012 Jun; 23(11):2066-75.

### Example 4

Assuming that the light chain CDR3 of the human monoclonal antibody #129 had an amino acid sequence that is likely to undergo glycosylation, as described above, two types of amino acid sequence variants of the light chain CDR3 (human monoclonal antibody #129-L1 and human monoclonal antibody #129-L2 were produced according to a conventional method (Table 10).

**[Table 10]**

| | Amino acid sequence of light chain CDR3 |
|---|---|
| Human monoclonal antibody #129 | LQHNSTPLT |
| Human monoclonal antibody #129-L1 | LQHNANPLT |
| Human monoclonal antibody #129-L2 | LQHASTPLT |

The amino acid sequences shown by underlines in the table indicate the modified sites of the amino acid sequences of the light chain CDR3 of the human monoclonal antibody #129.

The antibody affinity of the human monoclonal antibody #129 and the human monoclonal antibody #129-L2 for human dsHMGB1 was analyzed by SPR described in Example 1. The results indicated that the dissociation constants (K_{D}) of the human monoclonal antibody #129 and the human monoclonal antibody #129-L2 for the human dsHMGB1 were 1.47 × 10⁻¹¹ and 1.44 × 10⁻¹¹, respectively, confirming that the two antibodies have almost equivalent binding strength to human dsHMGB1 (Figures 46B and A). Further, the dissociation constants (K_{D}) of the human monoclonal antibody #129 and the human monoclonal antibody #129-L2 for the human reduced HMGB1 were 2.07 × 10⁻⁷ and 1.51 × 10⁻⁵, respectively, confirming that the human monoclonal antibody #129-L2 has weaker binding strength than the human monoclonal antibody #129 (Figures 46D and C).

Also, when determining the dissociation constants (K_{D}) using ELISA, no difference was observed in the binding strength of the human monoclonal antibody #129 and the human monoclonal antibody #129-L2 to human dsHMGB1 (Figures 47B and A). Further, the binding strength of both the human monoclonal antibody #129 and the human monoclonal antibody #129-L2 to human reduced HMGB1 was weak, and in this case, no difference was observed in the binding strength of the two antibodies (Figures 47D and C).

Therefore, using the human monoclonal antibody #129 and the human monoclonal antibody #129-L2, treatment effects on three types of FTLD-ALS model mice (TDP43^{N267S}-KI mice, PGRN^{R504X}-KI mice, and VCP^{T262A}-KI mice) were investigated using the Y-maze test described in Example 1. Physiological saline containing 0.2 mg/kg body weight (6 µg/animal) of the human monoclonal antibody #129 or human monoclonal antibody #129-L2 was administered once per month to one mouse each of various FTLD-ALS model mice by intravenous injection via caudal vein, according to the therapeutic experimental protocol shown in Figure 48. Also, as a control group, the same amount of physiological saline was similarly administered to various FTLD-ALS model mice or normal mice (sibling mice that do not have mutant genes of various FTLD-ALS model mice). Of note, the administration was initiated after development of the diseases was confirmed by a Y-maze test.

As a result, improvement in cognitive function was observed by the Y-maze test from one month after initiating administration of the two types of anti-human HMGB1 antibodies of the present invention (the human monoclonal antibody #129-L2 or the human monoclonal antibody #129) in all of the FTLD-ALS model mice (Figure 48). Although the human monoclonal antibody #129 showed superior treatment effects at some time points, in general, no substantial difference in treatment effects was observed between the human monoclonal antibody #129 and the human monoclonal antibody #129-L2, confirming that both have statistically significant treatment effects (Figure 48).

In addition, after the administration of the two types of anti-human HMGB1 antibodies of the present invention (the human monoclonal antibody #129-L2 or the human monoclonal antibody #129) once per month was performed two more times, brain tissues were pathologically analyzed according to the method described in Example 3. In immunohistochemical staining, TRIAD necrosis was examined by staining of pSer46-MARCKS (Figure 49) and KDEL (Figure 50), DNA damage was examined by staining of γH2AX (Figure 51) and 53BP1 (Figure 52), and inclusions were examined by staining of pSer409/410-TDP43 (Figure 53), and hence, in general, no substantial difference in treatment effects was observed between the human monoclonal antibody #129 and the human monoclonal antibody #129-L2, confirming that both have statistically significant treatment effects (Figures 49 to 53).

Meanwhile, differences were observed between the human monoclonal antibody #129 and the human monoclonal antibody #129-L2 at the following points. Specifically, the human monoclonal antibody #129 was purified from a plurality of clones of a CHO cell line that stably expresses human monoclonal antibody #129 according to a conventional method, and analyzed by non-reducing capillary electrophoresis according to a conventional method, and as a result, gentle peaks (arrow indicated site in the figure) were observed on the high-molecular weight side from the peak of IgG in all of the clones, as shown in Figure 54. Meanwhile, the human monoclonal antibody #129-L2 was purified from a plurality of clones of a CHO cell line that stably expresses human monoclonal antibody #129-L2, and analyzed by non-reducing capillary electrophoresis, and as a result, no gentle peaks were observed on the high-molecular weight side in any of the clones, as shown in Figure 55.

The results indicate that the human monoclonal antibody #129 underwent N-glycan glycosylation, whereas the human monoclonal antibody #129-L2 did not undergo N-glycan glycosylation.

In addition, SDS-PAGE under normal reducing conditions was conducted using 5 µg of three types of the anti-human HMGB1 antibodies of the present invention (the human monoclonal antibody #129, the human monoclonal antibody #129-L1, and the human monoclonal antibody #129-L2), and acrylamide gel was stained with Coomassie brilliant blue (CBB), and as a result, a pale band was observed above the band derived from the light chain of the human monoclonal antibody #129. Contrary to this, no such band was observed above the band derived from the light chain of the human monoclonal antibody #129-L1 or the band derived from the light chain of the human monoclonal antibody #129-L2 (Figure 56) .

The results indicate that the light chain of the human monoclonal antibody #129 underwent N-glycan glycosylation, whereas the light chain of the human monoclonal antibody #129-L1 or the light chain of the human monoclonal antibody #129-L2 did not undergo N-glycan glycosylation.

Further, in order to examine the stability of two types of the anti-human HMGB1 antibodies of the present invention (the human monoclonal antibody #129, and the human monoclonal antibody #129-L2), antibody affinity of the two types of anti-human HMGB1 antibodies of the present invention for HMGB1 was analyzed by SPR, before and after storage for 6 months at 4°C. As a result, K_{D} of the human monoclonal antibody #129 was 1.47 × 10⁻¹¹ before the storage for 6 months at 4°C (Figure 57C), whereas it was 6.51 × 10⁻¹⁰ after the storage for 6 months at 4°C (Figure 57D), indicating an increase of about 45-fold. Meanwhile, K_{D} of the human monoclonal antibody #129-L2 was 1.44 × 10⁻¹¹ before the storage for 6 months at 4°C (Figure 57A), whereas it was 3.51 × 10⁻¹¹ after the storage for 6 months at 4°C (Figure 57B), indicating an increase of only about 2.5-fold.

The results indicate that the human monoclonal antibody #129-L2 has higher antibody stability than the human monoclonal antibody #129.

Further, using the two types of the anti-human HMGB1 antibodies of the present invention (the human monoclonal antibody #129, and the human monoclonal antibody #129-L2), treatment effects on Alzheimer's disease model mice (5 × FAD mice) were investigated. Specifically, physiological saline containing 0.2 mg/kg body weight (6 µg/animal) of the human monoclonal antibody #129 or human monoclonal antibody #129-L2 was administered to one mouse of the Alzheimer's disease model mice by intravenous injection via caudal vein, once per month for a total of three times starting from 6 months of age after development of the disease (Figure 58A). Also, as a control group, physiological saline containing 0.2 mg/kg body weight (6 µg/animal) of human IgG was similarly administered (Figure 58A). After administration, measurement of the number of synapses of the cerebral cortex by two-photon microscopic observation (Figure 58B), cognitive function test by a Y-maze test (Figure 58C), and immunohistochemical staining for mouse cerebral cortex using anti-pSer46-MARCKS antibody and anti-Aβ antibody were performed to evaluate cell death and extracellular Aβ deposits (Figure 59).

The results indicated that, in 5 × FAD mice at 8 months of age, the human monoclonal antibody #129 and the human monoclonal antibody #129-L2 equivalently normalize the decrease of synapses (Figure 58B), and equivalently normalize the decreased cognitive function (Figure 58C). Further, in pathological analysis of Alzheimer's disease pathology, it has been reported that three stages of TRIAD necrosis of one neuron due to intracellular amyloid accumulation (primary TRIAD necrosis), TRIAD necrosis of surrounding multiple nerve cells induced by HMGB1 released from the primary TRIAD necrosis (secondary TRIAD necrosis), and extracellular Aβ deposits (amyloid plaques) in which cellular debris of the secondary TRIAD necrosis has been removed (Figure 59A) occur in sequential order ("Tanaka et al, Nature Commun 2020"). It was indicated that, although both the human monoclonal antibody #129 and the human monoclonal antibody #129-L2 do not affect the primary TRIAD necrosis, they remarkably suppress the secondary TRIAD necrosis and amyloid plaques, and hence, the two has equivalent effects (Figure 59B).

### Industrial Applicability

The present invention contributes to prevention and/or treatment of amyotrophic lateral sclerosis, Parkinson's disease, Huntington's disease, spinocerebellar ataxia, Alzheimer's disease, frontotemporal lobar degeneration, aging-related degenerative or neurological disease, brain aging, or diseases associated with brain aging.

## Claims

1. An agent for preventing or treating amyotrophic lateral sclerosis, Parkinson's disease, Huntington's disease, spinocerebellar ataxia, aging-related degenerative or neurological diseases, brain aging, or diseases associated with brain aging, comprising a human monoclonal antibody that specifically binds to human HMGB1 and comprises:
a heavy chain complementarity determining region (CDR) 1 consisting of the amino acid sequence shown in SEQ ID No: 1 or an amino acid sequence in which one or more amino acids are substituted, deleted, added, and/or inserted in the amino acid sequence shown in SEQ ID No: 1; a heavy chain CDR2 consisting of the amino acid sequence shown in SEQ ID No: 2 or an amino acid sequence in which one or more amino acids are substituted, deleted, added, and/or inserted in the amino acid sequence shown in SEQ ID No: 2; and a heavy chain CDR3 consisting of the amino acid sequence shown in SEQ ID No: 3 or an amino acid sequence in which one or more amino acids are substituted, deleted, added, and/or inserted in the amino acid sequence shown in SEQ ID No: 3; and
a light chain CDR1 consisting of the amino acid sequence shown in SEQ ID No: 4 or an amino acid sequence in which one or more amino acids are substituted, deleted, added, and/or inserted in the amino acid sequence shown in SEQ ID No: 4; a light chain CDR2 consisting of the amino acid sequence shown in SEQ ID No: 5 or an amino acid sequence in which one or more amino acids are substituted, deleted, added, and/or inserted in the amino acid sequence shown in SEQ ID No: 5; and a light chain CDR3 consisting of the amino acid sequence shown in SEQ ID No: 6, 20 or 17, or an amino acid sequence in which one or more amino acids are substituted, deleted, added, and/or inserted in the amino acid sequence shown in SEQ ID No: 6, 20 or 17.

2. The agent according to claim 1, wherein the human monoclonal antibody comprises a heavy chain variable region consisting of an amino acid sequence having at least 80% or more sequence identity to the amino acid sequence shown in SEQ ID No: 7, and a light chain variable region consisting of an amino acid sequence having at least 80% or more sequence identity to the amino acid sequence shown in SEQ ID No: 8, 21 or 18.

3. The agent according to claim 1 or 2, wherein the human monoclonal antibody comprises a heavy chain consisting of an amino acid sequence having at least 80% or more sequence identity to the amino acid sequence shown in SEQ ID No: 9, and a light chain consisting of an amino acid sequence having at least 80% or more sequence identity to the amino acid sequence shown in SEQ ID No: 10, 22 or 19.

4. The agent according to claim 1 or 2, wherein the agent is intravenously administered.

5. A human monoclonal antibody that specifically binds to human HMGB1, comprising:
a heavy chain complementarity determining region (CDR) 1 consisting of the amino acid sequence shown in SEQ ID No: 1; a heavy chain CDR2 consisting of the amino acid sequence shown in SEQ ID No: 2; and a heavy chain CDR3 consisting of the amino acid sequence shown in SEQ ID No: 3; and
a light chain CDR1 consisting of the amino acid sequence shown in SEQ ID No: 4; a light chain CDR2 consisting of the amino acid sequence shown in SEQ ID No: 5; and a light chain CDR3 consisting of the amino acid sequence shown in SEQ ID No: 20 or 17.

6. The human monoclonal antibody according to claim 5, comprising a heavy chain variable region consisting of an amino acid sequence having at least 80% or more sequence identity to the amino acid sequence shown in SEQ ID No: 7, and a light chain variable region consisting of an amino acid sequence having at least 80% or more sequence identity to the amino acid sequence shown in SEQ ID No: 21 or 18.

7. The human monoclonal antibody according to claim 5, comprising a heavy chain consisting of an amino acid sequence having at least 80% or more sequence identity to the amino acid sequence shown in SEQ ID No: 9, and a light chain consisting of the amino acid sequence having at least 80% or more sequence identity to the amino acid sequence shown in SEQ ID No: 22 or 19.

8. An agent for preventing or treating Alzheimer's disease or frontotemporal lobar degeneration, comprising the human monoclonal antibody according to any one of claims 5 to 7.

9. The agent according to claim 8, wherein the agent is intravenously administered.

10. An agent for preventing or treating amyotrophic lateral sclerosis, Parkinson's disease, Huntington's disease, spinocerebellar ataxia, aging-related degenerative or neurological diseases, brain aging, or diseases associated with brain aging, comprising the human monoclonal antibody according to any one of claims 5 to 7.

11. The agent according to claim 10, wherein the agent is intravenously administered.
